# EUROPÄISCHE PATENTSCHRIFT

(11) **EP 3 265 480 B1**
(45) Veröffentlichungstag und Bekanntmachung des Hinweises auf die Patenterteilung: **24.03.2021**
(21) Anmeldenummer: 16710935.4
(22) Anmeldetag: 04.03.2016
(51) Int. Cl.: C07K 14/705

(54) **SYSTEM ZUR PRÄSENTATION VON PEPTIDEN AUF DER ZELLOBERFLÄCHE**
SYSTEM FOR PRESENTING PEPTIDES ON THE CELL SURFACE
SYSTÈME DE PRÉSENTATION DE PEPTIDES À LA SURFACE DE CELLULES

(30) Priorität: 05.03.2015 DE 102015002851
(43) Veröffentlichungstag der Anmeldung: 10.01.2018
(73) Patentinhaber: Peter und Traudl Engelhorn-Stiftung zur Förderung der Lebenswissenschaften, 82362 Weilheim i. Obb (DE)
(72) Erfinder: IVANUSIC, Daniel, 13347 Berlin (DE)
(74) Vertreter: Weickmann & Weickmann PartmbB
(86) Internationale Anmeldenummer: PCT/EP2016/054682
(87) Internationale Veröffentlichungsnummer: WO 2016/139354

(56) Entgegenhaltungen:
- WO-A2-2014/186649
- FUMINORI RYU ET AL: "Domain Analysis of the Tetraspanins. Studies of CD9/CD63 Chimeric Molecules on Subcellular Localization and Upregulation Activity for Diphtheria Toxin Binding.", CELL STRUCTURE AND FUNCTION., Bd. 25, Nr. 5, 1. Januar 2000 (2000-01-01) , Seiten 317-327, XP055271431, JP ISSN: 0386-7196, DOI: 10.1247/csf.25.317
- STIPP C S ET AL: "Functional domains in tetraspanin proteins", TRENDS IN BIOCHEMICAL SCIENCES, ELSEVIER, HAYWARDS, GB, Bd. 28, Nr. 2, 1. Februar 2003 (2003-02-01), Seiten 106-112, XP004405618, ISSN: 0968-0004, DOI: 10.1016/S0968-0004(02)00014-2 in der Anmeldung erwähnt
- M. SEIGNEURET ET AL: "Structure of the Tetraspanin Main Extracellular Domain: A PARTIALLY CONSERVED FOLD WITH A STRUCTURALLY VARIABLE DOMAIN INSERTION", JOURNAL OF BIOLOGICAL CHEMISTRY, Bd. 276, Nr. 43, 1. August 2001 (2001-08-01), Seiten 40055-40064, XP055271433, US ISSN: 0021-9258, DOI: 10.1074/jbc.M105557200

## Beschreibung

Gegenstand der vorliegenden Anmeldung sind Fusionsproteine basierend auf Tetraspanin-Proteinen, bei denen die extrazelluläre Schleife ganz oder teilweise durch Peptidsequenzen anderer Zusammensetzung ersetzt ist sowie deren Herstellung und Verwendung als Transportvehikel. Die offenbarten Fusionsproteine werden in der Membran von Zellen verankert und können genutzt werden, um mit ihnen fusionierte Fremdpeptide auf der Zelloberfläche zu präsentieren.

Der strukturelle Bauplan von Zellen und deren Physiologie wird von Genen kodiert. Die Gene bestehen aus DNA und finden sich entweder im Genom, oder auf extra-chromosomalen Elementen. Sie exprimieren primäre und sekundäre Genprodukte, wie RNA und Proteine. Diese können vielseitig enzymatisch weiter modifiziert vorkommen.

Die Genprodukte finden sich vorwiegend im Cytosol. Die Proteine finden sich teilweise auch in den intrazellulären Membranen der Kompartimente und in der äußeren Zellmembran sowie gegebenenfalls auch der Zellwand wieder. Bei Eukaryoten finden sich Proteine und Vorläufer-RNAs im Zellkern. Proteine werden in intrazellulären Membranen im Cytosol sortiert und auf Zellkompartimente verteilt, können aber auch durch Sekretion aus der Zelle hinaus exportiert werden. Exportproteine aus dem endoplasmatischen Retikulum (ER) können zudem im Golgi-Apparat während des Transports glykosyliert werden.

Eine besondere Proteinspezies, die membranständigen Proteine werden aktiv an die Zellmembran geleitet und verankern sich dort vertikal zur Membran. Horizontal dagegen sind sie aber aufgrund der Membranfluidität flexibel in der Lage, innerhalb Membran zu migrieren. Zum Teil sind Membranproteine mit anderen heterologen Membranproteinen im Verbund komplexiert. So agieren humane Proteine zu 80% in assoziierten Proteinkomplexen ¹. Manche Spezies interagieren kooperativ untereinander in der Membran und bilden dort stabile Strukturen.

Über Membranproteine wird eine Vielzahl von Signalprozessen gesteuert, die die Zelle mit ihrer Umwelt interagieren lassen. Membrantransporte von Proteinen über die Zellmembran im Zusammenhang mit Sekretionsprozessen nach außen sind ebenfalls möglich.

Zur Präsentation von Epitopen nutzt das Immunsystem MHC-Membranproteine auf Zellen, um potenzielle eingedrungene Pathogene in der Zelle zu identifizieren. Diese physiologischen Prozesse sind in der Zelle meist sehr gut etabliert und die involvierten Proteine sind hoch spezifisch auf diese Funktion hin evolviert, d.h. meist sehr spezifisch auf eine bestimmte Funktion hin ausgerichtet. Deshalb kann man erwarten, dass zusätzliche, artifizielle Aminosäuresequenzen diese Funktionen stören.

Tetraspanine gehören zu einer Protein-Superfamilie mit 33 Mitgliedern in Säugetieren. Das Hauptcharakteristikum der Tetraspanine sind ihre 4 Transmembrandomänen TM1-4, was auch zur Namensgebung geführt hat. Tetraspanine enthalten zudem eine kleine und eine große extrazelluläre Proteinschleife (*small* und *large extracellular loop*), auch als SEL und LEL bezeichnet. Der LEL enthält ein hoch konserviertes CCG-Aminosäure-Motiv. Des Weiteren ist eine intrazelluläre Proteinschlaufe (*small intracellular loop,* S*I*L) enthalten. N- und C-Terminus sind intrazellulär lokalisiert ³⁴⁻³⁶. Tetraspanine organisieren sich durch laterale Protein-Protein-Interaktionen (PPIs) in Tetraspanin angereicherten Arealen (*tetraspanin enriched microdomains, TEMs*) ^{37,38}.

Um Fremdproteine und Epitope heterologer Zusammensetzung in beabsichtigter Weise, beispielsweise an die Oberfläche von Säugetierzellen, z.B. humanen Zellen, zu transportieren und dort zu präsentieren, werden besonders geeignete Trägerproteine (Carrier) benötigt. Diese sollten genügend elektromotorische Kraft besitzen und in der Lage sein, die Membranpotenziale zu nutzen, um einen Proteintransport mit einer hohen Effizienz der Translokation über die Membran hinweg zur Zelloberfläche zu gewährleisten ²⁻⁸. Es sind in der Vergangenheit sogenannte "Display"-Systeme bekannt geworden, im Allgemeinen membranständige Proteine, deren man sich als Carrierproteine für die Präsentation von Fremdproteinen bedienen kann, indem man diese gentechnisch modifiziert. Eines der bekanntesten Protein-Display-Systeme stellt das Phagen-Display dar. Es findet Verwendung in der Präsentation von Proteinbestandteilen auf der Oberfläche von filamentösen Bakteriophagen im bakteriellen System ⁹⁻¹². Es konnten jedoch auch bei Gram-positiven sowie Gram-negativen Bakterien in der Vergangenheit erfolgreich heterologe Proteine auf der äußersten Membran ohne Verwendung des Phage-Displays präsentiert werden. Hierbei wurden unterschiedliche Transportmechnismen innerhalb der Bakterienzelle ausgenutzt, um Proteine zur äußersten Membran der Bakterienzelle zu leiten ¹³⁻²⁰_{.} Weitere Entwicklungen bei Display-Systemen bewegten sich hin zu eukaryotischen Systemen wie Hefen und Insektenzellen, um eine Proteinfaltung sowie Modifizierungen an rekombinanten Proteinen wie z.B. N-Glykosylierungen sicherzustellen ²¹⁻²⁹ . Auch für den Einsatz im Säugetier-System wurden bereits erste Display-Systeme entwickelt ³⁰⁻³³.

Die Aufgabe der vorliegenden Erfindung war ausgehend von diesem Stand der Technik die Bereitstellung einer neuen Möglichkeit, beliebige heterologe Peptide in einer Plasmamembran zu verankern und auf der Zelloberfläche zu präsentieren. Überraschenderweise wurde nun gefunden, dass Tetraspanine sich hervorragend als Vehikel eignen und eine hocheffiziente Transportleistung für mit ihnen fusionierte Fremdpeptide zur Verankerung und Präsentation auf der Oberfläche von Zellen besitzen. Sie sind in der Lage, Fremdpeptide hocheffizient über das endoplasmatische Retikulum und den Golgi-Apparat zur Plasmamembran von Säugerzellen zu geleiten.

Proteine der Tetraspanin-Familie weisen zwischen den Transmembrandomänen TM3 und TM4 im Wildtyp eine Proteinschleife (*large extracellular loop,* LEL) auf. Überraschenderweise konnte im Rahmen der vorliegenden Erfindung gefunden werden, dass diese extrazelluläre Schleife ganz oder teilweise durch Peptidsequenzen anderer Zusammensetzung ersetzt werden kann und es so möglich ist, das eingefügte heterologe Peptid über das Tetraspanin-Trägerprotein über eine Membran zu transportieren und in der Membran zu verankern.

Die Erfindung begründet sich auf der Einfachheit einer einheitlichen, molekularen Struktur zur flexiblen und variablen Sequenzmodulierung in einem modularen Protein-Expressions-System in Verbindung mit der Funktion eines spezifischen Transportvehikels über zelluläre Membranen und deren dortige Verankerung.

Die bereitgestellten Vektorsysteme ermöglichen es mittels einfacher Klonierungsstrategien Proteinbestandteile flexibel auszutauschen oder zu ergänzen (z.B. einzelne Epitope zu addieren und auch ganze Proteinbibliotheken anzulegen).

Nach der Expression der erfindungsgemäßen Tetraspanin-Fusions-Konstruktionen (transient oder stabil ins Genom integriert) ist die Lokalisation der Fusionsproteine in der Membran nahezu quantitativ. Die Tetraspaninproteine werden sehr effizient auf die Membran transloziert. Dies ist bei den bereits bekannten Systemen in der Form bei Weitem nicht (z.B. EGFR-Rezeptor) erreicht.

Die Expression der erfindungsgemäßen Tetraspanin-Fusionsproteine erfolgt vergleichsweise schnell, in der Regel bereits über Nacht, wohingegen die Expression bei anderen Transportsystemen üblicherweise innerhalb von zwei Tagen erfolgt.

Andererseits ist auch die Stabilität der Fusionskonstrukte und deren Lebensdauer im Vergleich mit den bekannten Proteinen mit den sieben Transmembran-Domänen im Vergleich besser, da man keine Anhäufung von cytosolischen Abbauprodukten findet (GPCR-Rezeptoren sind rekombinant als instabil bekannt).

Mit dem erfindungsgemäßen System, das nur vier Transmembrandomänen besitzt, kann man stabil und problemlos ein bis mehrere Proteine auf zellulären Oberflächen transportieren und dort beispielsweise als stationären Partner in Interaktionsexperimenten mit kleinen Molekülen, weiteren Proteinen wie Antikörpern, sowie anderen Proteinen (z.B. katalytischen Enzymen, DNAs, RNAs und Zuckern etc. fixieren bzw. immobilisieren.

Die Reaktionspartner der Fusionsproteine auf der Membran können nun flexibel aus der umgebenden Lösung heraus spezifische Reaktionen mit den Proteinen bzw. den Glycosylresten auf der Membran eingehen.

Die Voraussetzung für diese Reaktionen liegen in der funktionalen Konformation der Proteine. Diese ist bei den erfindungsgemäßen Fusionsproteinen vollkommen gewährleistet.

Ein erster Gegenstand der vorliegenden Erfindung ist daher ein Fusionsprotein, umfassend eine erste Domäne (i), eine zweite Domäne (ii) und eine dritte Domäne (iii), wobei die zweite Domäne zwischen der ersten und der dritten Domäne angeordnet ist, wobei
(i) eine Teilsequenz eines Tetraspanins, welche die Transmembrandomäne 1 (TM1), die kleine extrazelluläre Schleife (SEL), die Transmembrandomäne 2 (TM2), die kleine intrazelluläre Schleife (SIL) und die Transmembrandomäne 3 (TM3) einschließt, oder eine dazu homologe Sequenz mit einer Sequenzidentität von mindestens 95% über die gesamte Länge umfasst,
(ii) ein Peptid mit einer vorbestimmten Aminosäuresequenz umfasst, die zu der großen extrazellulären Schleife (LEL) eines Tetraspanins eine Sequenzidentität von weniger als 70% über die gesamte Länge aufweist und
(iii) eine Teilsequenz eines Tetraspanins umfasst, welche die Transmembrandomäne 4 (TM4) oder eine dazu homologe Sequenz mit einer Sequenzidentität von mindestens 95 % über die gesamte Länge umfasst,
und wobei zwischen der ersten und der zweiten Domäne und/oder zwischen der zweiten und der dritten Domäne flexible Serin- und Arginin-freie Linker angeordnet sind,
wobei das Fusionsprotein zur Verankerung in der Membran einer Zelle in der Lage ist.

In der vorliegenden Erfindung wurde gefunden, dass für die Membranständigkeit des Fusionsproteins die Sequenzen TM1-3 und TM4 verantwortlich sind. Daher umfasst ein erfindungsgemäßes Fusionsprotein zum einen eine erste Domäne (i), die eine Teilsequenz eines Tetraspanins mit den Transmembrandomänen TM1-TM3 umfasst und zum anderen eine Domäne (iii), welche eine Teilsequenz eines Tetraspanins mit der Transmembrandomäne TM4 umfasst, oder dazu homologe Sequenzen.

Der Begriff "*Tetraspanin*" bezeichnet im Sinne der Erfindung beliebige Proteine der Tetraspanin-Familie, insbesondere die 33 humanen Tetraspanine. Tetraspanine können untereinander in einer Membran spezifische Wechselwirkungen eingehen. Dadurch wird der Transport über die Membran und die dortige Verankerung begünstigt. Dieser Effekt kann in den erfindungsgemäßen Fusionsproteinen genutzt werden, um auch das Fremdpeptid der Domäne (ii) effektiv über die Membran zu transportieren und zu verankern. Bevorzugte Beispiele für Tetraspanine im Sinne der Erfindung sind CD63, CD9 CD82, CD81, CD151 und CD53 (DNA-Sequenzen und Proteinsequenzen, siehe Sequenzprotokoll. Besonders bevorzugt stammen die in einem erfindungsgemäßen Fusionsprotein enthaltenen Teilsequenzen von dem Tetraspanin CD63 (DNA- und Proteinsequenzen, siehe Sequenzprotokoll).

Grundsätzlich ist es möglich, für die Domäne (i) eine Teilsequenz eines ersten Tetraspanins zu wählen und für die Domäne (iii) eine Teilsequenz eines anderen Tetraspanins zu wählen. Bevorzugt sind jedoch alle Teilsequenzen aus demselben Tetraspanin, beispielsweise CD63, CD9, CD82, CD81, CD151 oder CD53, bevorzugt CD63 (DNA- und Proteinsequenzen, siehe Sequenzprotokoll).

Die Domäne (i) umfasst vorzugsweise die Transmembrandomäne TM1, die kleine extrazelluläre Schleife (SEL), die Transmembrandomäne TM2, die kleine intrazelluläre Schleife (SIL) und die Transmembrandomäne TM3 eines Tetraspanins, wobei die verschiedenen Transmembrandomänen und - schleifen unmittelbar aufeinander folgen, ohne dass zwischen diesen zusätzliche heterologe Sequenzabschnitte eingefügt sind. Besonders bevorzugt entspricht die Teilsequenz dem Wildtyp eines Tetraspanins ohne jegliche Abweichungen in Form von beispielsweise Substitutionen, Insertionen oder Deletionen einzelner oder mehrerer Aminosäuren. Es ist jedoch grundsätzlich möglich, für die Domäne (i) modifizierte Teilsequenzen eines Tetraspanins zu verwenden sofern diese eine Sequenzidentität von mindestens 70% über die gesamte Länge der Sequenz aufweisen, bezogen auf das Wildtyp Tetraspanin. Weiter bevorzugt beträgt die Sequenzidentität mindestens 80%, 85%, 90%, 95% oder 99% über die gesamte Länge der in der Domäne (i) enthaltenen Teilsequenz eines Tetraspanins.

Gemäß der Erfindung besteht die erste Domäne (i) aus der vorstehend beschriebenen Teilsequenz eines Tetraspanins oder einer dazu homologen Sequenz mit einer Sequenzidentität von mindestens 95% (bevorzugt mindestens 99%) über die gesamte Länge.

Die Domäne (iii) umfasst eine Teilsequenz eines Tetraspanins, welche die Transmembrandomäne TM4 eines Tetraspanins oder eine dazu homologe Sequenz mit einer Sequenzidentität von mindestens 95% über die gesamte Länge umfasst. Bevorzugt ist die Sequenzidentität zu der Teilsequenz eines Tetraspanin-Wildtyps mindestens 99% über die gesamte Länge. In einer besonders bevorzugten Ausführungsform besteht die Domäne (iii) aus der vorstehend beschriebenen Teilsequenz eines Tetraspanins oder einer dazu homologen Sequenz.

Die Domäne (ii) umfasst ein Peptid mit einer vorbestimmten Aminosäuresequenz. Dieses wird hierin auch "Fremdpeptid" oder "Fremdprotein" genannt. Die Aminosäuresequenz des Fremdpeptids unterscheidet sich von der Sequenz der großen extrazellulären Schleife (LEL) eines Wildtyp-Tetraspanins, insbesondere des (der) Tetraspanin(e) der Domänen (i) und (iii). Im Vergleich zu der LEL eines Wildtyp-Tetraspanins beträgt die Sequenzidentität über die gesamte Länge weniger als 70%, bevorzugt weniger als 50%, weiter bevorzugt weniger als 40% oder weniger als 30%.

Die Aminosäureabfolge in der Domäne (ii) kann grundsätzlich beliebig gewählt werden. Unabhängig von der Sequenz des Fremdpeptids bzw. der Domäne (ii) kann ein erfindungsgemäßes Fusionsprotein auf der Oberfläche von Zellen, insbesondere von Säugerzellen (z.B. HEK293T-Homo sapiens) verankert und präsentiert werden.

Unter einem *"Peptid"* wird im Sinne der vorliegenden Erfindung eine Verbindung aus mindestens zwei Aminosäuren verstanden, die untereinander über Peptidbindungen verknüpft sind. Die Länge des Peptids ist dabei grundsätzlich nicht begrenzt. Es sind sowohl Oligopeptide mit bis zu 10 Aminosäuren als auch Polypeptide mit über 10 Aminosäuren und Makropeptide mit mehr als 100 Aminosäuren umfasst. In einer bevorzugten Ausführungsform beträgt die Größe des Peptids weniger als 100 kDa, bevorzugt weniger als 50 kDa oder weniger als 30 kDa, beispielsweise 10-30 kDa.

Im Sinne der Erfindung werden unter dem Begriff *"Aminosäure"* vorzugsweise die in zwanzig natürlich vorkommenden Peptiden, Polypeptiden und Proteinen gefundenen Aminosäuren verstanden, die jeweils L-Isomere sind. Der Begriff umfasst erfindungsgemäß aber außerdem Analoga der Aminosäuren und D-Isomere der Aminosäuren und ihrer Analoga.

Im Sinne der Erfindung werden die Begriffe *"Peptid", "Polypeptid"* und *"Protein"* jeweils austauschbar verwendet. In einer bevorzugten Ausführungsform handelt es sich bei dem Peptid um ein Protein mit mindestens 50 Aminosäuren. Dabei ist es bevorzugt, dass das Protein eine vorbestimmte Faltung einnimmt, die beispielsweise für eine gewünschte Funktion des Proteins erforderlich ist.

In der Domäne (ii) kann das Peptid mit vorgestimmter Aminosäuresequenz beispielsweise ein Antigen oder ein Epitop eines Antigens umfassen. Unter einem *"Epitop"* wird dabei ein Bereich eines Antigens verstanden, an dem ein Antikörper oder T-Zell-Rezeptor spezifisch bindet. Ein einzelnes Antigen kann ein oder mehrere Epitope umfassen. In einer Ausführungsform der Erfindung umfasst das Peptid mit vorbestimmter Aminosäuresequenz mehrere Epitope, die durch Linker miteinander verknüpft sind. Beispielsweise handelt es sich um mehrere Epitope eines Antigens. Beispiele für Epitope sind Krebsepitope, HIV-Epitope (z.B. gp41 von HIV-1). Beispiele hierfür sind V5-6xHis (SEQ ID NOs: 35, 36) oder 2F5-4E10 (SEQ ID NOs: 37, 38).

In einer weiteren Ausführungsform der Erfindung umfasst das Peptid mit vorbestimmter Aminosäuresequenz der Domäne (ii) ein Protein, welches eine gewünschte Funktion aufweist, beispielsweise ein Enzym wie z.B. eine Protease.

Die zweite Domäne (ii) kann neben dem Peptid mit vorbestimmter Aminosäuresequenz weiterhin ein oder mehrere Teilsequenzen der LEL eines Tetraspanins oder dazu homologe Sequenzen mit einer Sequenzidentität von mindestens 70% über die gesamte Länge umfassen. Bevorzugt beträgt die Sequenzidentität mindestens 80%, mindestens 85%, mindestens 90%, mindestens 95% oder mindestens 99%. Die Teilsequenzen der LEL eines Tetraspanins können C- und/oder N-terminal zu dem Peptid mit der vorbestimmten Aminosäuresequenz angeordnet sein. Vorzugsweise stammen Teilsequenzen der LEL in der Domäne (ii) von demselben Tetraspanin wie die Sequenzabschnitte der Domänen (i) und (iii).

*"Homolog"* wie hierin verwendet beschreibt die Sequenzähnlichkeit zwischen zwei Polypeptiden, Molekülen oder zwischen zwei Nukleinsäuren. Wenn eine Position in beiden der zwei verglichenen Sequenzen durch die gleiche Basen- oder Aminosäure-Monomeruntereinheit besetzt ist, so sind die entsprechenden Moleküle an dieser Position homolog. Die prozentuale Homologie zwischen zwei Sequenzen ist die Funktion der Zahl von Übereinstimmungen oder homologen Positionen, welche die beiden Sequenzen gemeinsam haben, geteilt durch die Zahl der verglichenen Positionen mal Hundert. Wenn z.B. 6 von 10 Positionen in zwei Sequenzen übereinstimmen oder homolog sind, dann sind die beiden Sequenzen zu 60% homolog. Im Allgemeinen wird ein Vergleich durchgeführt, wenn zwei Sequenzen so aneinander ausgerichtet werden, dass eine möglichst große Homologie erreicht wird. Ein solches Ausrichten kann bereitgestellt werden, indem z.B. das Verfahren von Niedelman et al., J. Mol. Biol. 48: 443-453, 1970, vorzugsweise unter Zuhilfenahme von Computerprogrammen, durchgeführt wird.

Homologe Sequenzen haben identische oder ähnliche Aminosäurereste gemeinsam, wobei ähnliche Aminosäurereste konservative Substitutionen für entsprechende Aminosäurereste oder erlaubte Punktmutationen von entsprechenden Aminosäureresten in einer ausgerichteten Referenzsequenz darstellen. In dieser Hinsicht stellt eine *"konservative Substitution"* eines Restes in einer Referenzsequenz diejenigen Substitutionen dar, die zu den entsprechenden Referenzresten physikalisch oder funktionell ähnlich sind, z.B. die eine ähnliche Größe, Form, elektrische Ladung, chemische Eigenschaften, einschließlich der Fähigkeit, kovalente Bindungen oder Wasserstoffbindungen zu bilden und dergleichen aufweisen. Besonders bevorzugte konservative Substitutionen sind diejenigen, welche die Kriterien erfüllen, die für eine akzeptierte Punktmutation in Dayhoff et al., "Atlas of Protein Sequence and Structure", 5: Suppl. 3, Kap. 22: 354-352, Nat. Biomed. Res. Foundation, Washington, D.C., 1978, definiert sind.

Die Domänen eines erfindungsgemäßen Fusionsproteins sind in der Reihenfolge jeweils (i)-(ii)-(iii) angeordnet. Der Begriff *"Fusion"* bezieht sich dabei auf eine kolineare Kopplung der Domänen über ihre einzelnen Peptidgrundgerüste.

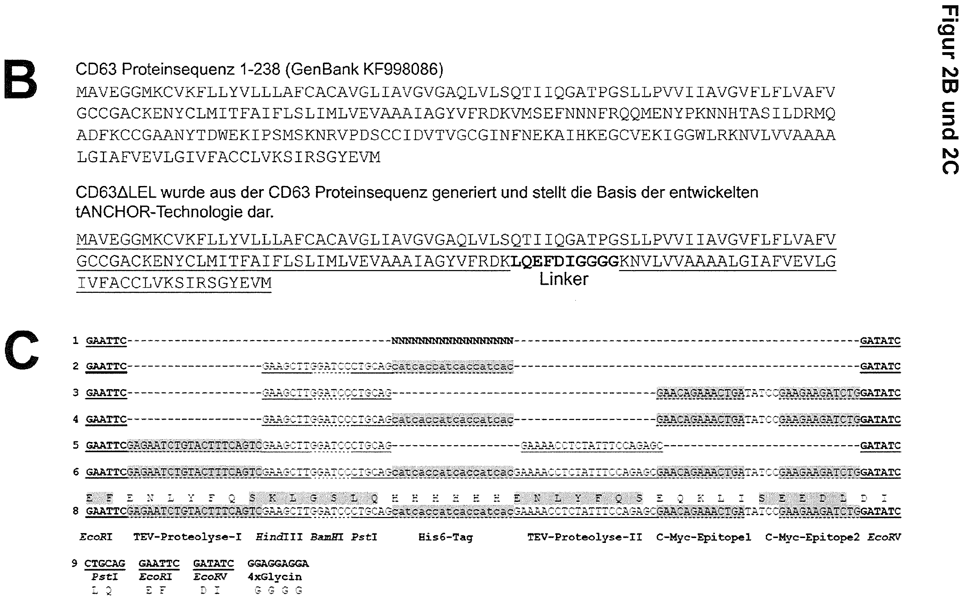

Zwischen der ersten und der zweiten Domäne und/oder zwischen der zweiten und der dritten Domäne sind flexible Serin- und Arginin-freie Linker angeordnet. Die vorhandenen Linker können gleich oder unterschiedlich sein. Beispielhafte Linker zur Verwendung in den erfindungsgemäßen Fusionsproteinen umfassen z.B. Glycinpolymere (G)ₙ, worin n eine ganze Zahl von mindestens 1 ist, Glycin-Alanin-Polymere und andere flexible Linker, die dem Fachmann bekannt sind. Ganz besonders bevorzugt ist der Linker LQEFDIGGGG (entspricht SEQ ID NOs: 41, 42). Weitere Linker sind in Figur 2C beschrieben (siehe auch SEQ ID NOs: 39, 40).

Die Domäne (ii) kann weiterhin eine oder mehrere Erkennungssequenzen umfassen, die von einem Enzym gespalten werden können. Beispiele sind Erkennungsstellen für spezifische Proteasen, Nukleasen oder Endoglycosidasen. Alternativ kann die Erkennungsstelle eine Substraterkennungsstelle für ein spezifisches Hydrolaseenzym umfassen, z.G. Phosphatase, Glycosidase, Amidase oder Esterase. Der Begriff *"Proteaseerkennungssequenz"* wird hierin zusammenfassend für jegliche Erkennungssequenzen verwendet, die enzymatisch gespalten werden können. Diese können C- und/oder N-terminal zu dem Peptid mit der vorbestimmten Aminosäuresequenz angeordnet sein. In einer besonders bevorzugten Ausführungsform flankieren die Proteaseerkennungssequenzen unmittelbar das Peptid mit der vorbestimmten Aminosäuresequenz. Grundsätzlich sind hierfür beliebige Proteaseerkennungssequenzen geeignet. Wenn die zweite Domäne (ii) neben dem Peptid mit vorbestimmter Aminosäuresequenz noch ein oder mehrere Teilsequenzen der LEL eines Tetraspanins umfasst, so ist es bevorzugt, die Proteaseerkennungssequenzen jeweils zwischen die LEL Teilsequenz und den C- bzw. N-Terminus des Peptids mit vorbestimmter Aminosäuresequenz einzufügen.

In einer weiteren Ausführungsform kann ein erfindungsgemäßes Fusionsprotein zusätzlich eine vierte Domäne (iv) am N-Terminus und/oder eine fünfte Domäne (v) am C-Terminus des Fusionsproteins umfassen. Die Domänen (iv) und (v) können jeweils direkt oder über flexible Linker wie vorstehend definiert angebunden sein. Beispielsweise kann die Domäne (iv) direkt oder über einen flexiblen Linker am N-Terminus der Domäne (i) angebunden sein. Die Domäne (v) kann direkt oder über einen flexiblen Linker am C-Terminus der Domäne (iii) angebunden sein. Außerdem ist es möglich, Proteaseerkennungssequenzen wie vorstehend definiert zwischen die Domänen (iv) und (i) und/oder zwischen die Domänen (iii) und (v) anzuordnen bzw., sofern vorhanden, zwischen Linker und einer oder mehrerer der Domänen (i), (iii), (iv) und (v).

Bei einer Verankerung eines Tetraspanins in der Plasmamembran einer Zelle liegen die C- und N-Termini des Tetraspanins auf der cytoplasmatischen Seite. Entsprechendes gilt auch bei einer Membranverankerung eines erfindungsgemäßen Fusionsproteins.

Die vierte und die fünfte Domäne (iv) und (v) eines erfindungsgemäßen Fusionsproteins können beispielsweise so gewählt werden, dass sie eine Markergruppe oder einen sogenannten Tag umfassen. Unter einem "*Tag*" wird dabei eine kurze Peptidsequenz verstanden, beispielsweise ein Protein-Tag zur Markierung und Identifizierung, z.B. ein Flash-Tag oder das fluoreszierende Reporterprotein mCherry.

Es wurde gefunden, dass sich ein erfindungsgemäßes Fusionsprotein, welches sich von einem Wildtyp-Tetraspanin zumindest durch die Domäne (ii) unterscheidet, dennoch korrekt faltet, analog zu einem Wildtyp-Tetraspanin. Dies konnte am Beispiel der fluoreszierenden Proteine CFP und YFP in der Domäne (ii) gezeigt werden (siehe Figur 3B). Epitope zwischen der Transmembrandomäne TM3 und TM4 lassen sich mit einer extrazellulären Orientierung unter Zuhilfenahme der Rasterelektronenmikroskopie auf der Zelloberfläche nachweisen (Figur 4). Das Einführen von Epitopen zwischen der TM3 und der TM4 eines Tetraspanins und der Transport zur Zelloberfläche mit einer extrazellulären Orientierung und Epitopen am N- und C-Terminus wurde für das Beispiel CD63 mittels Rasterelektronenmikroskopie gezeigt (Figuren 5A-5D). Die erfolgreiche Proteinexpression der Fluoreszenzproteine konnte zusätzlich durch Western-Blot-Analyse bestätigt werden. Dabei konnte für das jeweilige Fusionsprotein eine spezifische Bande über das FLAG sowie mCherry Epitop detektiert werden (Figur 6). Die Lokalisierung für weitere Tetraspanine (CD9, CD81, CD82 und CD151) auf der Zelloberfläche wurde ebenfalls gezeigt (Figur 8). Des Weiteren konnte gezeigt werden, dass die Verwendung von Serin- und Arginin-armen Linkern die Effizienz der Lokalisierung auf der Zelloberfläche steigert (Figur 10).

Ein weiterer Gegenstand der vorliegenden Erfindung ist ein Nukleinsäuremolekül, welches für ein erfindungsgemäßes Fusionsprotein kodiert. Ein erfindungsgemäßes Nukleinsäuremolekül umfasst einen für ein vorstehend beschriebenes Fusionsprotein kodierenden Sequenzabschnitt oder besteht aus diesem. Dieser Sequenzabschnitt wird hierin auch als *"Fusionsgen"* bezeichnet. Besonders bevorzugt liegt ein erfindungsgemäßes Nukleinsäuremolekül in isolierter Form vor.

Ein *"Nukleinsäuremolekül"* im Sinne der Erfindung bezieht sich auf eine Abfolge von natürlich vorkommenden Nukleinsäurebasen, Basenanaloga, Basenderivaten oder Mischformen davon. Der Begriff schließt insbesondere DNA und RNA sowie davon abgeleitete Nukleinsäuren wie z.B. cDNA und mRNA mit ein. Zu den Nukleinsäuren gehören im Sinne der Erfindung auch Nukleinsäureanaloga wie PNA (peptide nucleic acid), LNA (locked nucleic acid), PSNA (phosphothioate nucleic acid). Diese Nukleinsäureanaloga können prinzipiell die natürlich vorkommenden Nukleinsäurebasen aufweisen, die allerdings in anderer Art und Weise miteinander verknüpft sind, als beispielsweise in DNA oder RNA. Zu den Nukleinsäuren gehören auch Nukleinsäuren, die Nukleinsäurederivate wie z.B. Hypoxanthine, 2,5-Diaminoporin und/oder Methylcytosin aufweisen, und modifizierte, insbesondere markierte, Nukleinsäuren. Diese Nukleinsäurederivate können neben Basenderivaten prinzipiell auch die natürlich vorkommenden Nukleinsäurebasen aufweisen. Markierungen können an Basen und/oder Rückgrat eingeführt sein. Die Verknüpfungen können DNA bzw. RNA oder den Nukleinsäureanaloga entsprechen. Die Nukleinsäuren können doppelsträngig oder einzelsträngig, linear, verzweigt oder zirkulär sein.

Die vorliegende Erfindung betrifft weiterhin einen Vektor, welcher ein Nukleinsäuremolekül wie vorstehend beschrieben umfasst. Vorzugsweise ist das Nukleinsäuremolekül in operativer Verknüpfung mit einer Expressionskontrollsequenz.

Der Begriff *"Expressionskontrollsequenz"* im Sinne der Erfindung umfasst sowohl Elemente, die die Expression auf Transkriptionsebene regulieren, wie beispielsweise Enhancer oder Silencer, als auch Elemente, die die Expression auf posttranskriptioneller Ebene regulieren (z.B. Splicing, Nuclear Export oder mRNA Stabilität). Der Begriff umfasst auch mehrere Elemente, die die Expression auf transkriptioneller bzw. posttranskriptioneller Ebene regulieren, bzw. Kombinationen von transkriptionellen und posttranskriptionellen Regulationselementen. Techniken und Hilfsmittel zur Identifizierung von Expressionskontrollsequenzen, die zu einer erhöhten Expression führen und zur Isolierung derselben sind dem Fachmann wohl bekannte Routinetechniken. Die Expressionskontrollsequenz kann insbesondere ein Promotor sein, bevorzugt ein eukaryotischer Promotor, wie z.B. ein CMV- oder ein SV40-Promotor; ein prokaryotischer Promotor oder Phagen-spezifischer Promotor, wie z.B. ein SP6- oder ein T7-Promotor. Besonders bevorzugt ist der Promotor ein CMV-Promotor. Der Vektor kann zusätzlich einen Sequenzabschnitt für die Termination und/oder Polyadenylierung des Nukleinsäuremoleküls umfassen.

Ein erfindungsgemäßer Vektor enthält weiterhin bevorzugt ein Selektionsmarkergen, d.h. einen Sequenzabschnitt der für einen Selektionsmarker kodiert. Beispiele für Selektionsmarker sind Antibiotikaresistenz oder Auxotrophie-Marker. Vorzugsweise wird ein amplifizierbares Selektionsmarkergen, wie z.B. Dihydrofolatreduktase, verwendet. Optional kann der erfindungsgemäße Vektor weiterhin einen Replikationsursprung umfassen.

Der Begriff *"in operativer Verknüpfung"* beschreibt im Sinne der vorliegenden Anmeldung eine Verknüpfung zweier oder mehrerer Nukleinsäuresequenzen oder Teilsequenzen, die so positioniert sind, dass sie ihre beabsichtigte Funktion ausüben können. Beispielsweise ist ein Promoter/Enhancer funktionell mit einer kodierenden Gensequenz verknüpft, wenn er in cis-Stellung die Transkription der verknüpften Gensequenz kontrollieren oder modulieren kann. Im Allgemeinen, jedoch nicht notwendigerweise, befinden sich funktionell verknüpfte Sequenzen in enger Nachbarschaft und, sofern zwei kodierende Gensequenzen verknüpft werden oder im Falle einer Sekretionssignalsequenz, im gleichen Leseraster. Obwohl sich ein funktionell verknüpfter Promoter im Allgemeinen stromaufwärts von der kodierenden Gensequenz befindet, muss er nicht notwendigerweise eng benachbart sein. Enhancer müssen ebenfalls nicht in enger Nachbarschaft vorliegen, solange sie die Transkription der Gensequenz begünstigen. Zu diesem Zweck könnten sie sowohl stromaufwärts als auch stromabwärts von der Gensequenz vorliegen, ggf. auch in einigem Abstand. Eine Polyadenylierungsstelle ist funktionell mit einer Gensequenz verknüpft, wenn sie am 3'-Ende der Gensequenz derart positioniert ist, dass die Transkription über die kodierende Sequenz bis hin zum Polyadenylierungssignal fortschreitet. Die Verknüpfung kann nach üblichen rekombinanten Methoden erfolgen, z.B. mittels der PCR-Technik, durch Ligation an geeigneten Restriktionsschnittstellen oder durch Spleißen. Wenn keine geeigneten Restriktionsschnittstellen vorhanden sind, können in an sich bekannter Weise synthetische Oligonukleotidlinker oder Adaptoren verwendet werden. Vorzugsweise erfolgt die funktionelle Verknüpfung nicht über Intron-Sequenzen.

Ein erfindungsgemäßer Vektor ist bevorzugt für mindestens eine (Wirts)zelle geeignet, insbesondere zur Replikation des Vektors in dieser Zelle und/oder zur Expression eines Proteins oder Peptids in dieser Zelle. Replikation kann in einer prokaryotischen und/oder eukaryotischen Zelle erfolgen. Expression erfolgt bevorzugt in einer eukaryotischen Zelle. Der Vektor kann ein Plasmid sein.

Vektorkonstruktionen für die eukaryotische Expression nach Stand der Technik besitzen in der Regel einen starken, möglichst Zelltyp-unspezifischen eukaroytischen Promoter (z.B. den CMV- oder SV40-Promoter) und einen für die transkriptionale Termination und die Polyadenylierung verantwortlichen Sequenzteil (z.B. SV40-Termination-polyA). Zwischen diesen beiden Elementen befindet sich die zu exprimierende CDS (kodierende Sequenz), angeführt von einer mehr oder weniger gut ausgeprägten Kozak-Sequenz um das Startcodon herum. Im Falle der erfindungsgemäßen Vektor-Konstruktion ist die zu exprimierende CDS ein Gen aus der Superfamilie der Tetraspanine. Hierbei sind alle Tetraspaningene aus allen Organismenspezies, die über Gene dieser Gensuperfamilie verfügen, welche die gewünschte Transportleistung über die Membran erbringen, erfindungsgemäß. Allerdings sind die erfindungsgemäßen Fusionsgene dadurch charakterisiert, dass diese mindestens eine artifiziell eingeführte heterologe DNA-Sequenz als interne und ggf. terminale Fusion besitzen können. Terminale CDS-Fusionen sind heterologe DNA-Sequenzen, die 5'-proximal und/oder 3'-distal zum Tetraspaningen für ein Fusionsprotein bzw. ein Peptid kodieren. Zwischen der dritten (TM3) und der vierten (TM4) Transmembrandomäne sind erfindungsgemäß interne, kodierende heterologe DNA-Sequenzen eingeführt. Die Transmembrandomänen der Tetraspanine sind u.a. für den Transport der heterologen kodierenden Sequenzen zur Membran, die Translokation durch die Membran und die dortige Verankerung verantwortlich.

Ein durchschnittlicher Fachmann, der in der molekularen Biologie ausgebildet ist, wird in der Lage sein, funktionale DNA-Fusionskonstrukte gegebenenfalls mittels der Zuhilfenahme einschlägiger Literatur ³⁹ herzustellen.

Weiterführende erfindungsgemäße Vektorkonstruktionen sind hier besonders hervorzuheben. Diese besitzen statt generellen eukaryotischen Promotoren auch bakterielle und phagen-spezifische, aber auch eukaryotische Zelltyp- bzw. organspezifische Promotoren, die eine Zell-differenzierungsabhängige Expression ermöglichen. Man kann solche Promotortypen einsetzen, um die erfindungsgemäßen Genkonstrukte gleich welcher Anwendung zu verwenden. Weitere erfindungsgemäße Ausführungsformen der Vektoren können beispielsweise auch RNA-Polymerase-Promotoren von Phagen aufweisen, um hocheffizient *in vitro* oder gegebenenfalls *in vivo* mRNA-Moleküle zu erzeugen. Beispiele sind die Phagenpromotoren, wie SP6- oder T7-spezifische Promotoren für die Herstellung von mRNA-Molekülen *in vitro,* um gegebenenfalls die Fusionsgen-mRNA für therapeutische Zwecke und Anwendungen zuführen zu können. Diese Variante wird als Darreichungsform von Regulierern gerne gesehen, weil sie die Rekombination von Nukleinsäuren ins Genom verhindern soll.

Die Expression von Tetraspaningen-Derivaten in Bakterien oder Hefen kann zur Isolierung der Fusionsproteine genutzt werden und in künstlichen Membranen Verwendung finden. Aber auch die eukaryotische differenzierungs- bzw. zellspezifische Expression beispielsweise in Mukosa- oder Muskelzellen kann genutzt werden, um DNA-Vakzine zellspezifisch zu exprimieren (s. z.B. EP1390490 B1).

In Zulassungsverfahren für Therapeutika sind Regulierungsvorschriften etabliert worden, die solche hochspezifischen Expressionseigenschaften zwingend fordern.

Weiterhin besitzen erfindungsgemäße Vektorsysteme ein spezifisches Design, das die Expression multipler Fusionsproteine in derselben Zelle mittels eines einzigen Vektor-Konstruktes erlaubt (s. Patentanmeldung DE 10 2013 006 487 A9). Mit diesem Design können zusätzliche Elemente vereinfacht hinzugefügt werden.

Gegenstand der Erfindung sind auch solche Nukleinsäuren und Vektoren, die einen für ein Fusionsprotein kodierenden Abschnitt aufweisen, wie vorstehend definiert, wobei jedoch anstelle einer für das Peptid mit vorbestimmter Aminosäuresequenz kodierenden Sequenz lediglich eine multiple Klonierungsstelle vorhanden ist, die die Klonierung eines für ein beliebiges Peptid mit vorbestimmter Aminosäuresequenz kodierenden Gens über Erkennungssequenzen für Restriktionsendonukleasen ermöglicht. Zusätzlich können auch multiple Klonierungsstellen in 5'- und/oder 3'-Position für N- und/oder C-terminale Domänen (iv) bzw. (v) vorhanden sein.

Ein Aspekt der Erfindung betrifft daher ein Nukleinsäuremolekül umfassend einen ersten Sequenzabschnitt (i), einen zweiten Sequenzabschnitt (ii) und einen dritten Sequenzabschnitt (iii), wobei der zweite Sequenzabschnitt zwischen dem ersten und dem dritten Sequenzabschnitt angeordnet ist, wobei
(i) für eine Teilsequenz eines Tetraspanins, welche die Transmembrandomäne 1 (TM1), die kleine extrazelluläre Schleife (SEL), die Transmembrandomäne 2 (TM2), die kleine intrazelluläre Schleife (SIL) und die Transmembrandomäne 3 (TM3) einschließt, oder eine dazu homologe Sequenz mit einer Sequenzidentität von mindestens 95% über die gesamte Länge, kodiert,
(ii) eine Multiple Cloning Site umfasst und
(iii) für eine Teilsequenz eines Tetraspanins kodiert, welche die Transmembrandomäne 4 (TM4) oder eine dazu homologe Sequenz mit einer Sequenzidentität von mindestens 95 % über die gesamte Länge umfasst,
wobei die von dem ersten Sequenzabschnitt (i) und dem dritten Sequenzabschnitt (iii) kodierten Teilsequenzen zur Verankerung in der Membran einer Zelle in der Lage sind, und
wobei Nukleinsäuresequenzen, die für einen flexiblen Serin- und Arginin-freien Linker kodieren, zwischen dem ersten und dem zweiten Sequenzabschnitt und/oder zwischen dem zweiten und dem dritten Sequenzabschnitt angeordnet sind.

Die Sequenzabschnitte (i) bis (iii) sind hierbei bevorzugt von 5' nach 3' angeordnet. Eine *"Multiple Cloning Site"* oder *"multiple Klonierungsstelle"* umfasst mehrere, hintereinander liegende Restriktionsschnittstellen für Restriktionsendonukleasen. Die Multiple Cloning Site ist insbesondere zur Integration eines Nukleinsäuremolküls geeignet, wobei das integrierte Nukleinsäuremolekül im Leseraster mit dem ersten Sequenzabschnitt (i) und dem dritten Sequenzabschnitt (iii) für ein zu präsentierendes Peptid kodiert. Das Peptid umfasst bevorzugt eine vorbestimmte Aminosäuresequenz, die zu der großen extrazellulären Schleife (LEL) eines Tetraspanins eine Sequenzidentität von weniger als 70% über die gesamte Länge aufweist.

Die vorliegende Erfindung betrifft weiterhin einen Vektor umfassend ein vorstehend beschriebenes Nukleinsäuremolekül, vorzugsweise in operativer Verknüpfung mit einer Expressionskontrollsequenz.

Im Stand der Technik sind zahlreiche Erkennungssequenzen für die verschiedensten Restriktionsendonukleasen sowie die hier zugehörigen Restriktionsendonukleasen bekannt. Bevorzugt werden Sequenzen verwendet, die aus zumindest sechs Nukleotiden als Erkennungssequenz bestehen. Eine Auflistung geeigneter Kennungssequenzen findet sich beispielsweise in Sambrook et al. (1989).

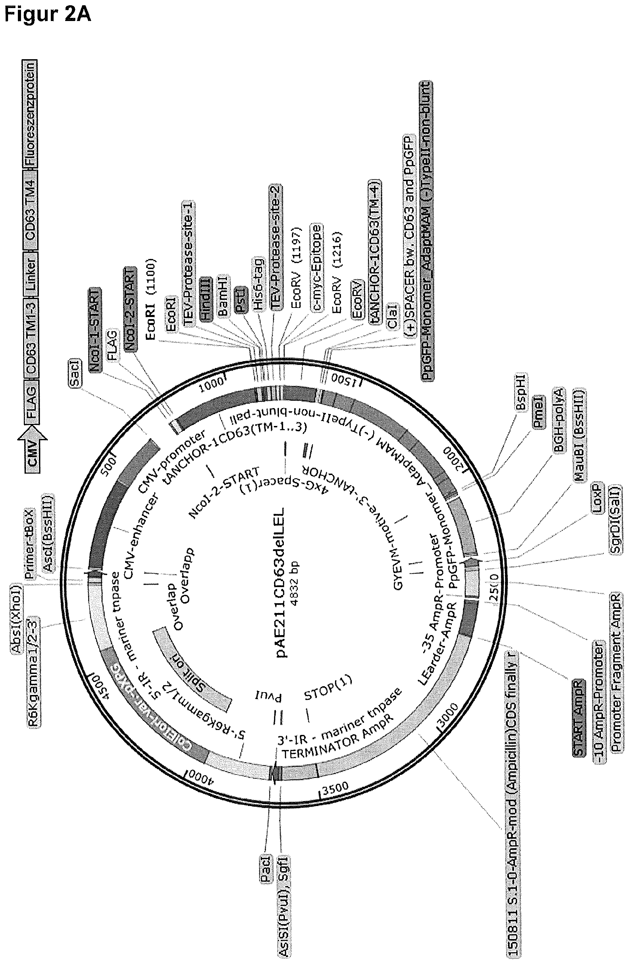

Spezifische Sequenzelemente eines erfindungsgemäßen Vektors mit multipler Klonierungsstelle betreffen die Einbringung von heterologen DNA-Sequenzen an die entsprechenden terminalen und internen, funktional zulässigen Positionen der Tetraspaningene (wie z.B. CD63) selbst. So kann man in die "uniquen", d.h. einzelnen, Restriktionsschnittstellen immer wieder neue Epitop-CDS bzw. verschiedene Gene und Genfragmente additiv in einer zyklischen Reaktion in bestehende Vektorkonstrukte einfügen. Beispielgebend ist der erfindungsgemäße Vektor pAE211 in Fig. 2A, SEQ ID NO: 1). Restriktionsstellen sind an der Position zwischen den Transmembrandomänen TM3 und TM4 positioniert. Für diese Position wurde experimentell gezeigt, dass sie für die Fusion mit heterologen DNA-Sequenzen, die für ein heterologes Peptid kodieren, besonders geeignet ist, weil sie die Funktion des Membrantransports nicht stört. An dieser Stelle befindet sich eine Proteinschleife, die über die Membran hinaussteht, deren Sequenz variabel sein kann, ohne mit dem Proteintransport zur Plasmamembran des Tetraspaninproteins in Zellkulturen wesentlich zu interferieren.

Die 5-terminale CDS-Fusion, um ein N-terminales Fusionsprotein zu erzeugen, kann mittels zweier *Nco*I im 5'-Bereich des CD63-Gens derart eingebracht werden, dass die Kozak-Sequenz im Startbereich regeneriert wird. Diese ist unique.

Das mögliche 3'-terminale-CDS-Fusionselement, welches zu einer C-terminalen Proteinfusion führt, wird mittels der TypIIS-Restriktionsendonuklease *Bsa*I inseriert, die in dem Konstrukt spezifische Enden für *Nde*I und *Bcl*I erzeugt. Auch diese Sites können gegebenenfalls zum Einfügen von 3'-terminalen Genfusionselementen Verwendung finden.

Die oben angegebenen Realisierungen von Vektorsystemen sind beispielhaft und jede andere Methodik bzw. Technologie der Modifikation der Vektoren neben der beschriebenen Restriktion-Ligationsverfahren ist ebenso erfindungsgemäß, wie beispielsweise die Sequenzmodulation der Systeme mittels geeigneten Rekombinationssystemen, *in vivo,* wie auch *in vitro.*

Ein weiterer Gegenstand der vorliegenden Erfindung ist eine Zelle, welche ein erfindungsgemäßes Nukleinsäuremolekül oder einen erfindungsgemäßen Vektor umfasst.

In einer erfindungsgemäßen Ausführungsform ist das Nukleinsäuremolekül bzw. der Vektor umfassend eine für ein Fusionsprotein kodierende Sequenz und geeignete genetische Kontrollfunktionen genomisch stabil integriert oder liegt extra-chromosomal in den Zellen vor und ein erfindungsgemäßes Fusionsprotein wird dort konstitutiv oder induzier- oder reprimierbar exprimiert.

Bei einer Zelle im Sinne der vorliegenden Erfindung handelt es sich vorzugsweise um eine eukaryotische Zelle, bevorzugt um eine Säugerzelle wie insbesondere eine humane Zelle. Weitere Beispiele für geeignete Säugerzellen sind Zelllinien von Nagern wie z.B. Mäuse-, Ratten- und Hamsterzellen, oder Affenzellen. Ein erfindungsgemäßes Nukleinsäuremolekül bzw. ein erfindungsgemäßer Vektor kann mithilfe von an sich bekannten Techniken in eine Zelle, insbesondere eine eukaryotische Zelle eingebracht werden. Die erfolgreiche Transfektion resultiert in transformierten, genetisch modifizierten, rekombinanten oder transgenen Zellen.

Die Transfektion von eukaryotischen Wirtszellen mit einem erfindungsgemäßen Nukleinsäuremolekül oder einem erfindungsgemäßen Vektor erfolgt nach üblichen Methoden (Sambrook et al., 1989; Ausubel et al., 1994). Geeignete Transfektionsmethoden sind z.B. die Liposomen-vermittelte Transfektion, Kalziumphosphat-Kopräzipitation, Elektroporation, Polykationen, (z.B. DEAE-Dextran)-vermittelte Transfektion, Protoplastenfusion, Mikroinjektion und virale Infektionen. Erfindungsgemäß werden die Zellen vorzugsweise über eine stabile Transfektion erhalten, wobei die Konstrukte entweder in das Genom der Wirtszelle oder ein artifizielles Chromosom/Minichromosom integriert werden oder in stabiler Weise episomal in der Wirtszelle enthalten sind. Die Transfektionsmethode, die die optimale Transfektionsfrequenz und Expression des heterologen Gens in der jeweiligen Wirtszelle ermöglicht, ist dabei bevorzugt. Per Definition wird jede Sequenz oder jedes Gen, das in eine Wirtszelle eingebracht wird, im Bezug auf die Wirtszelle als *"heterologe Sequenz"* oder *"heterologes Gen"* bezeichnet, selbst dann, wenn die einzubringende Sequenz oder das einzubringende Gen identisch zu einer endogenen Sequenz oder einem endogenen Gen der Wirtszelle ist.

In einer erfindungsgemäßen Ausführungsform kann es sich um Zellen von transgenen Zellverbänden verschiedener Zelltypen handeln, in denen ein erfindungsgemäßes Fusionsgen auch in unterschiedlichen zellulären Differenzierungszuständen exprimiert wird. Diese Zellen können *ex vivo* erzeugt werden und ggf. anschließend *in vivo* therapeutisch eingesetzt werden.

In einer weiteren erfindungsgemäßen Ausführungsform handelt es sich um Zellen von Zellverbänden transgener Organismen, deren Zellen die Keimbahn durchlaufen haben und sich zu vielzelligen Systemen, vorzugsweise zu vitalen Organismen, entwickelt haben.

In einer bevorzugten Ausführungsform wurde eine erfindungsgemäße Zelle weiterhin mit mindestens einem weiteren Nukleinsäuremolekül oder mindestens einem weiteren Expressionsvektor transformiert oder transfiziert. Das weitere Nukleinsäuremolekül bzw. der weitere Expressionsvektor enthält vorzugsweise einen Abschnitt, welcher für ein weiteres vorbestimmtes Protein kodiert. Bei dem weiteren Nukleinsäuremolekül oder Expressionsvektor kann es sich gemäß einer Ausführungsform ebenfalls um ein erfindungsgemäßes Nukleinsäuremolekül oder einen erfindungsgemäßen Expressionsvektor handeln, wobei in diesem Fall für die Domäne (ii) ein anderes Peptid mit einer anderen vorbestimmten Aminosäuresequenz gewählt würde. In einer anderen Ausführungsform enthält das weitere Nukleinsäuremolekül oder der weitere Expressionsvektor einen Abschnitt, der für ein Enzym kodiert, beispielsweise für eine Protease. Wenn in einer solchen Zelle das erfindungsgemäße Nukleinsäuremolekül und das weitere Nukleinsäuremolekül exprimiert werden, werden sowohl das Peptid der Domäne (ii) mit vorbestimmter Aminosäuresequenz als auch das weitere Protein an der Membranoberfläche präsentiert. Wenn beispielsweise das Peptid der Domäne (ii) von Proteaseerkennungssequenzen flankiert wird, so könnte eine über das weitere Nukleinsäuremolekül exprimierte Protease an diesen Erkennungsstellen schneiden und so das Peptid freisetzen.

Erfindungsgemäß werden die Wirtszellen vorzugsweise unter serumfreien Bedingungen etabliert, adaptiert und kultiviert, ggf. in Medien, die frei von tierischen Proteinen/Peptiden sind.

Ein weiterer Gegenstand der vorliegenden Erfindung ist eine Membranzubereitung, die aus einer erfindungsgemäßen Zelle erhalten wurde, umfassend eine Membran und ein erfindungsgemäßes Fusionsprotein.

Eine Membranzubereitung im Sinne der Erfindung umfasst mindestens eine isolierte Membran, stärker bevorzugt eine isolierte, zelluläre Membran, noch stärker bevorzugt eine isolierte, zelluläre Plasmamembran und/oder eine isolierte, zelluläre Membran anderer Zellorganelle. Der Begriff "Membran" im Sinne der vorliegenden Erfindung beschreibt ein in sich geschlossenes System umfassend mindestens ein Lipid und optional mindestens ein Protein. Die Lipide einer Membran bilden üblicherweise eine Lipiddoppelschicht. Die Membranzubereitung umfasst bevorzugt Membranpartikel, stärker bevorzugt Membranvesikel.

Die Membranzubereitung kann aus einer erfindungsgemäßen Zelle durch dem Fachmann bekannte Verfahren erhalten werden, bevorzugt durch Lyse der Zelle (Zelllysat), stärker bevorzugt durch Lyse der Zelle und anschließendes Isolieren und optional Reinigen der zellulären Membran (isolierte und optional gereinigte Membran). Optional können Tenside, wie z.B. Triton X-100, zur Solubilisierung der Membranen verwendet werden. Das Verfahren kann zusätzlich einen Schritt zum Entfernen von Zelltrümmern beinhalten. Der Durchmesser der Membranpartikeln kann im Bereich von 1 - 1000 nm sein, bevorzugt 50 - 500 nm, stärker bevorzugt 75 - 200 nm. Mindestens 80%, mindestens 90%, mindestens 95% oder mindestens 98% der Membranpartikel weisen einen Durchmesser in einem der hierin beschriebenen Bereiche auf.

Eine erfindungsgemäße Membranzubereitung wird von einer erfindungsgemäßen Zelle erhalten, die ein erfindungsgemäßes Nukleinsäuremolekül oder einen erfindungsgemäßen Vektor umfasst. In der Zelle wird das erfindungsgemäße Fusionsprotein durch das Nukleinsäuremolekül oder den Vektor exprimiert. Eine erfindungsgemäße Membranzubereitung umfasst daher mindestens eine isolierte Membran und mindestens ein erfindungsgemäßes Fusionsprotein. Das erfindungsgemäße Fusionsprotein ist hierbei bevorzugt in der Membran verankert, stärker bevorzugt auf der Oberfläche der Membran, insbesondere auf der extrazellulären Seite der Membran. Das erfindungsgemäße Fusionsprotein wird auf der Membran präsentiert, bevorzugt auf der Oberfläche der Membran, insbesondere auf der extrazellulären Seite der Membran. Die Membranzubereitung kann optional zusätzlich ein oder mehrere Wild-Typ Tetraspanine umfassen.

In einer Ausführungsform handelt es sich bei den erfindungsgemäßen Membransystemen um die äußere Membran von Zellen, z.B. von *in vitro* Zellkulturen, in denen mindestens ein erfindungsgemäßes Fusionsprotein transient exprimiert wird.

Die vorliegende Erfindung betrifft weiter ein synthetisches Membransystem umfassend eine Membran und ein Fusionsprotein wie vorstehend beschrieben.

Bei der Membran kann es sich um eine zelluläre (natürliche) oder synthetische (künstliche) Membran handeln. Eine zelluläre Membran ist insbesondere eine zelluläre Plasmamembran, die sich typischerweise an der Oberfläche (Außenschicht) einer Zelle befindet. Eine zelluläre Membran im Sinne der vorliegenden Erfindung kann Teil einer Zelle sein, oder aus einer Zelle isoliert worden sein, d.h. eine isolierte, zelluläre Membran sein. Eine zelluläre Membran ist typischerweise asymmetrisch und weist eine dem Cytoplasmazugewandte Seite (plasmatische oder intrazelluläre Seite, innere Seite) und eine dem Cytoplasma-abgewandte Seite auf (extraplasmatische oder extrazelluläre Seite, äußere Seite).

Eine synthetische Membran kann synthetische und/oder isolierte, zelluläre Membranen umfassen. Insbesondere kann sich die Lipid- und/oder Proteinzusammensetzung bei synthetischen Membranen von den natürlich vorkommenden Zusammensetzungen zellulärer Membranen unterscheiden. Eine synthetische Membran ist bevorzugt kein Teil einer Zelle und liegt insbesondere als isolierte, synthetische Membran vor. Der Fachmann kann die Eigenschaften einer synthetischen Membran durch die Wahl der Lipide und/oder Proteine beeinflussen.

In einer Ausführungsform enthält ein synthetisches Membransystem umfassend ein erfindungsgemäßes Nukleinsäuremolekül oder einen erfindungsgemäßen Vektor zusätzlich ein System zur Expression des Nukleinsäuremoleküls oder des Vektors, wodurch das Fusionsprotein exprimiert werden kann. Systeme zur Expression von Proteinen sind im Stand der Technik bekannt und sind bevorzugt zellfrei. Das System zur Expression umfasst bevorzugt ein System zur Translation eines Nukleinsäuremoleküls, insbesondere einer RNA oder mRNA; und, optional ein System zur Transkription eines Nukleinsäuremoleküls oder eines Vektors, insbesondere um eine RNA oder mRNA zu erhalten.

In einer alternativen Ausführungsform kann das Fusionsprotein direkt zur Membran zugegeben werden, um ein erfindungsgemäßes Membransystem zu erhalten.

Bei einem Membransystem wird das exprimierte oder zugegebene Fusionsprotein in der Membran verankert, bevorzugt auf der Oberfläche der Membran, insbesondere auf der äußeren Seite der Membran. Das erfindungsgemäße Fusionsprotein wird auf der Membran präsentiert, bevorzugt auf der Oberfläche der Membran, insbesondere auf der äußeren Seite der Membran.

Es wurde gefunden, dass auch in einem synthetischen Membransystem die erfindungsgemäßen Fusionsproteine zur Membranoberfläche dirigiert, translociert und dort verankert werden können. In diesem Fall ist es möglich, *in vivo* oder *in vitro* artifizielle, nicht-kanonische Aminosäuren in die erfindungsgemäßen Fusionsproteine einzubauen. Mittels zellfreier Proteinsynthese könne Fusionsproteine gewonnen werden, die geeignet sind, auch synthetische Membranen zu besiedeln und dort verankert vorzuliegen.

Ein weiterer Aspekt der vorliegenden Erfindung ist ein Verfahren zur Präsentation eines Peptids mit einer vorbestimmten Aminosäuresequenz auf der Oberfläche einer Zelle umfassend die Schritte
(a) Bereitstellen einer erfindungsgemäßen Zelle wie vorstehend beschrieben, und
(b) Kultivieren der Zelle unter Bedingungen bei denen ein erfindungsgemäßes Fusionsprotein exprimiert wird.

Die vorliegende Erfindung betrifft weiter ein Verfahren zur Verankerung eines Peptids mit einer vorbestimmten Aminosäuresequenz an einer Membran umfassend die Schritte
(a) Bereitstellen einer Membran, und
(b) Inkontaktbringen der Membran mit einem Fusionsprotein wie vorstehend beschrieben unter Bedingungen, bei denen eine Verankerung des Fusionsproteins in der Membran erfolgt oder Inkontaktbringen der Membran mit einer Nukleinsäure wie vorstehend beschrieben oder einem Vektor wie vorstehend beschrieben unter Bedingungen, bei denen das Fusionsprotein exprimiert und in der Membran verankert wird.

Ein weiterer Gegenstand der Erfindung ist die Verwendung einer vorstehend beschriebenen Zelle, einer Membranzubereitung oder eines synthetischen Membransystems in einem Verfahren zum Screening nach Interaktionspartnern des Peptids, zur Untersuchung von Wechselwirkungen des Peptids mit Interaktionspartnern (z.B. Antikörpern), in einem Verfahren zur Herstellung des Peptids und/oder als Nachweisreagenz.

Die vorliegende Erfindung betrifft weiterhin eine Zelle, Membranzubereitung oder ein synthetisches Membransystem wie vorstehend beschrieben zur Verwendung als Vakzin und/oder als Medikament.

In einer erfindungsgemäßen Ausführungsform werden die Fusionsproteine von den transfizierten, transient oder stabil exprimierenden Zellen, transgenen Organismen oder sythetischen Membransystemen entnommen, um in Forschung und Entwicklung Verwendung zu finden, wie auch ggf. als Bestandteil eines Verfahrens oder Kits einer kommerzialisierbaren Anwendung zugeführt zu werden. Beispiele sind die Herstellung von bioanalytisch-diagnostischen Systemen und Therapeutika.

In einer weiteren erfindungsgemäßen Ausführungsform werden die Membranen, welche die Fusionsproteine enthalten, den Zellen, transgenen oder synthetischen Membransystemen entnommen, um in Forschung und Entwicklung Verwendung zu finden, wie auch ggf. als Bestandteil eines Verfahrens oder Kits einer kommerzialisierbaren Anwendung zugeführt zu werden. Beispiele sind die Herstellung von bioanalytisch-diagnostischen Systemen und Therapeutika.

Die exprimierten Fusionsproteine der vorliegenden Erfindung werden auf der Zelloberfläche transfizierte Zellen als stationäre Reaktionspartner gezeigt und können für die Analyse spezifischer Interaktionen mit anderen Molekülen verwendet werden. Einerseits sind hier Protein-Protein-Interaktionen (z.B. mono- oder polyklonale Antikörper - Paratop-Epitop Wechselwirkungen, Coiled-Coil Wechselwirkungen u.v. spezifischen Interaktionen von pharmazeutisch-medizinischer, insbesondere auch molekularmechanistischer Bedeutung) interessant. Auch für die Analyse von Wechselwirkungen mit anderen Molekülen, wie Mono- und Polysacchariden, RNAs, DNA und insbesondere mit kleinen chemischen Molekülen wie Naturstoffen und chemischen Substanzbibliotheken können Tetraspanin-Fusionsmoleküle in Anspruch genommen werden.

Die Expression von multiplen Tetraspanin-Fusionsmolekülen oder Proteinvarianten einer Proteinspezies in einer Zelle ist für bestimmte experimentelle Ansätze genauso interessant wie die kombinatorische Nutzung von verschiedenen interagierenden Proteinen.

In einer Ausführungsform der Erfindung exprimiert eine erfindungsgemäße Zelle eine Proteindomäne (A), die zur hochspezifischen Wechselwirkung mit einer anderen Proteindomäne (B) in der Lage ist. Die Proteindomäne (A) entspricht hierbei dem Peptid mit vorbestimmter Aminosäuresequenz der Domäne (ii) eines erfindungsgemäßen Fusionsproteins.

Diese externe Proteindomäne könnte dann mit verschiedenen funktionalen weiteren Proteindomänen verbunden sein. Für alle diese Proteindomänmodule könnte man sicherstellen, dass diese, wenn sie exogenen Ursprungs sind, an die Gegendomäne (A) der exprimierenden Zelle spezifisch binden und damit die Proteinliganden ausgetauscht werden können, die ihrerseits wieder in Verbindung treten könnten und somit eine durch spezifische Interaktion vermittelte Ko-existenz verschiedener Proteinmodule auf der Zelloberfläche nachgewiesen werden könnte.

Bevorzugte Beispiele für die Anwendung der erfindungsgemäßen Fusionsproteine, Vektoren, Zellen und Membransysteme sollen nachfolgend ausführlicher beschrieben werden.

### Analyse von Protein-Protein-Interaktionen (PPI)

Zugänglich an Oberflächen fixierte Proteine können mittels besonderer Strukturen auf der Oberfläche der jeweiligen Proteine spezifische Kontakte zu weiteren Proteinen herstellen. Diese weiteren Proteine können die Proteinbausteine von Homo- bzw. Heteroproteinkomplexen sein, die sich wechselseitig erkennen können oder aber auch spezifische Proteine, wie Antikörper und *single chains,* die spezifisch und zielgerichtet an bestimmte Protein-Oberflächenstrukturen binden können. Genomisch kodierte natürlich vorkommende Proteine, die spezifische Interaktionen mit anderen Proteinen eingehen, üben viele zelluläre Funktionen aus - von Funktionen im Zellkern bis hin zu vielfältigen cytosolischen und Membranfunktionen. Diese spezifischen und funktionalen Protein-Protein-Interaktionen (PPI) sind im Verlauf der Evolution entstanden und sind für die Integrität der zellulären Funktion maßgeblich verantwortlich. Zielgerichtete PPI wird durch zelluläre Prozesse und die Selektion von Proteinvariante vermittelt, die eine spezifische Interaktion mit Oberflächen besitzen. Das Immunsystem ist auf die sensiblen Reaktionen gegen die Veränderung der Oberflächen innerhalb eines Organismus ausgelegt und auf die Optimierung von Oberflächeninteraktionen von Antikörpern und Cytotoxizität in sehr kurzen Zeiträumen spezialisiert. In der humoralen Immunität werden große Mengen Epitop-spezifische, polyklonale Antikörper in kurzer Zeit produziert. Auch die besonderen Einzelkettenantikörper von Kamelartigen (*single chains*) und anderen natürlich vorkommenden immunogenen Systemen, die hochspezifische Proteininteraktionen entwickeln, sind bekannt geworden.
In der medizinischen Forschung hat man diese Prozesse zur Erzeugung von monoklonalen Antikörpern technisch mittels Zellkulturtechnolgien umgesetzt und damit die Erzeugung von spezifischen PPI *in vitro* ermöglicht.

Antikörperengineering nutzt künstliche, mittels Displaytechnologien (Ribosome Display, Phagedisplay, Yeast Display) hergestellte Paratope, die Epitope spezifisch erkennen können, um diese in Antikörpermoleküle einzubauen. Die effizienten Exportfunktionen für Proteineinheiten des hier beschriebenen tANCHOR-Systems und deren Verankerung in der Membran sind in hohem Maße für PPI-Analysen geeignet. Ferner sind auch mit Zuckern spezifisch modifizierte Proteine mittels der tANCHOR-Technologie auf Zelloberflächen präsentierbar.

Detektionssysteme, mit denen der durchschnittlich qualifizierte Fachmann in der Lage ist, eine spezifische PPI zu identifizieren, werden als Zellsortersysteme (FACS), Fluoreszenz- oder Biolumineszenzbasierte Imaging-Systeme aber auch als FRET-photonische Gerätschaften im Handel angeboten. Auch die massenspektroskopische Analyse kommt hier als Analysewerkzeug in hervorragender Weise in Frage.

Alleine das zur Verwendung kommende zelluläre System macht die Qualität der zu erzeugenden Daten und den entscheidenden Unterschied. Es ist, vergleichbar mit der Auflösung in der Optik, der alle weiteren Prozessschritte entscheidende Parameter, der die Qualität der erhaltenen Daten am Ende der Prozesskette festlegt.

Displayfunktionen für bioanalytisch-diagnostische Anwendungen wie auch für Therapeutika bereitzustellen, dazu ist das tANCHOR-System in besonderer Art und Weise geeignet. Mit diesem kann die Integrität von Proteinstrukturen unter Erhalt von beispielsweise strukturellen Epitopen, aber auch die von Bindedomänen bei der PPI von zellulären Regulatorproteinen untereinander, strukturell intakt bleiben.

### Analyse von kleinen Molekülen mit tANCHOR-Konstrukten

Mittels strukturell intakten Proteinstrukturen ist es zusätzlich möglich, Wechselwirkungsstudien der Bindung von, aber auch der Verdrängung aus, einer PPI-Bindung durch die kleinen Moleküle mit den auf den Zellen präsentierten Proteinen durchzuführen. Solche Wechselwirkungen kleiner Moleküle sind interessant, die mit PPIs interferieren und bei denen bestimmte Proteinaggregate unter dem Einfluss der kleinen Moleküle wieder aufgelöst werden könnten identifiziert werden. PPI-Analysen sind für die Entdeckung und die Entwicklung von kleinen Molekülwirkstoffen besonders gut geeignet.

Die tANCHOR-Technologie bietet hierfür ein hervorragendes Werkzeug, das eine Proteinexpression mit gleichzeitiger Verankerung der Proteine auf den Zelloberflächen zur Verfügung stellt. In Verbindung mit geeigneten Proteinpaaren aus einer weiteren Expressionsquelle oder auch gegebenenfalls durch die Ko-expression von verschiedenen tANCHOR-Molekülen kann man daraus Testsysteme für die Analyse von Wechselwirkungen kleiner Moleküle mit Proteinkomplexen etablieren^{1,40-48}.

Darüber hinaus könnten die an der Zelloberfläche lokalisierten Abschnitte der erfindungsgemäßen Fusionsproteine mittels ggf. induzierbarer Co-Expression von hochspezifisch spaltenden sekretierten Proteasen (wie z.B. der TEV-Protease) spezifisch an einer vorgegebenen Stelle gespalten und so in Lösung gebracht werden. Die defekten Fusionsproteine könnten in einem Resynthesezyklus in der Membran stetig ersetzt werden und somit auch für eine dauerhafte Überexpression sorgen.

Kleine Moleküle (z.B. aus Extrakten von Sekundärmetabolite exprimierenden Organismen) könnten so an die erfindungsgemäßen Proteine binden und diese als Target identifizieren.

In den resultierenden Komplexen aus kleinen Molekülen und erfindungsgemäß präsentierten Proteinabschnitten, können die kleinen Moleküle entweder spezifisch an die an der Zelloberfläche fixierten Target-Peptide oder an die bereits abgespaltenen, als Carrier-Peptide in Lösung befindlichen Target-Peptide gebunden sein.

Die resultierenden Komplexe und somit die kleinen Moleküle und deren Bindepartner könnten dann mittels Massenspektroskopie identifiziert werden. Beispielsweise könnten im Fall nicht-peptidischer kleiner Moleküle diese nach Isolierung der in Lösung befindlichen Komplexe und Zerstörung der Peptide, z.B. mittels unspezifischer enzymatischer Proteolyse etc., mittels Massenspektroskopie identifiziert werden.

### Entwicklung und Produktion von Immundiagnostika

Entsprechend einer Ausführungsform der Erfindung sind beispielsweise Anwendungen interessant, bei denen Antikörper in einem bestimmten Format, z.B. dem standardisierten 96-Well-Format, auf die Bindung mit tANCHOR exponierten Epitopen getestet werden sollen. Mittels des erfindungsgemäßen Systems werden beispielsweise Zellen in Mikrotiterplatten mit dem 96-Well-Format ausgesät und die Fusionskonstrukte in transient transfiziertem Zustand exprimiert. Ebenso ist dies mit stabilen Fusionsproteine exprimierenden Zelllinien möglich.

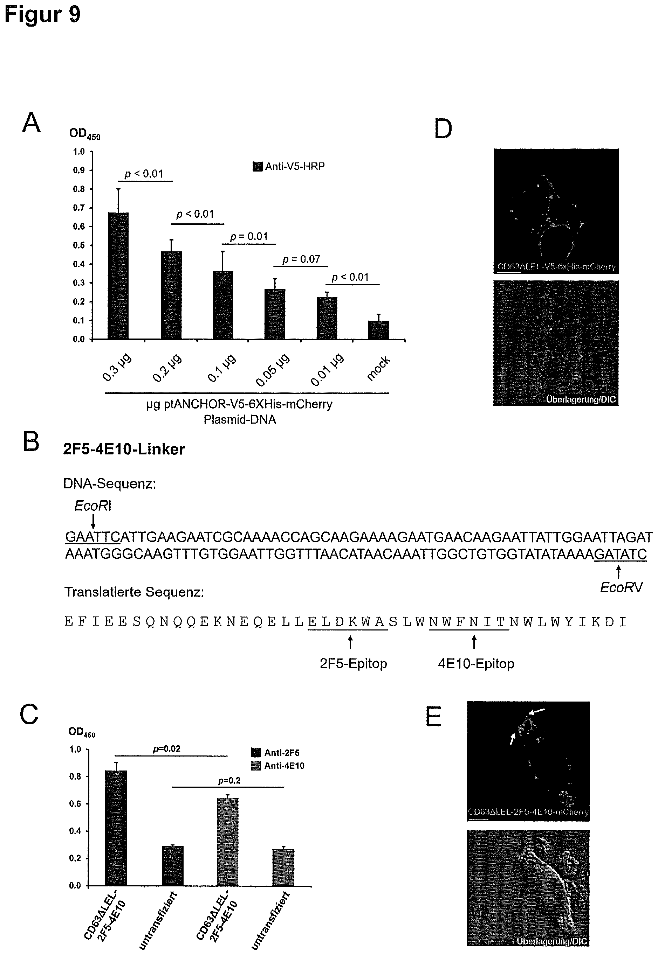

Beispielhaft wird die Verwendung des tANCHOR-Systems zur Detektion von Antikörpern in ELISA-Experimenten gezeigt (Figur 9).

Die Fusionsproteine finden sich auf den Zelloberflächen und werden dort präsentiert. Diese artifizielle Konfiguration der Membran kann direkt zur Entwicklung und Produktion von analytischen und therapeutischen Anwendungen umgesetzt werden.

Auf diese Weise können Analysesysteme hergestellt werden, in denen z.B. für klinische Studien geeignete, der jeweiligen zu entwickelnden Anwendung entsprechende Blutseren von Patientenkohorten getestet werden. Für die Entwicklung von Immundiagnostika und Therapeutika, insbesondere auch für die therapiebegleitende Diagnostik, kann mit solchen Systemen das Patientenserum auf die Anwesenheit von Antikörpern und deren Bindungseigenschaften qualitativ wie quantitativ überprüft werden.

Darüber hinaus ist es möglich, Mikrotiterplatten und andere Formate, in denen transgene Zellen gewachsen sind, zu fixieren, einzutrocknen um in großem Umfang damit bio-analytische Testsysteme und Diagnostika zu herzustellen und diese in großem Umfang zu produzieren.

Außerdem ist es auch möglich, Membranen von transgenen Organismen, die Tetraspanin-Protein-Fusionsprodukte exprimieren, zu nutzen und diese aufzureinigen. Ebenso können künstliche Membranen, die erfindungsgemäße Fusionsprodukte beinhalten, solchen Verwendungen, wie sie oben beschrieben sind, zugeführt werden.

Die Kostenersparnis ist hier immens, da ein vergleichsweise teures Beschichten (*coating*) mit Peptiden bzw. exprimierten und gereinigten Proteinen entfällt.

### Gewinnung von reinem Protein

In Forschung und Entwicklung, sowie auch in der höher-skalierten Produktion, ist die Gewinnung von hinreichenden Mengen Zielproteinen mit hoher spezifischer Aktivität mittels deren Expression immer noch eine Herausforderung, insbesondere, wenn man die Proteine in einem funktionalen Faltungszustand und gegebenenfalls modifiziert aufreinigen möchte. Hierzu ist es vorteilhaft, das erfindungsgemäße System zu verwenden. Dies kann von großem Vorteil sein, weil die Lokalisation des gewünschten Zielproteins außerhalb der Zelle, im Medium, ein sehr viel weniger komplexes Milieu darstellt als es das Cytosol innerhalb der Zellen darstellt. Dementsprechend ist es leichter anzureichern und zu isolieren. Normalerweise sind die exprimierten Proteine intrazellulär zu finden und werden aus dem Cytosol aufgereinigt.

In einer erfindungsgemäßen Ausführungsform dieser Verwendung kann die Abspaltung membranständigen Proteins, das mit tANCHOR exprimiert wurde, mittels Aminosäure-Sequenzmotiv-spezifischen Proteinasen von der Membran abgespalten werden. Die spezifischen Proteasen können einerseits exogen dem Medium zugeführt oder endogen mit dem Zielprotein ko-exprimiert werden. Dieses Vorgehen würde eventuell die Proform einer sequenzspezifischen Endoprotease erfordern, die im Medium aktiviert wird. In einer zeitlich andauernden Kultivierung der Zellen mit dem Ziel der Isolierung des Proteins erfolgt die Anreicherung von spezifischem Zielprotein schließlich im Medium, weil das von tANCHOR freigesetzte Zielprotein durch den Integritätsverlust des Proteins in die Membran stetig nachgeliefert wird.

### DNA-Vakzinierung als therapeutische Anwendung

In Verbindung mit differenzierungs-spezifischen und/oder speziesspezifischen Promotoren (u.a. im Veterinärbereich), die nur in ganz bestimmten Zielzellen ihre Expression finden, ist das tANCHOR-System aufgrund des effizienten Membrantransports in der Lage, heterologe Genfusionselemente auch in den intakten Zellen eines Organismus zu exprimieren. Diese können exogen in Zellen eingebracht werden. Man kann zur Impfung mit DNA- oder auch RNA-Molekülen Tetraspaningen-Fusionskonstrukte direkt mittels sogenannter *"Gene Guns"* oder auch *"Particle Guns"* mit hoher Geschwindigkeit Zellen beschießen und die getroffenen transfizierten Zellen zur transienten Expression von Fusionsprotein veranlassen. Diese Proteine können Antigene oder sub-spezifische antigene Determinanten, Epitope, darstellen, die zuvor auf ihre Eignung hin getestet wurden. Sowohl für die Bereitstellung von zu diesem Zweck geeigneten diagnostischen bioAssays, wie auch für die therapeutische Vakzinierung kann die tANCHOR-Technologie eingesetzt werden.

Es wird diskutiert, dass DNA in die Genome von mit Genpartikeln getroffenen Zellen integrieren könnten und dort durch die Integration z.B. in ein Tumorrepressorgen gegebenenfalls Schäden verursachen könnten. Die Praxis lehrt aber, dass flankierend zu den erfindungsgemäßen Genkonstrukten sehr ausgedehnte homologe Genbereiche notwendig sind, um wirklich eine höhere und relevante Intergrationsrate zu erzeugen. Solche Elemente sind in den erfindungsgemäßen Konstrukten nicht vorgesehen. Allerdings sind aufgrund solcher Assoziationsketten über die Gefährlichkeit von DNA-Vakzinierungen seitens sensibler Regulatoren die mRNA-Vakzine im Kommen. So ist es erfindungsgemäß auch möglich, beispielsweise mittels *in vitro* Transkription mRNA-Moleküle zu erzeugen, die für die Expression in eukaryotischen Zellen optimiert sind, diese zu stabilisieren und diese auf Nanobeads zu fixieren oder in geeignete Mikro-bis Nanokompartimente (*virus like particles,* VLPs) zu verpacken und die mRNAs in dieser Form therapeutischen Anwendungen zuzuführen.

Durch die spezifischen Eigenschaften der Tetraspaninfusionen werden die dabei exprimierten Proteine auf die Zellmembranen der exprimierenden Zellen transportiert und dort nach außen gezeigt.

Die Exposition von spezifischen Aminosäuresequenzen kann von dem Immunsystem erkannt werden und dort entsprechende, gewünschte Immunreaktionen humorale oder cytotoxische Immunreaktionen hervorrufen.

### Therapeutische Applikationen unter Verwendung isolierter Zellen bzw. Zellmembranen

Zusätzlich ist es möglich, idealerweise wegen der HLA Inkompatibilitätsgruppen und damit verbunden möglichen unerwünschten Immunreaktionen, auch körpereigene Zellen nach der Entnahme *ex vivo,* gegebenenfalls nach oder während einer Zwischenkultivierungsphase, *in vitro* zu transfizieren. Die Transfektion körpereigener Zellen von Probanden (z.B. Menschen aber auch Tiere) mit DNA-Konstrukten basierend auf dem tANCHOR-System wird es ermöglichen, diese in autologen bzw. autogenen Vakzinierungen in Verwendung zu bringen. Dabei ist der Einsatz von intakten und auf Qualität geprüften autologen Zellen, wie auch der von isolierten Zellmembranen aus solchen Zellen möglich. Diese können beispielsweise durch Dichtegradienen Ultrazentrifugation gewonnen werden. Dabei kann das Verhältnis von Epitop zu Gesamtprotein verbessert werden und damit zur Erhöhung der spezifischen Aktivität einer Immunreaktion beitragen. Unspezifische Begleitreaktionen und Autoimmunreaktionen werden vermindert.

Die Verbindung von exprimierten Adjuvantien, die gegebenenfalls mit multiplen tANCHOR-Konstruktionen in einer Zelle ko-exprimiert werden können, ist eine Variante, die ebenfalls im Bereich der Möglichkeiten einer Verbesserung in der Anwendung von erfindungsgemäßen tANCHOR-Systemen liegt.

Die Ko-expression von verschiedenen, die physiko-chemischen Eigenschaften, wie z.B. die Löslichkeit von Membranen verbessernden Proteine könnte die aus den Zellen isolierten Membranen für eine Vakzinierung wesentlich verbessern. Auch natürliche Adjuvantien könnten bei einer Koexpression mit CD63-Antigen/Epitop-Fusionen die Impfantwort verbessern helfen.

Ein weiterer Gegenstand der Erfindung ist ein Kit umfassend
(i) ein Nukleinsäuremolekül oder einen Vektor wie vorstehend beschrieben und
(ii) eine Zelle oder Membran.

Zusätzlich kann ein erfindungsgemäßer Kit abhängig von der beabsichtigten Anwendung verschiedene Komponenten wie z.B. Kontrollplasmide, Kontrollproteine o.ä. und spezielle Plasmide für verschiedene Applikationen enthalten.

Die vorliegende Erfindung soll durch die nachfolgenden Figuren und Beispiele ausführlicher beschrieben werden.

### Figuren und Tabellen

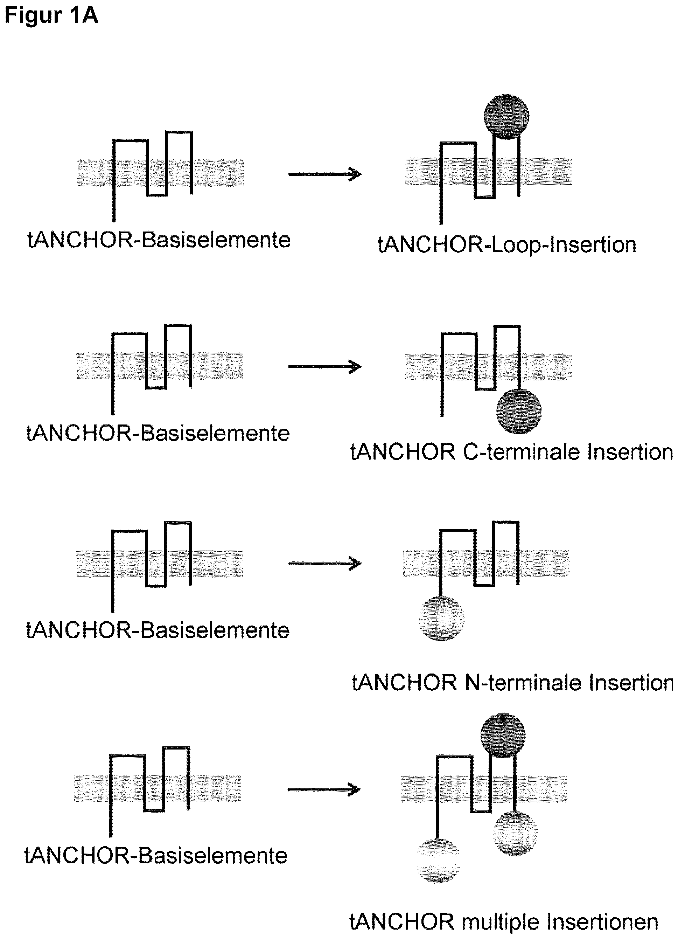

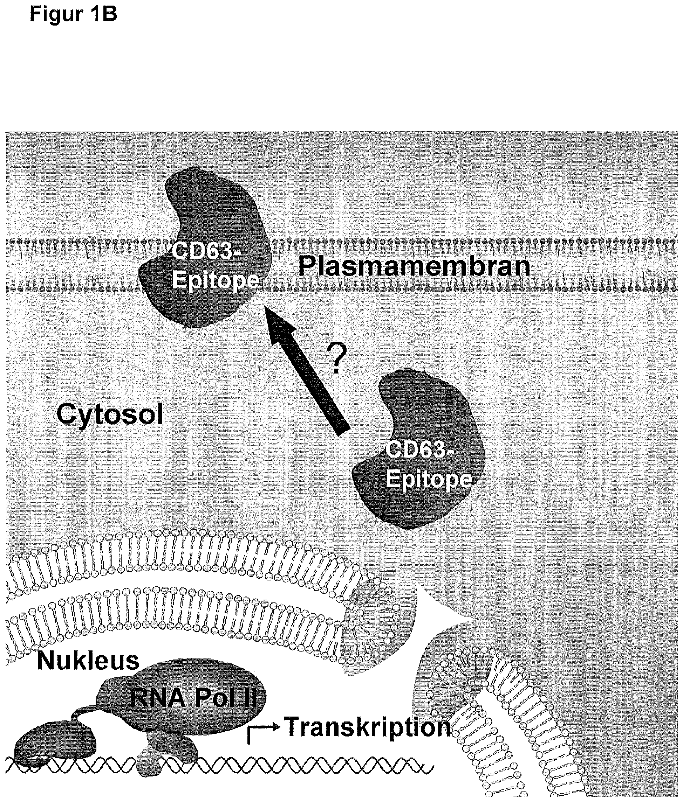

**Fig. 1****: Schematische Darstellung der erfindungsgemäßen Fusionen und Lokalisation der Fusionsproteine.**
   (A) Positionen, die nach Stand der Technik mit Fusionen und Insertionen verschiedenen applikativen Lösungsansätzen dienen können. Das erfindungsgemäße System, auch als tANCHOR-System bezeichnet, besteht aus vier Transmembrandomänen (4TM). Zwischen TM3 und TM4 können für Epitope bzw. Proteine kodierende DNA-Sequenzen einkloniert werden. Am N-terminalen Ende wurde exemplarisch das Epitop FLAG-Tag und am C-terminalen Ende von CD63 wurde beispielsweise das Fluoreszenzprotein mCherry als Transfektionskontrolle zusätzlich im tANCHOR-System durch Genfusion implementiert (s. auch Fig. 2). (B) Mit dem zum STATUS QUO entwickelten tANCHOR-System sind von humanen Zellen exprimierte Epitope bzw. Antigene vernehmlich auf der Plasmamembran lokalisiert zu finden. Das tANCHOR-System ist in der Lage die Fusionsproteine mit einer hohen Effizienz zur Plasmamembran zu leiten und dort zu verankern.
**Fig. 2A****: Schematischer Aufbau des beispielhaften tANCHOR-Vektorsystems.** Das erste beispielhafte tANCHOR-Vektor-System baut auf dem modularen multi-Expressionsvektor **pA4E211** (SEQ ID NO: 1) und funktionalen Derivaten dieses Vektors auf. Zusätzlich ist eine Übersicht der Sequenzanordnung der CD63ΔLEL Bestandteile (Figur 2B) sowie die N-terminale Fusion des FLAG-Epitops sowie C-terminale Fusionierung des fluoreszierenden Reporterproteins wie z.B. mCherry oder GFP aus *Pontellina plumata* dargestellt. 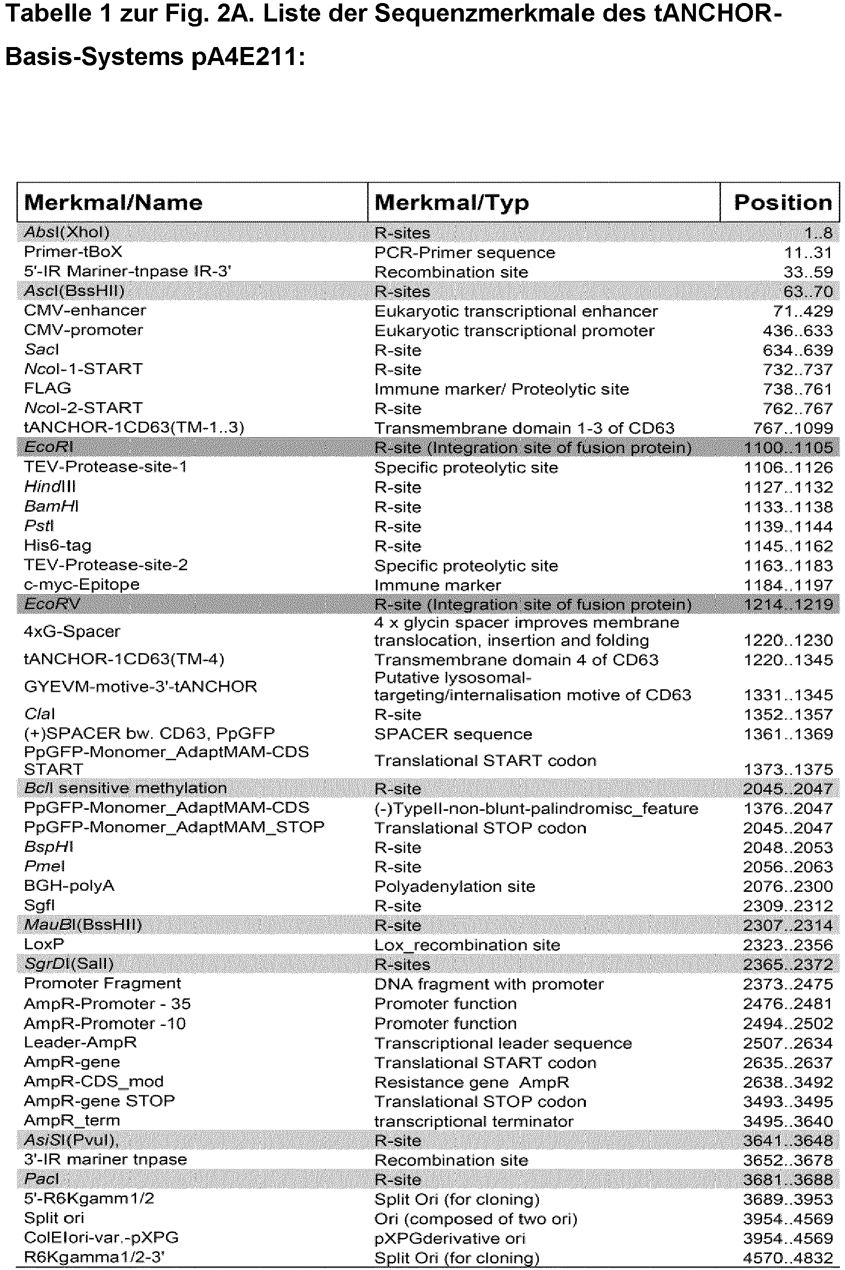
**Fig. 2B****: Ableitung der CD63ΔLEL-Sequenz (SEQ ID NO: 3) aus der kodierenden wildtypischen (WT) Sequenz von CD63 (SEQ ID NO: 17).** Die wildtypische CD63-Proteinsequenz besteht aus 238 Aminosäuren. Für die Generierung von CD63ΔLEL wurde die kodierende DNA-Sequenz der Transmembrandomänen 1-3 sowie 4 (unterstrichene Sequenz CD63 TM1-3, CD63 TM4) verwendet. Der Linker, der die Sequenz für den LEL von CD63 nicht enthält (ΔLEL), wird zwischen dem Fragment CD63 TM1-3 und CD63 TM4 platziert (SEQ ID NO: 42). Zusätzlich ist das GGGG-Strukturmotiv im Linker eingefügt. Zwischen der Gensequenz CD63ΔLEL und der Gensequenz für das Fluoreszenzprotein kann eine Gensequenz (SEQ ID NO: 45) für einen Linker mit der Aminosäure-Sequenz KLIDTVDLEK (SEQ ID NO: 46) umfasst sein.
   **Tabelle 2 zu** **Fig. 2A****: Lineare Organisation der modular aufgebauten tANCHOR-Kit-Vektorsysteme der Typ-I-Serie: pA4E211**
   (0) *Abs*I-*Asc*I-IFLAGICDXX:Epitop |Reporter |-MauBI-SgrDI- SELEKTOREN -*Asi*SI-*Pac*I
   (1) *Abs*I-*Asc*I-|FLAG| ----:------ |--------- |-*Mau*BI-*Sgr*DI-Puromycin R -*Asi*SI-*Pac*I
   (2) *Abs*I-*Asc*I-|FLAG|CD63:------- |--------- |-*Mau*BI-*Sgr*DI-Puromycin R -*Asi*SI-*Pac*I
   (3) *Abs*I-*Asc*I-|FLAG|CD63: gp41 |--------- |-*Mau*BI-*Sgr*DI-Puromycin R -*Asi*SI-*Pac*I
   (4) *Abs*I-*Asc*I-|FLAG|CD63: eYFP |eGFP-Gen|-*Mau*BI-*Sgr*DI-Puromycin R -*Asi*SI-*Pac*I
   (5) *Abs*I-*Asc*I-|FLAG|CD63:bio*Pep*ID|eGFP-Gen |-*Mau*BI-I-Puromycin R -*Asi*SI-*Pac*I
   (6) *Abs*I-*Asc*I-|FLAG|CD63:bio*Poi*ID|YFP-Gen |-MauBI-SgrDI-Puromycin R -*Asi*SI-*Pac*I
   (7) *Abs*I-*Asc*I-|FLAG|CD63:bio*Poi*ID|POI-Fusion|-*Mau*BI-*Sgr*DI-Puromycin R -*Asi*SI-*Pac*I

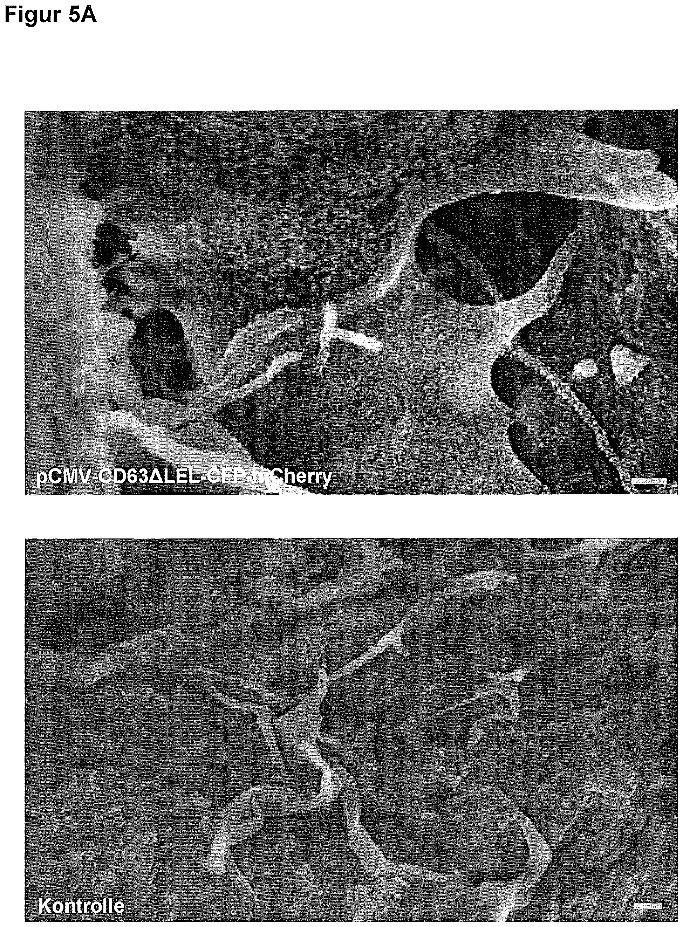

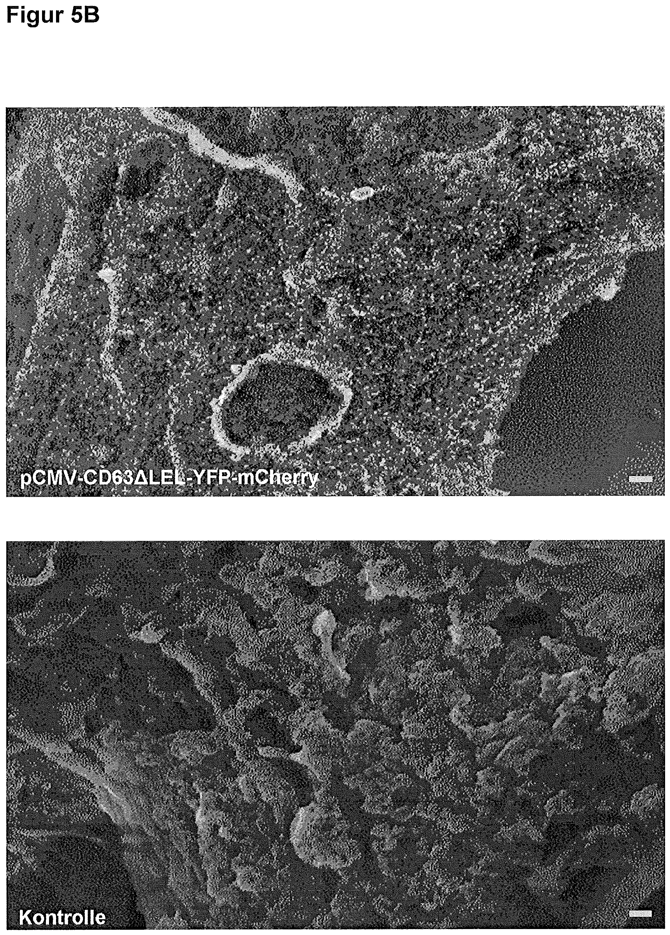

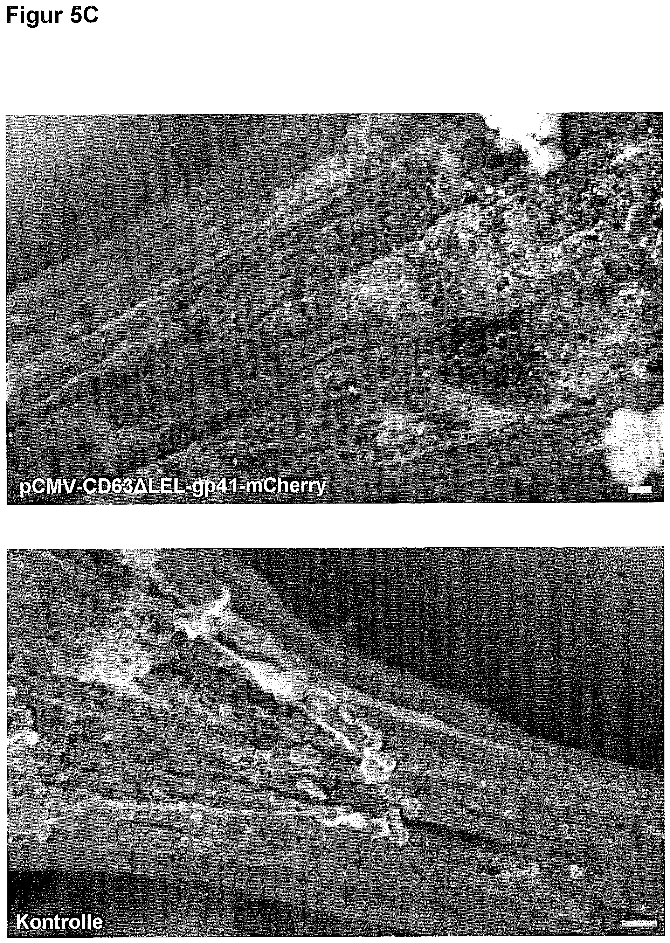

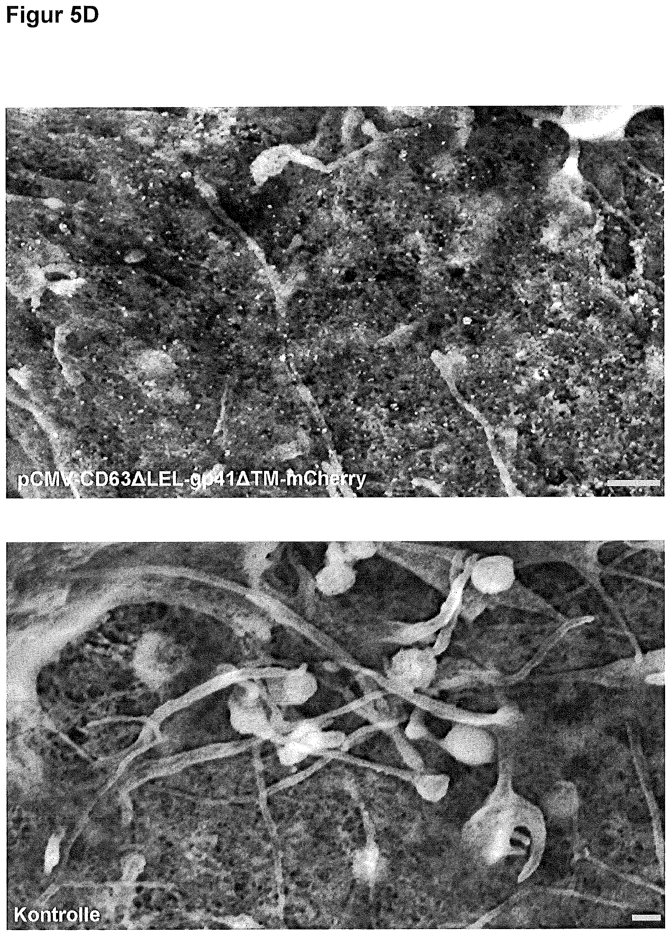

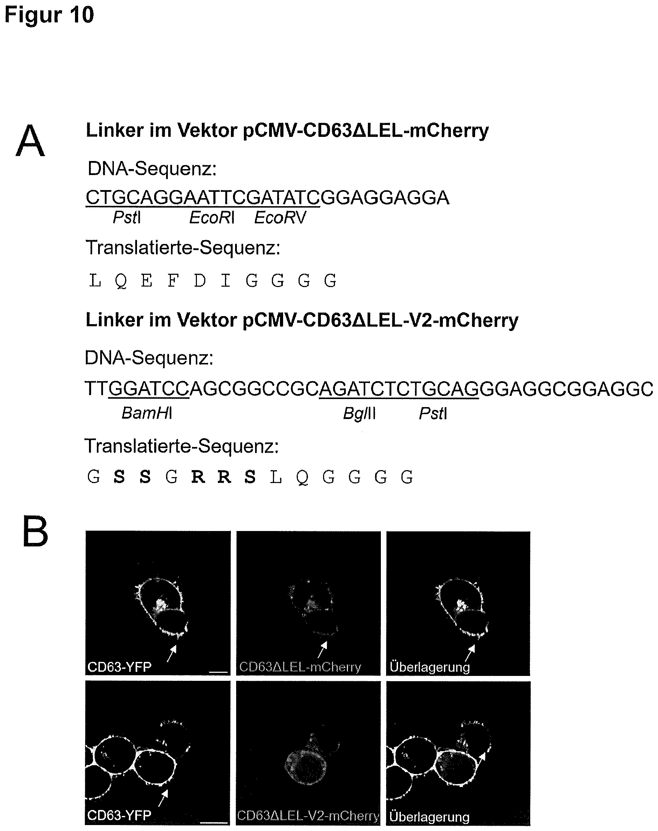

   Positive und negative Kontrollvektoren für das tANCHOR-CD63 Protein-Präsentationssystem (Display-System). **(0)** Formale Darstellung der funktionalen Elemente der tANCHOR-Technologie: **CDXX-** (=verschiedene Tetraspanin Proteinspezies) CDS (=kodierende Sequenz) **(1) tANCHOR-**Vektor ohne **CD63-Gen,** der internen CD63-Insertion und dem terminalen Reporter **(2) tANCHOR-Vektor** mit dem **CD63-Gen,** ohne interne CD63-Insertion und terminalen Reporter des **tANCHOR-**Vektors mit **CD63-Gen** und mit z.B. **FLAG-Tag-Epitop** als interne CD63-Insertion und keinen terminalen Reporter **(3) tANCHOR-**Vektor mit dem **CD63-Gen,** und dem **gp41-Epitop** als interne CD63-Insertion sowie dem terminalen Reporter des **tANCHOR-**Vektors mit **CD63-Gen (4) tANCHOR-**Vektor mit z.B. **CD63-Gen** und dem **YFP-Epitop** als interne CD63-Insertion und mit dem C-terminalen Reporter **GFP** (eGreen-Fluorescent-Protein-Gen). **(5) tANCHOR-**Vektor mit z.B. einem zur Analyse eingesetzten **POI-bio*Pep*ID-Epitope(s) (Protein/Peptide of interest)** als interne CD63-Insertion und mit dem C-terminalen Reporter **eGFP** (eGreen-Fluorescent-Protein-Gen), wie auch (6) mit zusätzlichen Reportergenen, wie **YFP** (Yellow-Fluorescent-Protein-Gen) und mit Fusionen des Proteins des jeweiligen experimentellen Interesses **(POI-Fusion). (7)** mit Fusionen des **POI-Fusion** etc. je nach dem experimentellen Design. bio*Pep*ID sind bioPeptid-Bibliotheken des *Pep*ID-Typs (EP09000893.9). Diese können im tANCHOR-System verwendet werden. ***Abs*I*-SgrD*I** und ***Asc*I*-Mau*BI** wie auch **AsiSI-PacI** sind verschachtelte und untereinander kompatible Restriktionsendonukleasen mit acht-meriger Erkennungssequenz für das Multiplexing der Expression von multiplen Genen. Jede andere Realisierung der erfindungsgemäßen Anwendungen ist ebenso von der Patenschrift abgedeckt.
**Fig. 2C****: Beispiele verschiedener erfindungsgemäßer Linkervarianten in Kombination mit CD63ΔLEL in dem beispielhaften Vektor pAE211_CMV-P-CD63ΔLEL.** Die Restriktionsschnittstellen *EcoRI, EcoRV, Hind*III*, BamH*I und *Pst*I sind für die Insertion von mehreren hintereinander folgenden Epitop-Einheiten nach einem Klonierungsstandardverfahren aber auch Klonierungsverfahren zur nahtlosen Klonierung möglich. **(1) Seq1,** nur die erfindungsgemäßen und auf Funktion hin getesteten Restriktionsschnittstellen **(R-sites) Eco*R*I** und **EcoRV** und **n*N=**Abstandshaltersequenz **(2)** Seq(1) plus **His6-Tag, (3)** Seq(1) plus **C-Myc Epitope, (4) Seq(1)** plus **His6-Tag** plus **C-Myc (5) TEV**-Proteolysestellen, die zentralen Klonierungs stellen einschließend, um das exprimierte Fremdprotein selektiv abzuernten. **(6)** alle in **(8)** genannten translatierten und nicht-translatierten molekularen Funktionselemente in einem Konstrukt vereint (SEQ ID NOs: 39, 40). **(9)** Minimalinker zur für den Transport zur Zelloberfläche (SEQ ID NOs: 41, 42).

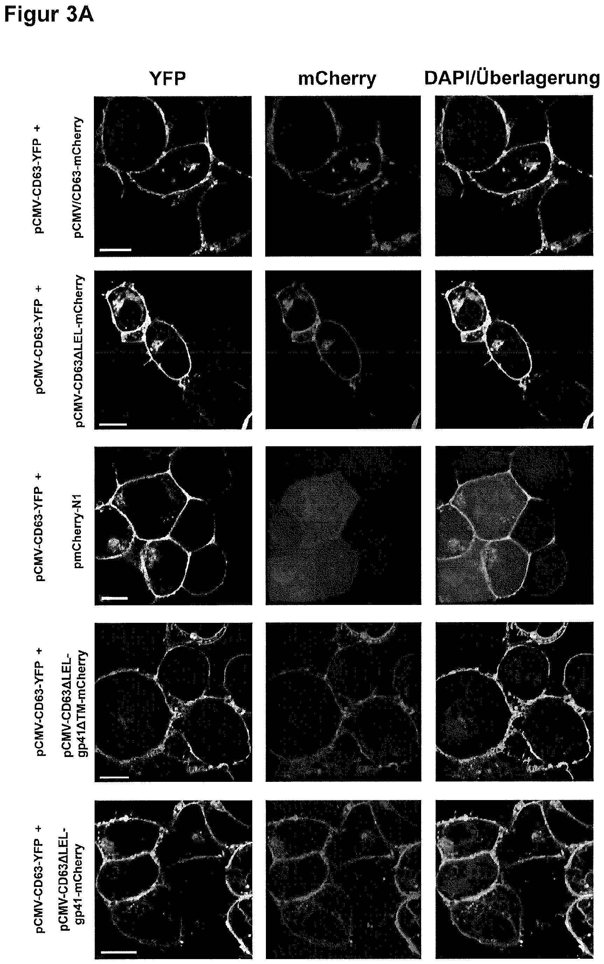

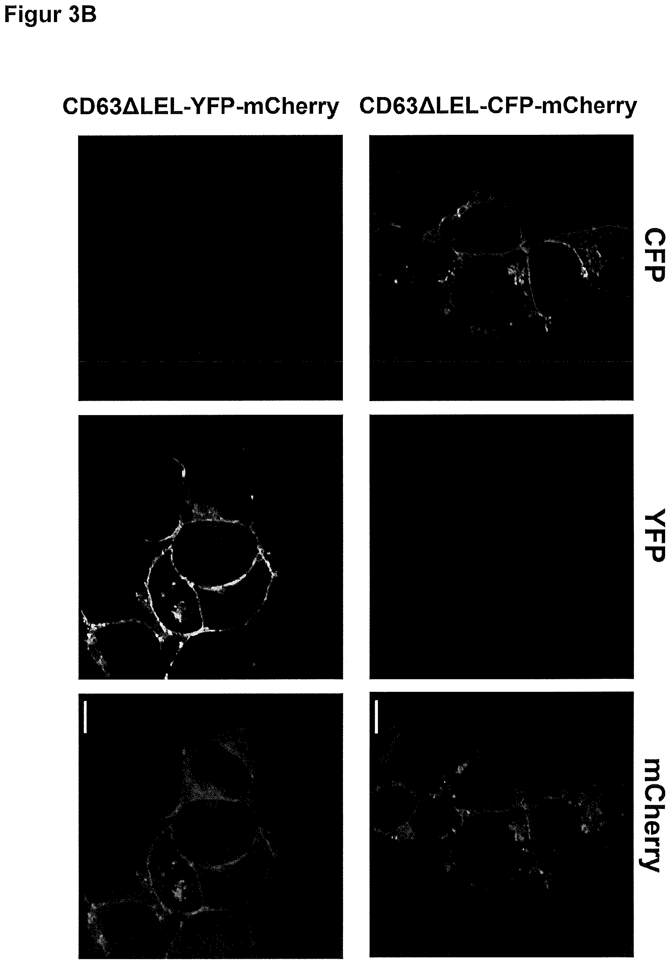

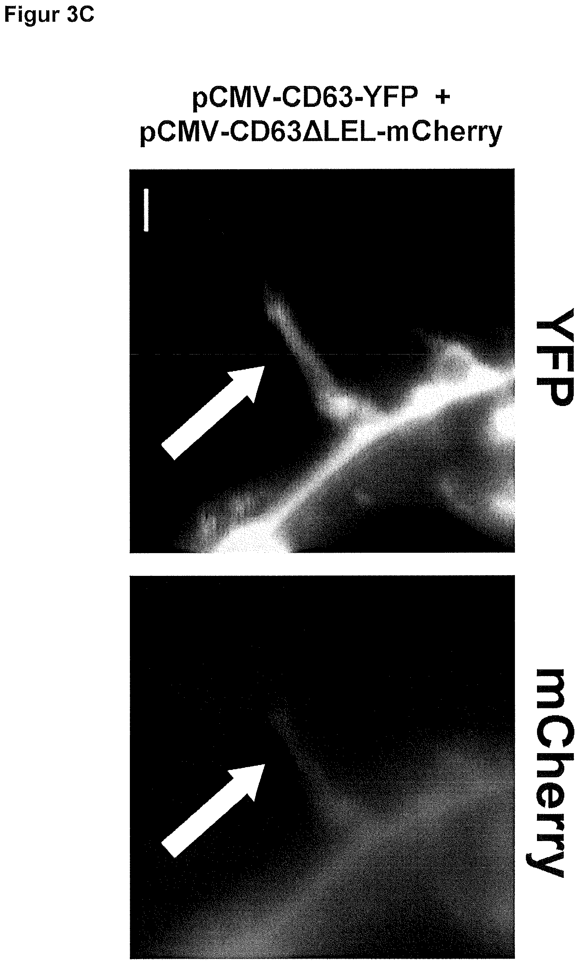

**Fig. 3****: Lokalisierung von Fusionsproteinen auf der Oberfläche von HEK293T-Zellen mittels cLSM.**
   **(A)**HEK293T-Zellen wurden mit den jeweiligen Vektoren kotransfiziert. Der Vektor pCMV-CD63-YFP diente bei den Lokalisationsstudien als Kontrolle für das exprimierte wildtypische CD63-YFP Protein. Für alle Proteine, die die tANCHOR-Technologie (CD63ΔLEL, Fig. 2B) enthielten, konnte eine prädominante Lokalisierung auf der Zelloberfläche durch das fusionierte Reporterprotein mCherry detektiert werden. Das mCherry-Protein ohne tANCHOR-Fusion ist cytoplasmatisch, gleichmäßig verteilt. Es zeigt keine Prädominanz auf der Zelloberfläche. Zusätzlich können weitere Proteine wie z.B. das Glykoprotein gp41 von HIV-1 mit und ohne Transmembrandomäne (gp41ΔTM) auf der Oberfläche präsentiert werden. **(B)** Die Proteinfaltung wurde durch CFP und YFP Lokalisation auf der Oberfläche überprüft. Beide Fluoreszenzproteine können nach Anregung durch den Dioden/Argon-Laser bei 405 nm (CFP) bzw. 514 nm (YFP) eines für das Fluorophor spezifischen Emissionswellenlänge detektiert werden. **(C)** Das exprimierte Protein mCherry, welches normalerweise verteilt im Cytosol vorkommt, wird durch das tANCHOR-System in die Plasmamembran mit hoher Effizienz geleitet. Das mCherry mit implementierter tANCHOR-Technologie wird dadurch sogar in den Filopodien (weißer Pfeil) der HEK293T-Zellen wie beim wildtypischen CD63-YFP detektierbar. Länge der Maßstabsbalken beträgt in den Fig. en 3A,3B = 10 µm bzw. bei der Fig. 3C = 1 µm.

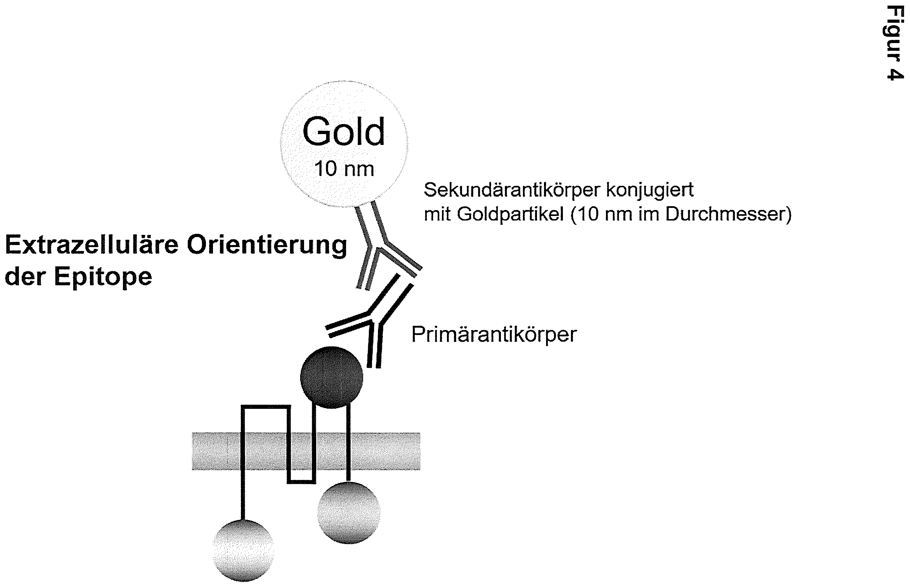

**Fig. 4****: Schematische Darstellung der extrazellulären Orientierungsüberprüfung der Epitope**
   Die Detektion der Epitoporientierung wurde mittels Rasterelekronenmikoskopie (REM) auf der Oberfläche von transfizierten HEK293T durchgeführt. Die Proteine wurden mittels Primärantikörper gebunden und mittels konjugierter Sekundärantikörper mit Goldpartikel (10 nm) visualisiert. Bei den Modellepitopen YFP, CFP, gp41 und gp41ΔTM konnten gebundene Antikörper auf der Oberfläche durch Immunogold-Partikel visualisiert werden (Fig. 5).
**Fig. 5A****-D: REM Aufnahme von Gold-Partikeln (10 nm) auf der Oberfläche von HEK293T-Zellen.**
   Die transfizierten HEK293T-Zellen wurden jeweils auf Epitoporientierung hin untersucht. Hierbei wurden die jeweiligen Epitope mit spezifischen Primärantikörpern gebunden und diese dann mit Gold-konjugierten Sekundärantikörpern nachgewiesen. Für alle Epitope mit der tANCHOR-Technologie konnte den Epitopen eine extrazelluläre Orientierung zugeschrieben werden. Länge der Maßstabsbalken beträgt in allen Figuren 300 nm.

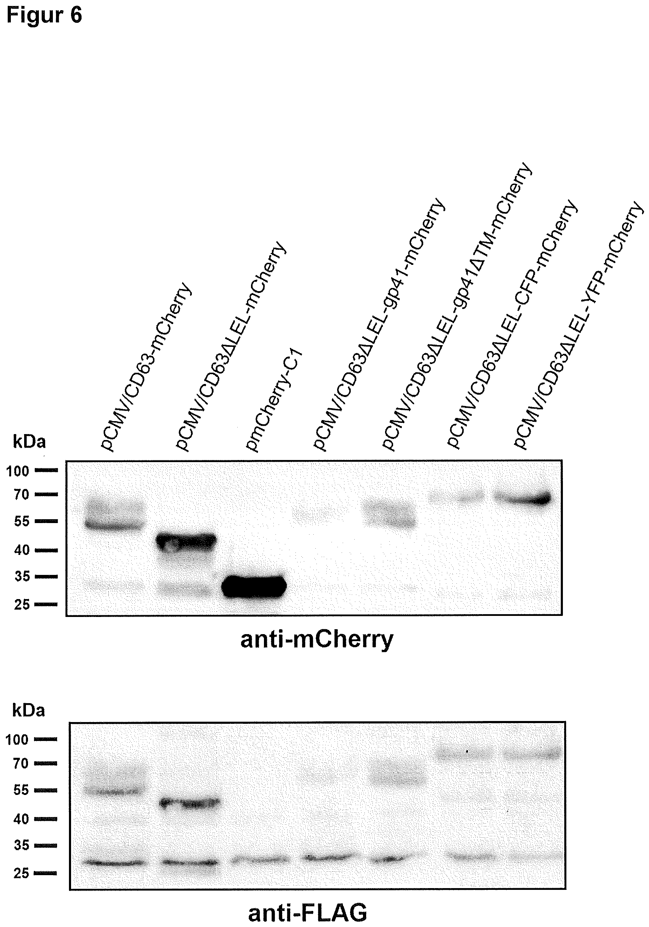

**Fig. 6****: Western-Blot-Analyse von transfizierten HEK293T-Zellen mit generierten tANCHOR-Vektoren.**
   Die transfizierten HEK293T-Zellen wurden einer Western-Blot-Analyse unterzogen. Die exprimierten Proteine in den aufgetrennten Zelllysaten wurden mittels Primärantikörper gerichtet gegen das C-terminale Reporterprotein mCherry und gegen das N-terminale Epitop FLAG nachgewiesen. Die Tansfektion mit dem Vektor pmCherry-N1 zeigt keine Bande bei der Inkubation mit anti-FLAG Antikörper, da dieser Vektor keine Sequenz für das FLAG-Epitop enthält und dient als eine Kontrolle für den spezifischen Nachweis der exprimierten Proteine.

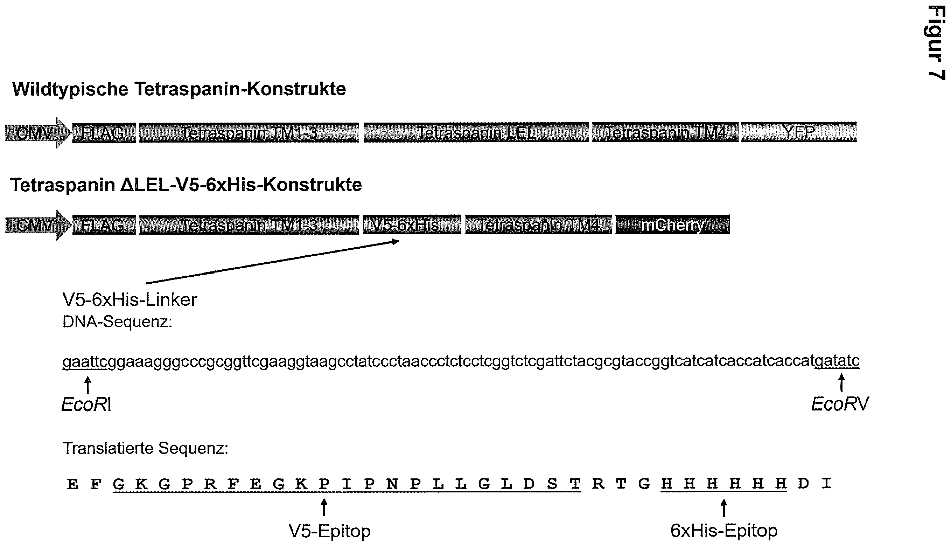

**Fig. 7****: Schematische Darstellung der Konstrukte zur Überprüfung weiterer Tetraspanine auf Proteintransport mit interner Fusionsstelle mit V5-6xHis-Linker.**
   Zur Überprüfung der Gültigkeit des Proteintransports unabhängig der großen extrazellulären Schleife innerhalb der Tatraspanin-Superfamilie wurden Expressionsvektoren generiert, die eine interne Fusion V5-6xHis zwischen der 3. und 4. Transmembrandomäne gemäß der tANCHOR-Prototyp-Konstrukte basierend auf CD63ΔLEL beinhalten. Die interne Fusion V5-6xHis beinhaltet den Linker zwischen *EcoR*I und *EcoR*V (SEQ ID NOs: 35, 36). N-terminal ist eine FLAG-Fusion integriert, C-terminal sind die Fluoreszenzproteine YFP bei wildtypische Tetraspanin-Konstrukten bzw. mCherry bei ΔLEL-V5-6xHis-Konstrukten als Reporterproteine fusioniert.

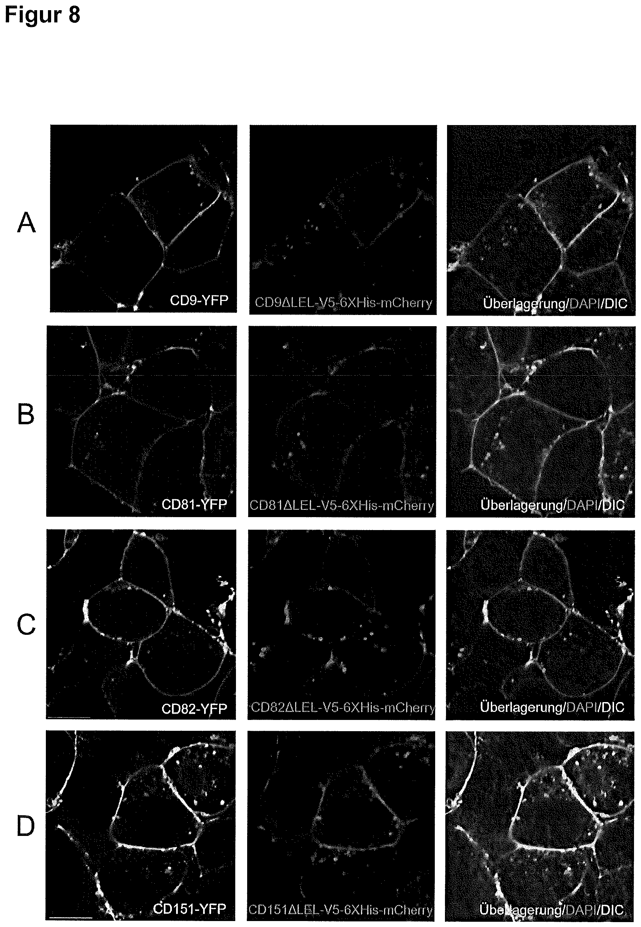

**Fig. 8****: Lokalisation von Fusionsproteinen auf der Oberfläche von HEK293T-Zellen.**
   HEK293T-Zellen wurden mit den generierten Vektoren (A) pCMV-CD9-YFP/pCMV-CD9ΔLEL-V5-6xHis-mCherry, (B) pCMV-CD81-YFP/pCMV-CD81ΔLEL-V5-6xHis-mCherry, (C) pCMV-CD82-YFP/pCMV-CD82ΔLEL-V5-6xHis-mCherry und (D) pCMV-CD151-YFP/ pCMV-CD151ΔLEL-V5-6xHis-mCherry kotransfiziert. Alle exprimierten Proteine können mittels Fluoreszenz des Reporterproteins mCherry dominant auf der Zelloberfläche detektiert werden. Das Konstrukt, welches die interne Fusion mit dem Linker V5-6xHis zwischen der 3. und 4. Transmembrandomäne (Fig. 7) der Tetraspanine CD9, CD81, CD82 und CD151 beinhalten, kolokalisieren mit den entsprechenden exprimierten wildtypischen Tetraspaninen mit der Fusion des Fluoreszenzproteins YFP. Länge der Maßstabsbalken beträgt in allen Abbildungen 10 µm.
**Fig. 9****: Anwendung von des tANCHOR-Systems zur Detektion von Antikörpern**
   (A) Ergebnisse der ELISA-Experimente⁵³ mittels tANCHOR-System im 96-Well-Format. Das V5-Epitop auf der Zelloberfläche von HEK293T-Zellen wurde mit HRP-konjugierten anti-V5 Antikörpern detektiert. Die Proteinexpression der tANCHOR-Proteine kann konzentrationsabhängig und signifikant durch anti-V5-HRP Antikörper detektiert werden. (B) Schematische Abbildung der Linker 2F5-4E10 zwischen der 3. Und 4. Transmembrandomäne von CD63 (SEQ ID NOs: 37, 38). Die Linker wurden in den Vektor pCMV-CD63ΔLEL eingeführt. (C) Die HIV-1 neutralisierenden Antikörper anti-2F5 und anti-4E10 wurden bei einer konstanten DNA-Menge von 0,6 µg per Well transfizierten HeLa-Zellen primer an das 2F5- bzw. 4E10-Epitop gebunden. Die gebundenen anti-2F5 bzw. anti-4E10 Antikörper konnten signifikant detektiert werden, (E) exprimierte Fusionsproteine CD63ΔLEL-V5-6xHis sowie CD63ΔLEL-2F5-4E10 konnten zu Kontrolle dominant auf der Oberfläche von HEK293T-Zellen lokalisiert werden. Die Länge der Maßstabsbalken beträgt in allen Abbildungen 10 µm.
**Fig. 10** **Effizienzsteigerung durch Verwendung von serin- und argininarmen Linkern.**
   (A) Die verwendeten Linker sind auf DNA- und Aminosäure-Sequenz Ebene dargestellt. Die Analyse der subzellulären Lokalisation der Fusionsproteine mit zwei verschiedenen Linkern (SEQ ID NOs: 41, 42 bzw. SEQ ID NOs: 43, 44) zeigt deutlich, dass die Verwendung von *EcoR***I**/*EcoR***V** flankierenden Restriktionsschnittstellen zu einer hohen Effizienz des Proteintransport zur Oberfläche der HEK293T-Zellen führt und das Fusionsprotein an den ausgebildeten Filopodien (weißer Pfeil) der Zellen detektierbar ist (B). Die Länge der Maßstabsbalken beträgt in allen Abbildungen 10 µm.

### 1. Generierung von CD63-basierten Modelvektoren zur Durchführung von Lokalisationsexperimenten

Die Generierung von CD63-basierten Modellvektoren basierte auf der Verwendung des Vektors pCMV-Tag2B (Stratagene). In diesen Vektor wurden mittels gängiger Klonierungstechniken die jeweiligen DNA-Sequenzen über Restriktionsschnittstellen eingebracht ³⁹. Der Kontrollvektor pCMV-CD63-YFP bzw. als pCMV-CD63-YFP-FLAG bezeichnet ⁴⁹, der die Volllängensequenz von CD63 enthält (Aminosäuren 1-238, GenBank accession no. KF998086) wurde verwendet, um über die Restriktionsschnittstellen *Xho*I und *Apa***I** die DNA-Sequenz von dem verwendeten rot fluoreszierenden Reporterproteins mCherry mittels der Primer PA1-01/PA1-02 und dem Matrizen-Vektor pmCherry-N1 (Clontech) einzuführen. Die Teilsequenz mit den Transmembrandomänen TM1-3 (Aminosäuren 1-110 GenBank accession no. KF998086) wurde über die Restriktionsschnittstellen *BamH***I** und *Pst***I** mit Hilfe der Primer PA1-03/PA1-04 eingebracht und anschließend wurde die TM4 (Aminosäuren 201-238, GenBank accession no. KF998086) über EcoRV und *Hind***III** mithilfe der Primer PA1-05/PA1-06 sowie dem Matrizen-Vektor pPR3-N-CD63 ⁴⁹ in den Vektor pCMV-Tag2B eingebracht. Der resultierende Vektor enthält die CD63 Gensequenz ohne LEL (pCMV-CD63ΔLEL) (Fig. 2B).

Zur vereinfachten Detektion der Proteine wurde über die Restriktionsschnittstellen *Xho***I** und *Apa***I** das restringierte mCherry-DNA-Fragment vom Vektor pCMV-CD63-mCherry in den restringierten Vektor pCMV-CD63ΔLEL ligiert. Die Vektoren pCMV-CD63ΔLEL-CFP und pCMV-CD63ΔLEL-YFP wurden über die Restriktionsschnittstellen EcoRI und EcoRV mit Hilfe der Primer PA1-07/PA1-08 sowie dem Matrizen-Vektor pSCFP3A-C1 bzw. pSYFP2-C1 ⁵⁰, generiert. In gleicher Weise wurden die Vektoren pCMV-gp41ΔTM und pCMV-gp41 mit Hilfe der Primer PA1-09/PA1-010 bzw. PA1-09/PA1-11 und des Matrizen-Vektors pNL4-3⁵¹, welcher die Gensequenz des gp41-Proteins von HIV-1 enthält, generiert.

### 2. Transfektion und konfokale Laser-Scanning-Mikroskopie (cLSM)

Zur Überprüfung der Lokalisation exprimierter Fusionsproteine wurden die in Beispiel 1 generierten Plasmide in HEK293T-Zellen mittels Transfektionsreagenz transfiziert. Hierbei wurden HEK293T (*human embryonic kidney cells* 293T) Zellen in ibiTreat 8-Wells ausgesät und in Dulbecco's modified Eagle's Medium, komplementiert mit 10% fötalem Kälber Serum, L-Glutamin und Penicillin/Streptomycin, kultiviert. Bei einer ca. 50%igen Konfluenz wurden die Zellen mittels Metafectene®PRO (Biontex) mit den generierten Plasmiden nach Herstelleranweisung transfiziert. Nach 24 h wurde die transfizierten Zellen in 2% Paraformaldehyd in PBS fixiert, in PBS zweimalig gewaschen und in 90%igem Glycerin in PBS mit 0,1% p-Phenylenediamin und DAPI (4',6-Diamidin-2-phenylindol) zur Visualisierung des Nukleus aufgenommen. Die transfizierten Zellen wurden mit einem inversen, konfokalen Lasermikroskop LSM 780 (Carl Zeiss) auf subzelluläre Lokalisation hin untersucht (Fig. 3A-3C). Die Einstellungen der Anregungs- und Emissionswellenlängen wurden mittels Smart-Setup-Option der LSM 780 Software ZEN 2010 für die Fluoreszenzproteine CFP, YFP und mCherry gewählt.

### 3. Orientierungsanalyse der Epitope auf der Oberfläche von HEK293T-Zellen mittels Rasterelektronenmikroskopie

Die exprimierten Proteine wurden mittels Rasterelektronenmikroskopie von Gold-immunmarkierten Proteinen auf der Plasmamembran von HEK293T-Zellen nachgewiesen (Fig. 4). Hierzu wurden die transfizierten und in 2% Paraformaldehyd in PBS fixierten Zellen dreimalig in PBS gewaschen und mit 0,5% BSA/0,1 % Gelatine in PBS geblockt. Die Proteine CD63ΔLEL-CFP und CD63ΔLEL-YFP wurden mit Kaninchen anti-GFP (ab6556, Abcam) inkubiert, gewaschen und die Primärantikörper mit Ziege anti-Kaninchen mit konjugierten 10 nm Gold-Partikel detektiert. Der Immunmarkierung schloss sich eine Nachfixierung mit Glutaraldehyd (2,5% in HEPES 0,05M) an. Es folgte die Rasterpräparation. Dazu wurden die Proben in Ethanolstufen (30 %, 50 %, 70 %, 90 %, 96 %) für jeweils 15 min schrittweise entwässert und in absoluten Ethanol für 30 min belassen, in HMDS (Hexamethyldisilazan) überführt, aus HMDS getrocknet und die Probenoberfläche mit Kohle bedampft. Die Goldpartikel wurden mit dem Leo 1530 Gemini Elektronenrastermikroskop mittels Rückstreudetektor (Centaurus) visualisiert. In gleicher Weise wurde das gp41 -Protein mittels Human anti-2F5 Antikörpers (National Institutes of Health, NIH) sowie unter Verwendung des Antikörpers Ziege anti-Human mit konjugierten IgG H&L 10 nm Gold-Partikel (BBInternational) detektiert (Fig. 5A-5D).

### 4. Western Blot-Analyse exprimierter Fluoreszenzproteine

Zur Überprüfung der Proteinexpression wurden Zelllysate transfizierter HEK293T-Zellen einer Western-Blot-Analyse unterzogen. Hierbei wurden HEK293T-Zellen mit den Vektoren nach Herstelleranweisung im 6-Well-Format mit METAFECTENE®PRO transfiziert. Nach 48 h wurde das Medium vollständig entfernt und in jedes 6-Well 100 µl Laemmli-Probenpuffer 2x sowie 25 U einer Endonuklease aus *Serratia marcescens* (Benzonase®) zugegeben. Die Zellen wurden lysiert und nach etwa 10 min zu den Proben 5 µl β-Mercaptoethanol zugegeben und bei 95°C für 5 min denaturiert. Diese Zelllysate wurden mittels SDS-PAGE aufgetrennt, auf eine PVDF-Membran transferiert und mit Maus anti-mCherry (ab125096, Abcam) bzw. Ziege anti-FLAG (NB600-344, NovusBio) Antikörpern sowie HRP-konjugierten anti-Maus/anti-Ziege Antikörpern (Dako) und die gebundenen HRP-konjugierte Antikörper mittels Pierce ECL-Western-Blot-Substrat (Thermo Fisher Scientific) nachgewiesen. Die PVDF-Membran wurde mit Roti®-Free Stripping Buffer 2.2 plus (Carl Roth) behandelt um dieselben Proteine mit einem weiteren primären Antikörper detektieren zu können (Fig. 6).

### 5. Überprüfung der subzellulären Lokalisation Tetraspanin-basierter Hybridproteine aus der Tetraspanin-Superfamilie

Zur Überprüfung der Peptidpräsentation auf der Zelloberfläche innerhalb der Tetraspanin-Superfamilie wurden Expressionsvektoren gemäß der schematischen Abbildung (Fig. 7) mittels Gensynthese⁵⁴ (ATG:Biosynthetics) sowie standardisierten Klonierungsmethoden generiert. Die Gensequenzen von CD9 (SEQ ID NO: 19), CD81 (SEQ ID NO: 21), CD82 (SEQ ID NO: 23) und CD151 (SEQ ID NO: 25) wurden in den Vektor pCMV-CD63-YFP bzw. die Gensequenzen für CD9ΔLEL-V5-6xHis (SEQ ID NO: 27), CD81ΔLEL-V5-6xHis (SEQ ID NO: 29), CD82ΔLEL-V5-6xHis (SEQ ID NO: 31) und CD151ΔLEL-V5-6xHis (SEQ ID NO: 33) in den Vektor pCMV-CD63-mCherry (siehe 1: Generierung von CD63-basierten Modellvektoren zur Durchführung von Lokalisationsexperimenten) über die Restriktionsschnittstellen Notl/Xhol gegen die Tetraspaninsequenz von CD63 ausgetauscht und die folgenden Expressionsvektoren wildtypischer Gensequenz pCMV-CD9-YFP, pCMV-CD81-YFP, pCMV-CD82-YFP, pCMV-CD151-YFP sowie Deletionsmutanten der großen extrazellulären Schleife (ΔLEL) mit dem internen Linker V5-6xHis pCMV-CD9ΔLEL-V5-6xHis-mCherry, pCMV-CD81ΔLEL-V5-6xHis-mCherry, pCMV-CD82ΔLEL-V5-6xHis-mCherry und pCMV-CD151ΔLEL-V5-6xHis-mCherry wie im Absatz 2 Transfektion und konfokale Laser-Scanning-mikroskopie (cLSM) beschrieben, transfiziert und mittels cLSM auf subzelluläre Lokalisation hin untersucht (Fig. 8).

### 6. Anwendung des tANCHOR-Systems zur Detektion von Antikörpern mittels ELISA-Verfahrens⁵³

Die hocheffiziente Oberflächenexpression von Fusionsproteinen mittels tANCHOR-Systems eignet sich zur Detektion von gebundenen Antikörpern an präsentierten Peptiden. Als Modell wurde der Linker V5-6xHis über EcoRI und EcoRV aus dem Konstrukt pCMV-CD9ΔLEL-V5-6xHis-mCherry isoliert und in den Vektor pCMV-CD63ΔLEL-mCherry über standard Klonierungsmethoden wie im Abschnitt 1 Generierung von CD63-basierten Modellvektoren zur Durchführung von Lokalisationsexperimenten beschrieben, eingefügt. Ebenfalls wurden die Epitope 2F5 und 4E10 (HIV-1) in den Vektor pCMV-CD63ΔLEL-mCherry mittels der Primer PA1-12/PA1-13 und den Matrizen Vektor pNL4-3 eingeführt (Fig. 9B). Zur Detektion von gebundenen Antikörpern auf der Oberfläche von (Fig. 9A) HEK293T wurden hierfür HEK293T-Zellen bzw. (Fig. 9C) HeLa-Zellen im 96-well-Format ausgesät mit den Plasmide pCMV-CD63ΔLEL-V5-6xHis-mCherry (Fig. 9A) bzw. pCMV-CD63ΔLEL-2F5-4E10-mCherry (Fig. 9C) mittels Metafectene®PRO nach Herstelleranweisung transfiziert. Die Zellen wurden nach 24 h mit 4% Paraformaldehyd (Carl Roth) für 20 min fixiert, 2 mal mit PBS gewaschen und über Nacht mit einer Lösung aus 3% Bovines Serumalbumin Grade V (Carl Roth) und 2% Hühnerei-Albumin (Carl Roth) geblockt. Danach wurden die Zellen 1 x mit PBS gewaschen und mit verdünnten Antikörper (Fig. 9A) anti-V5-HRP (Invitrogen) bzw. (Fig. 9C) Human anti-2F5/Human anti-4E10 (NIH) in einer Verdünnung von 1:3000 für 1 h inkubiert. Bei der Figur 9C wurden die primären Antikörper mittels Hase anti-Human-HRP (DAKO) in einer Verdünnung von 1:1000 gebunden und beide ELISA-Varianten mit 3,3',5,5'-Tetramethylbenzidin als ELISA-Substrat (TMB-Substrat Kit, Thermo Fisher Scientific) nach Herstelleranweisung verwendet und die OD-Werte bei 450 nm mit dem Spektralphotometer Multiscan™GO mit 96-Well-Platteneinsatz (Thermo Fisher Scientific) gemessen. Die Expression der Fusionsproteine wurde mittels cLSM wie im Abschnitt 1 Generierung von CD63-basierten Modellvektoren zur Durchführung von Lokalisationsexperimenten detektiert.

### 7. Effizienzsteigerung des Proteintransports zur Oberfläche von humanen Zellen durch Verwendung von flankierenden EcoRI/EcoRV-Restriktionsschnittstellen beim tANCHOR-System

Der Einsatz von flankierenden *EcoR*I/*EcoR*V-Restriktionsschnittstellen beim tANCHOR-System erhöht die Transporteffizienz zur Zelloberfläche. Das Einbringen von weiteren Restriktionsschnittstellen in den Minimallinker (L Q E F D I G G G G) führt zur Translation von Serin und Arginin im Linker (G **S S** G **R R S** L Q G G G G) zwischen der 3. und 4. Transmembrandomäne von CD63 (Fig. 10A). Die Vektoren mit weiteren Schnittstellen wurde durch Gensynthese⁵⁴ (ATG:Biosynthetics) erzeugt und in HEK293T-Zellen wie bereits im Abschnitt 2 Transfektion und konfokale Laser-Scanning-mikroskopie (cLSM) beschrieben, zur Expression gebracht und auf subzelluläre Lokalisation hin untersucht (Fig. 10B).

### Sequenzprotokoll

SEQ ID NO: 1: DNA-Sequenz von pA4E211 (CD63ΔLEL)
SEQ ID NO: 2: DNA-Sequenz von CD63ΔLEL
SEQ ID NO: 3: Proteinsequenz von CD63ΔLEL
SEQ ID NO: 4: Primer PA1-01
SEQ ID NO: 5: Primer PA-02
SEQ ID NO: 6: Primer PA1-03
SEQ ID NO: 7: Primer PA1-04
SEQ ID NO: 8: Primer PA1-05
SEQ ID NO: 9: Primer PA1-06
SEQ ID NO: 10: Primer PA1-07
SEQ ID NO: 11: Primer PA1-08
SEQ ID NO: 12: Primer PA1-09
SEQ ID NO: 13: Primer PA1-10
SEQ ID NO: 14: Primer PA1-11
SEQ ID NO: 15: Primer PA1-12
SEQ ID NO: 16: Primer PA1-13
SEQ ID NO: 17: CD63, Genbank Accsession No. KF998086
SEQ IN NO: 18: Proteinsequenz von CD63
SEQ ID NO: 19: DNA-Sequenz von CD9 (Genbank Accession No. NM_001769.3)
SEQ ID NO: 20: Proteinsequenz von CD9
SEQ ID NO: 21: DNA-Sequenz von CD81 (Genbank Accession No. NM_004356.3)
SEQ ID NO: 22: Proteinsequenz von CD81
SEQ ID NO: 23: DNA-Sequenz von CD82 (Genbank Accession No. NM_002231.3)
SEQ ID NO: 24: Proteinsequenz von CD82
SEQ ID NO: 25: DNA-Sequenz von CD151 (Genbank Accession No. BT007397.1)
SEQ ID NO: 26: Proteinsequenz von CD151
SEQ ID NO: 27: DNA-Sequenz von CD9ΔLEL-V5-6xHis
SEQ ID NO: 28: Proteinsequenz von CD9ΔLEL-V5-6xHis
SEQ ID NO: 29: DNA-Sequenz von CD81ΔLEL-V5-6xHis
SEQ ID NO: 30: Proteinsequenz von CD81ΔLEL-V5-6xHis
SEQ ID NO: 31: DNA-Sequenz von CD82ΔLEL-V5-6xHis
SEQ ID NO: 32: Proteinsequenz von CD82ΔLEL-V5-6xHis
SEQ ID NO: 33: DNA-Sequenz von CD151ΔLEL-V5-6xHis
SEQ ID NO: 34: Proteinsequenz von CD151ΔLEL-V5-6xHis
SEQ ID NO: 35: DNA-Sequenz des V5-6xHis-Linkers
SEQ ID NO: 36: Proteinsequenz des V5-6xHis-Linkers
SEQ ID NO: 37: DNA-Sequenz des 2F5-4E10-Linkers
SEQ ID NO: 38: Proteinsequenz des 2F5-4E10-Linkers
SEQ ID NO: 39: DNA-Sequenz der Linkerversion (8)
SEQ ID NO: 40: Proteinsequenz der Linkerversion (8)
SEQ ID NO: 41: DNA-Sequenz des Linkers im Vektor pCMV-CD63ΔLEL-mCherry (Minimallinker (9))
SEQ ID NO: 42: Proteinsequenz des Linkers im Vektor pCMV-CD63ΔLEL-mCherry (Minimallinker (9))
SEQ ID NO: 43: DNA-Sequenz des Linkers im Vektor pCMV-CD63ΔLEL-V2-mCherry
SEQ ID NO: 44: Proteinsequenz des Linkers im Vektor pCMV-CD63ΔLEL-V2-mCherry
SEQ ID NO: 45: DNA-Sequenz des Linkers zwischen CD63ΔLEL und dem Fluoreszenzprotein
SEQ ID NO: 46: Protein-Sequenz des Linkers zwischen CD63ΔLEL und dem Fluoreszenzprotein

### Referenzen

1 Berggard, T., Linse, S. & James, P. Methods for the detection and analysis of protein-protein interactions. Proteomics 7, 2833-2842, doi:10.1002/pmic.200700131 (2007).
2 Glick, B. S. & Malhotra, V. The curious status of the Golgi apparatus. Cell 95, 883-889 (1998).
3 Call, M. E. & Wucherpfennig, K. W. The T cell receptor: critical role of the membrane environment in receptor assembly and function. Annu Rev Immunol 23, 101-125, doi:10.1146/annurev.immunol.23.021704.115625 (2005).
4 Sachs, J. N. & Engelman, D. M. Introduction to the membrane protein reviews: the interplay of structure, dynamics, and environment in membrane protein function. Annu Rev Biochem 75, 707-712, doi:10.1146/annurev.biochem.75.110105.142336 (2006).
5 Macher, B. A. & Yen, T.-Y. Proteins at membrane surfaces-a review of approaches. Molecular BioSystems 3, 705-713, doi:10.1039/B708581H (2007).
6 Lodish, H. F. Molecular cell biology. (W.H. Freeman, 2013).
7 Nicolson, G. L. The Fluid-Mosaic Model of Membrane Structure: still relevant to understanding the structure, function and dynamics of biological membranes after more than 40 years. Biochim Biophys Acta 1838, 1451-1466, doi:10.1016/j.bbamem.2013.10.019 (2014).
8 Whitelegge, J. P. Integral membrane proteins and bilayer proteomics. Anal Chem 85, 2558-2568, doi:10.1021/ac303064a (2013).
9 Smith, G. P. Filamentous fusion phage: novel expression vectors that display cloned antigens on the virion surface. Science 228, 1315-1317 (1985).
10 Crameri, R., Jaussi, R., Menz, G. & Blaser, K. Display of expression products of cDNA libraries on phage surfaces. A versatile screening system for selective isolation of genes by specific gene-product/ligand interaction. Eur J Biochem 226, 53-58 (1994).
11 Gu, H. et al. A phage display system for studying the sequence determinants of protein folding. Protein Sci 4, 1108-1117, doi:10.1002/pro.5560040609 (1995).
12 Kehoe, J. W. & Kay, B. K. Filamentous phage display in the new millennium. Chem Rev 105, 4056-4072, doi:10.1021/cr000261r (2005).
13 Chen, W. & Georgiou, G. Cell-Surface display of heterologous proteins: From high-throughput screening to environmental applications. Biotechnol Bioeng 79, 496-503, doi:10.1002/bit. 10407 (2002).
14 Hoischen, C. et al. Novel bacterial membrane surface display system using cell wall-less L-forms of Proteus mirabilis and Escherichia coli. Appl Environ Microbiol 68, 525-531 (2002).
15 Lee, S. Y., Choi, J. H. & Xu, Z. Microbial cell-surface display. Trends Biotechnol 21, 45-52 (2003).
16 Rutherford, N. & Mourez, M. Surface display of proteins by gram-negative bacterial autotransporters. Microb Cell Fact 5, 22, doi:10.1186/1475-2859-5-22 (2006).
17 Yang, Z., Liu, Q., Wang, Q. & Zhang, Y. Novel bacterial surface display systems based on outer membrane anchoring elements from the marine bacterium Vibrio anguillarum. Appl Environ Microbiol 74, 4359-4365, doi:10.1128/AEM.02499-07 (2008).
18 van Bloois, E., Winter, R. T., Kolmar, H. & Fraaije, M. W. Decorating microbes: surface display of proteins on Escherichia coli. Trends Biotechnol 29, 79-86, doi:10.1016/j.tibtech.2010.11.003 (2011).
19 Nicolay, T., Vanderleyden, J. & Spaepen, S. Autotransporter-based cell surface display in Gram-negative bacteria. Crit Rev Microbiol, doi:10.3109/1040841X.2013.804032 (2013).
20 Han, M.-J. & Lee, S. H. An efficient bacterial surface display system based on a novel outer membrane anchoring element from the Escherichia coli protein Yia T. (2014).
21 Boublik, Y., Di Bonito, P. & Jones, I. M. Eukaryotic virus display: engineering the major surface glycoprotein of the Autographa californica nuclear polyhedrosis virus (AcNPV) for the presentation of foreign proteins on the virus surface. Biotechnology (N Y) 13, 1079-1084 (1995).
22 Grabherr, R., Ernst, W., Doblhoff-Dier, O., Sara, M. & Katinger, H. Expression of foreign proteins on the surface of Autographa californica nuclear polyhedrosis virus. BioTechniques 22, 730-735 (1997).
23 Kim, S. Y., Sohn, J. H., Pyun, Y. R. & Choi, E. S. A cell surface display system using novel GPI-anchored proteins in Hansenula polymorpha. Yeast 19, 1153-1163, doi:10.1002/yea.911 (2002).
24 Rahman, M. M. & Gopinathan, K. P. Bombyx mori nucleopolyhedrovirusbased surface display system for recombinant proteins. J Gen Virol 84, 2023-2031 (2003).
25 Baneyx, F. & Mujacic, M. Recombinant protein folding and misfolding in Escherichia coli. Nat Biotechnol 22, 1399-1408, doi:10.1038/nbt1029 (2004).
26 Raty, J. K. et al. Enhanced gene delivery by avidin-displaying baculovirus. Mol Ther 9, 282-291, doi:10.1016/j.ymthe.2003.11.004 (2004).
27 Mao, H., Song, J., Liang, C., Yu, Z. & Chen, X. Construction of eukaryotic surface display based on the baculoviral F protein. BioTechniques 41, 266, 268, 270 passim (2006).
28 Weerapana, E. & Imperiali, B. Asparagine-linked protein glycosylation: from eukaryotic to prokaryotic systems. Glycobiology 16, 91R-101R, doi:10.1093/glycob/cwj099 (2006).
29 Wang, Q., Li, L., Chen, M., Qi, Q. & Wang, P. G. Construction of a novel system for cell surface display of heterologous proteins on Pichia pastoris. Biotechnol Lett 29, 1561-1566, doi:10.1007/s10529-007-9430-6 (2007).
30 Chesnut, J. D. et al. Selective isolation of transiently transfected cells from a mammalian cell population with vectors expressing a membrane anchored single-chain antibody. J Immunol Methods 193, 17-27 (1996).
31 Ellmark, P., Ohlin, M., Borrebaeck, C. A. & Furebring, C. A novel mammalian display system for the selection of protein-protein interactions by decoy receptor engagement. J Mol Recognit 17, 316-322, doi:10.1002/jmr.678 (2004).
32 Zhou, C., Jacobsen, F. W., Cai, L., Chen, Q. & Shen, W. D. Development of a novel mammalian cell surface antibody display platform. MAbs 2, 508-518, doi:10.4161/mabs.2.5.12970 (2010).
33 Tomimatsu, K. et al. A rapid screening and production method using a novel mammalian cell display to isolate human monoclonal antibodies. Biochem Biophys Res Commun 441, 59-64, doi:10.1016/j.bbrc.2013.10.007 (2013).
34 Stipp, C. S., Kolesnikova, T. V. & Hemler, M. E. Functional domains in tetraspanin proteins. Trends Biochem Sci 28, 106-112 (2003).
35 Kovalenko, O. V., Metcalf, D. G., DeGrado, W. F. & Hemler, M. E. Structural organization and interactions of transmembrane domains in tetraspanin proteins. BMC Struct Biol 5, 11, doi:10.1186/1472-6807-5-11 (2005).
36 Hemler, M. E. Targeting of tetraspanin proteins--potential benefits and strategies. Nat Rev Drug Discov 7, 747-758, doi:10.1038/nrd2659 (2008).
37 Levy, S. & Shoham, T. The tetraspanin web modulates immunesignalling complexes. Nat Rev Immunol 5, 136-148, doi:10.1038/nri1548 (2005).
38 Rubinstein, E. et al. CD9, CD63, CD81, and CD82 are components of a surface tetraspan network connected to HLA-DR and VLA integrins. Eur J Immunol 26, 2657-2665, doi:10.1002/eji.1830261117 (1996).
39 Green, M. R. & Sambrook, J. Molecular cloning : a laboratory manual. 1. (Cold Spring Harbor Laboratory Press, 2012).
40 Rognan, D. Rational design of protein-protein interaction inhibitors. MedChemComm 6, 51-60, doi:10.1039/C4MD00328D (2015).
41 Jin, L., Wang, W. & Fang, G. Targeting protein-protein interaction by small molecules. Annu Rev Pharmacol Toxicol 54, 435-456, doi:10.1146/annurev-pharmtox-011613-140028 (2014).
42 Milroy, L. G., Grossmann, T. N., Hennig, S., Brunsveld, L. & Ottmann, C. Modulators of protein-protein interactions. Chem Rev 114, 4695-4748, doi:10.1021/cr400698c (2014).
43 Rao, V. S., Srinivas, K., Sujini, G. N. & Kumar, G. N. Protein-protein interaction detection: methods and analysis. Int J Proteomics 2014, 147648, doi:10.1155/2014/147648 (2014).
44 Arkin, M. R. & Wells, J. A. Small-molecule inhibitors of protein-protein interactions: progressing towards the dream. Nat Rev Drug Discov 3, 301-317, doi:10.1038/nrd1343 (2004).
45 Jones, S. & Thornton, J. M. Principles of protein-protein interactions. Proc Natl Acad Sci U S A 93, 13-20 (1996).
46 Jones, S. & Thornton, J. M. Protein-protein interactions: a review of protein dimer structures. Prog Biophys Mol Biol 63, 31-65 (1995).
47 Sudhof, T. C. The synaptic vesicle cycle: a cascade of protein-protein interactions. Nature 375, 645-653, doi:10.1038/375645a0 (1995).
48 Mullard, A. Protein-protein interaction inhibitors get into the groove. Nat Rev Drug Discov 11, 173-175, doi:10.1038/nrd3680 (2012).
49 Ivanusic, D., Eschricht, M. & Denner, J. Investigation of membrane protein-protein interactions using correlative FRET-PLA. BioTechniques 57, 188-198, doi:10.2144/000114215 (2014).
50 Kremers, G. J., Goedhart, J., van Munster, E. B. & Gadella, T. W., Jr. Cyan and yellow super fluorescent proteins with improved brightness, protein folding, and FRET Forster radius. Biochemistry 45, 6570-6580, doi:10.1021/bi0516273 (2006).
51 Adachi, A. et al. Production of acquired immunodeficiency syndromeassociated retrovirus in human and nonhuman cells transfected with an infectious molecular clone. J Virol 59, 284-291 (1986).
52 Stephanie Charrin, Frangois le Naour, Olivier Silvie, Pierre-Emmanuel Milhiet, Claude Boucheix, Eric Rubinstein, Lateral organization of membrane proteins: tetraspanins spin their web, Bioch. J. (2009), 420 (2) 133-154; doi: 10.1042/BJ20082422
53 A Voller, A Bartlett, D E Bidwell, Enzyme immunoassays with special reference to ELISA techniques, J Clin Pathol., 1978 June, 31(6): 507-520
54 Hughes RA, Miklos AE, Ellington AD, Gene synthesis: methods and applications, Methods Enzymol. 2011; 498: 277-309, doi: 10.2016/B978-0-12-385120-8.00012-7

## Patentansprüche

1. Fusionsprotein, umfassend eine erste Domäne (i), eine zweite Domäne (ii) und eine dritte Domäne (iii), wobei die zweite Domäne zwischen der ersten und der dritten Domäne angeordnet ist, wobei
(i) eine Teilsequenz eines Tetraspanins, welche die Transmembrandomäne 1 (TM1), die kleine extrazelluläre Schleife (SEL), die Transmembrandomäne 2 (TM2), die kleine intrazelluläre Schleife (SIL) und die Transmembrandomäne 3 (TM3) einschließt, oder eine dazu homologe Sequenz mit einer Sequenzidentität von mindestens 95% über die gesamte Länge umfasst,
(ii) ein Peptid mit einer vorbestimmten Aminosäuresequenz umfasst, die zu der großen extrazellulären Schleife (LEL) eines Tetraspanins eine Sequenzidentität von weniger als 70% über die gesamte Länge aufweist und
(iii) eine Teilsequenz eines Tetraspanins, welche die Transmembrandomäne 4 (TM4) einschließt, oder eine dazu homologe Sequenz mit einer Sequenzidentität von mindestens 95% über die gesamte Länge umfasst,
und wobei zwischen der ersten und der zweiten Domäne und/oder zwischen der zweiten und der dritten Domäne flexible Serin- und Arginin-freie Linker angeordnet sind,
wobei das Fusionsprotein zur Verankerung in der Membran einer Zelle in der Lage ist.

2. Fusionsprotein nach Anspruch 1, wobei die zweite Domäne (ii) weiterhin ein oder mehrere Teilsequenzen der LEL eines Tetraspanins oder dazu homologe Sequenzen mit einer Sequenzidentität von mindestens 70% über die gesamte Länge umfasst, welche C- und/oder N-terminal zu dem Peptid mit der vorbestimmten Aminosäuresequenz angeordnet sind, und/oder wobei die zweite Domäne (ii) weiterhin eine oder mehrere Proteaseerkennungssequenzen umfasst, welche C- und/oder N-terminal zu dem Peptid mit der vorbestimmten Aminosäuresequenz angeordnet sind.

3. Fusionsprotein nach einem der vorherigen Ansprüche, wobei das Peptid ausgewählt ist aus einem Epitop, einem Protein, einem Antigen, oder einem Enzym.

4. Fusionsprotein nach einem der vorherigen Ansprüche, weiterhin umfassend eine vierte Domäne (iv) am N-Terminus des Fusionsproteins und/oder eine fünfte Domäne (v) am C-Terminus des Fusionsproteins, wobei die vierte und/oder fünfte Domäne vorzugsweise einen Tag, beispielsweise einen Affinitätstag oder fluoreszierenden Tag umfasst.

5. Nukleinsäuremolekül, das für ein Fusionsprotein nach einem der Ansprüche 1-4 kodiert.

6. Vektor, umfassend ein Nukleinsäuremolekül nach Anspruch 5, vorzugsweise in operativer Verknüpfung mit einer Expressionskontrollsequenz.

7. Zelle, umfassend ein Nukleinsäuremolekül nach Anspruch 5 oder einen Vektor nach Anspruch 6, wobei die Zelle vorzugsweise eine eukaryotische Zelle, bevorzugt eine Säugerzelle wie z.B. eine humane Zelle ist, und/oder wobei die Zelle vorzugsweise weiterhin mit mindestens einem weiteren Nukleinsäuremolekül oder mindestens einem weiteren Expressionsvektor transformiert oder transfiziert wurde.

8. Membranzubereitung, die aus einer Zelle nach Anspruch 7 erhalten wurde, umfassend eine Membran und ein Fusionsprotein nach einem der Ansprüche 1-4.

9. Synthetisches Membransystem, umfassend eine Membran und ein Fusionsprotein nach einem der Ansprüche 1-4.

10. Verfahren zur Präsentation eines Peptids mit einer vorbestimmten Aminosäuresequenz auf der Oberfläche einer Zelle, umfassend die Schritte:
(a) Bereitstellen einer Zelle nach Anspruch 7,
(b) Kultivieren der Zelle unter Bedingungen, bei denen das Fusionsprotein nach einem der Ansprüche 1 bis 4 exprimiert wird.

11. Verfahren zur Verankerung eines Peptids mit einer vorbestimmten Aminosäuresequenz an einer Membran, umfassend die Schritte:
(a) Bereitstellen einer Membran, und
(b) Inkontaktbringen der Membran mit einem Fusionsprotein nach einem der Ansprüche 1 bis 4 unter Bedingungen, bei denen eine Verankerung des Fusionsproteins in der Membran erfolgt, oder Inkontaktbringen der Membran mit einer Nukleinsäure nach Anspruch 5 oder einem Vektor nach Anspruch 6, unter Bedingungen bei denen das Fusionsprotein nach einem der Ansprüche 1 bis 4 exprimiert und in der Membran verankert wird.

12. Verwendung einer Zelle nach Anspruch 7, einer Membranzubereitung nach Anspruch 8 oder eines synthetischen Membransystems nach Anspruch 9
- in einem Verfahren zum Screening nach Interaktionspartnern des Peptids,
- zur Untersuchung von Wechselwirkungen des Peptids mit Interaktionspartnern
- in einem Verfahren zur Herstellung des Peptids oder
- als Nachweisreagenz.

13. Zelle nach Anspruch 7, Membranzubereitung nach Anspruch 8 oder synthetisches Membransystems nach Anspruch 9, zur Verwendung als Vakzin oder als Medikament.

14. Nukleinsäuremolekül, umfassend einen ersten Sequenzabschnitt (i), einen zweiten Sequenzabschnitt (ii) und einen dritten Sequenzabschnitt (iii), wobei der zweite Sequenzabschnitt zwischen dem ersten und dem dritten Sequenzabschnitt angeordnet ist, wobei
(i) für eine Teilsequenz eines Tetraspanins, welche die Transmembrandomäne 1 (TM1), die kleine extrazelluläre Schleife (SEL), die Transmembrandomäne 2 (TM2), die kleine intrazelluläre Schleife (SIL) und die Transmembrandomäne 3 (TM3) einschließt, oder eine dazu homologe Sequenz mit einer Sequenzidentität von mindestens 95% über die gesamte Länge, kodiert,
(ii) eine Multiple Cloning Site umfasst und
(iii) für eine Teilsequenz eines Tetraspanins, welche die Transmembrandomäne 4 (TM4) umfasst, oder eine dazu homologe Sequenz mit einer Sequenzidentität von mindestens 95 % über die gesamte Länge, kodiert,
wobei die von dem ersten Sequenzabschnitt (i) und dem dritten Sequenzabschnitt (iii) kodierten Teilsequenzen zur Verankerung in der Membran einer Zelle in der Lage sind, und
wobei Nukleinsäuresequenzen, die für einen flexiblen Serin- und Arginin-freien Linker kodieren, zwischen dem ersten und dem zweiten Sequenzabschnitt und/oder zwischen dem zweiten und dem dritten Sequenzabschnitt angeordnet sind.

15. Vektor, umfassend ein Nukleinsäuremolekül nach Anspruch 14, vorzugsweise in operativer Verknüpfung mit einer Expressionskontrollsequenz.

16. Kit, umfassend
(i) ein Nukleinsäuremolekül nach Anspruch 5 oder 14 oder einen Vektor nach Anspruch 6 oder 15 und
(ii) eine Zelle oder Membran.

## Claims

1. Fusion protein, comprising a first domain (i), a second domain (ii) and a third domain (iii), wherein the second domain is disposed between the first and the third domain, wherein
(i) comprises a partial sequence of a tetraspanin, which includes the transmembrane domain 1 (TM1), the small extracellular loop (SEL), the transmembrane domain 2 (TM2), the small intracellular loop (SIL) and the transmembrane domain 3 (TM3), or a sequence homologous thereto with a sequence identity of at least 95 % over the entire length,
(ii) comprises a peptide with a predetermined amino-acid sequence, having a sequence identity of less than 70 % over the entire length in respect of the large extracellular loop (LEL) of a tetraspanin and
(iii) comprises a partial sequence of a tetraspanin, which includes the transmembrane domain 4 (TM4) or a sequence homologous thereto with a sequence identity of at least 95 % over the entire length,
and wherein flexible serine- and arginine-free linkers are disposed between the first and the second domain and/or between the second and the third domain,
wherein the fusion protein is able to anchor in the membrane of a cell.

2. Fusion protein according to claim 1, wherein the second domain (ii) comprises furthermore one or more partial sequences of the LEL of a tetraspanin or sequences homologous thereto with a sequence identity of at least 70 % over the entire length, which are disposed C- and/or N-terminally to the peptide with the predetermined amino-acid sequence, and/or wherein the second domain (ii) comprises furthermore one or more protease recognition sequences, which are disposed C- and/or N-terminally to the peptide with the predetermined amino-acid sequence.

3. Fusion protein according to any one of the preceding claims, wherein the peptide is selected from an epitope, a protein, an antigen, or an enzyme.

4. Fusion protein according to any one of the preceding claims, comprising furthermore a fourth domain (iv) on the N-terminus of the fusion protein and/or a fifth domain (v) on the C-terminus of the fusion protein, wherein the fourth and/or fifth domain preferably comprises a tag, for example an affinity tag or fluorescent tag.

5. Nucleic acid molecule that codes for a fusion protein according to any one of claims 1-4.

6. Vector, comprising a nucleic acid molecule according to claim 5, preferably operably linked to an expression control sequence.

7. Cell, comprising a nucleic acid molecule according to claim 5 or a vector according to claim 6, wherein the cell is preferably a eukaryotic cell, preferably a mammalian cell such as e.g. a human cell, and/or wherein the cell was furthermore preferably transformed or transfected with at least one further nucleic acid molecule or at least one further expression vector.

8. Membrane preparation that was obtained from a cell according to claim 7, comprising a membrane and a fusion protein according to any one of claims 1-4.

9. Synthetic membrane system, comprising a membrane and a fusion protein according to any one of claims 1-4.

10. Method for the presentation of a peptide with a predetermined amino-acid sequence on the surface of a cell, comprising the steps:
(a) provision of a cell according to claim 7,
(b) cultivation of the cell under conditions where the fusion protein according to any one of claims 1 to 4 is expressed.

11. Method for the anchoring of a peptide with a predetermined amino-acid sequence on a membrane, comprising the steps:
(a) provision of a membrane, and
(b) bringing into contact of the membrane with a fusion protein according to any one of claims 1 to 4 under conditions where anchoring of the fusion protein takes place in the membrane, or
bringing into contact of the membrane with a nucleic acid according to claim 5 or a vector according to claim 6, under conditions where the fusion protein according to any one of claims 1 to 4 is expressed and anchored in the membrane.

12. Use of a cell according to claim 7, a membrane preparation according to claim 8 or a synthetic membrane system according to claim 9
- in a method for screening for interaction partners of the peptide,
- for the study of interactions of the peptide with interaction partners
- in a method for the production of the peptide or
- as detection reagent.

13. Cell according to claim 7, membrane preparation according to claim 8 or synthetic membrane system according to claim 9, for use as vaccine or as medication.

14. Nucleic acid molecule, comprising a first sequence segment (i), a second sequence segment (ii) and a third sequence segment (iii), wherein the second sequence segment is disposed between the first and the third sequence segment, wherein
(i) codes for a partial sequence of a tetraspanin, which includes the transmembrane domain 1 (TM1), the small extracellular loop (SEL), the transmembrane domain 2 (TM2), the small intracellular loop (SIL) and the transmembrane domain 3 (TM3), or a sequence homologous thereto with a sequence identity of at least 95 % over the entire length,
(ii) comprises a multiple cloning site and
(iii) codes for a partial sequence of a tetraspanin, comprising the transmembrane domain 4 (TM4) or a sequence homologous thereto with a sequence identity of at least 95 % over the entire length,
wherein the partial sequences coded from the first sequence segment (i) and the third sequence segment (iii) are able to anchor in the membrane of a cell, and wherein nucleic acid sequences that code for a flexible serine- and arginine-free linker are disposed between the first and the second sequence segment and/or between the second and the third sequence segment.

15. Vector, comprising a nucleic acid molecule according to claim 14, preferably operably linked to an expression control sequence.

16. Kit, comprising
(i) a nucleic acid molecule according to claim 5 or 14 or a vector according to claim 6 or 15 and
(ii) a cell or membrane.

## Revendications

1. Protéine de fusion comprenant un premier domaine (i), un deuxième domaine (ii) et un troisième domaine (iii), ledit deuxième domaine étant situé entre lesdits premier et troisième domaines, dans laquelle
(i) comprend une séquence partielle d'une tétraspanine comprenant le domaine transmembranaire 1 (TM1), la petite boucle extracellulaire (SEL), le domaine transmembranaire 2 (TM2), la petite boucle intracellulaire (SIL) et le domaine transmembranaire 3 (TM3), ou une séquence homologue à celle-ci avec une identité de séquence d'au moins 95% sur toute la longueur,
(ii) comprend un peptide avec une séquence d'acides aminés prédéterminée, ayant une identité de séquence avec la grande boucle extracellulaire (LEL) d'une tétraspanine inférieure à 70% sur toute la longueur, et
(iii) comprend une séquence partielle d'une tétraspanine comprenant le domaine transmembranaire 4 (TM4) ou une séquence homologue à celui-ci avec une identité de séquence d'au moins 95% sur toute la longueur,
et dans laquelle des segments de liaison flexibles exempts de sérine et d'arginine sont disposés entre les premier et deuxième domaines et/ou entre les deuxième et troisième domaines,
dans laquelle la protéine de fusion est capable de s'ancrer dans la membrane d'une cellule.

2. Protéine de fusion selon la revendication 1, dans laquelle le deuxième domaine (ii) comprend en outre une ou plusieurs séquences partielles de la LEL d'une tétraspanine ou des séquences homologues à celle-ci avec une identité de séquence d'au moins 70 % sur toute la longueur, qui sont disposées en position C- et/ou N-terminale par rapport au peptide avec la séquence d'acides aminés prédéterminée, et/ou dans laquelle le deuxième domaine (ii) comprend en outre une ou plusieurs séquences de reconnaissance de protéase, qui sont disposées en position C- et/ou N-terminale par rapport au peptide avec la séquence d'acides aminés prédéterminée.

3. Protéine de fusion selon l'une des revendications précédentes, dans laquelle le peptide est choisi parmi un épitope, une protéine, un antigène ou une enzyme.

4. Protéine de fusion selon l'une des revendications précédentes, comprenant en outre un quatrième domaine (iv) à l'extrémité N-terminale de la protéine de fusion et/ou un cinquième domaine (v) à l'extrémité C-terminale de la protéine de fusion, dans laquelle le quatrième et/ou le cinquième domaine comprend de préférence un marqueur, par exemple un marqueur d'affinité ou un marqueur fluorescent.

5. Molécule d'acide nucléique codant pour une protéine de fusion selon l'une des revendications 1 à 4.

6. Vecteur comprenant une molécule d'acide nucléique selon la revendication 5, de préférence en liaison opérationnelle avec une séquence de contrôle d'expression.

7. Cellule comprenant une molécule d'acide nucléique selon la revendication 5 ou un vecteur selon la revendication 6, dans laquelle la cellule est de préférence une cellule eucaryote, de préférence une cellule de mammifère telle qu'une cellule humaine, et/ou dans laquelle la cellule a de préférence en outre été transformée ou transfectée avec au moins une autre molécule d'acide nucléique ou au moins un autre vecteur d'expression.

8. Préparation membranaire obtenue à partir d'une cellule selon la revendication 7, comprenant une membrane et une protéine de fusion selon l'une des revendications 1 à 4.

9. Système de membrane synthétique comprenant une membrane et une protéine de fusion selon l'une quelconque des revendications 1 à 4.

10. Méthode pour présenter un peptide ayant une séquence d'acides aminés prédéterminée sur la surface d'une cellule, comprenant les étapes suivantes :
a) fournir une cellule selon la revendication 7,
b) cultiver la cellule dans des conditions dans lesquelles la protéine de fusion selon l'une des revendications 1-4 est exprimée.

11. Méthode pour ancrer un peptide ayant une séquence d'acides aminés prédéterminée à une membrane, comprenant les étapes suivantes :
a) fournir une membrane, et
b) mettre en contact la membrane avec une protéine de fusion selon l'une quelconque des revendications 1 à 4 dans des conditions dans lesquelles un ancrage de la protéine de fusion se produit dans la membrane, ou
mettre en contact la membrane avec un acide nucléique selon la revendication 5 ou un vecteur selon la revendication 6 dans des conditions dans lesquelles la protéine de fusion selon l'une quelconque des revendications 1 à 4 exprime et est ancrée dans la membrane.

12. Utilisation d'une cellule selon la revendication 7, d'une préparation de membrane selon la revendication 8 ou d'un système de membrane synthétique selon la revendication 9
- dans un procédé de dépistage des partenaires d'interaction du peptide,
- pour étudier les interactions du peptide avec les partenaires d'interaction,
- dans un procédé de production du peptide, ou
- comme réactif de détection.

13. Cellule selon la revendication 7, préparation de membrane selon la revendication 8 ou système de membrane synthétique selon la revendication 9, pour l'utilisation comme vaccin ou comme médicament.

14. Molécule d'acide nucléique comprenant une première partie de séquence (i), une deuxième partie de séquence (ii) et une troisième partie de séquence (iii), la deuxième partie de séquence étant située entre les première et troisième parties de séquence, dans laquelle
(i) code pour une séquence partielle d'une tétraspanine, comprenant le domaine transmembranaire 1 (TM1), la petite boucle extracellulaire (SEL), le domaine transmembranaire 2 (TM2), la petite boucle intracellulaire (SIL) et le domaine transmembranaire 3 (TM3), ou une séquence homologue à celui-ci ayant une identité de séquence d'au moins 95% sur toute la longueur,
(ii) comprend un site de clonage multiple, et
(iii) code pour une séquence partielle d'une tétraspanine comprenant le domaine transmembranaire 4 (TM4) ou une séquence homologue à celui-ci avec une identité de séquence d'au moins 95% sur toute la longueur,
dans laquelle les séquences partielles codées par la première section de séquence (i) et la troisième section de séquence (iii) sont capables de s'ancrer dans la membrane d'une cellule, et
dans laquelle des séquences d'acide nucléique codant pour un segment de liaison flexible exempt de sérine et d'arginine sont disposées entre la première et la deuxième section de séquence et/ou entre la deuxième et la troisième section de séquence.

15. Vecteur comprenant une molécule d'acide nucléique selon la revendication 14, de préférence en liaison opérationnelle avec une séquence de contrôle d'expression.

16. Kit comprenant
(i) une molécule d'acide nucléique selon la revendication 5 ou 14 ou un vecteur selon la revendication 6 ou 15, et
(ii) une cellule ou une membrane.


### SEQUENCE LISTING

<110> Peter und Traudl Engelhorn-Stiftung zur Förderung der Lebenswissenschaften
<120> System zur Präsentation von Peptiden auf der Zelloberfläche
<130> 61317P WO
<150> DE102015002851
   <151> 2015-03-05
<160> 46
<170> PatentIn version 3.5
<210> 1
   <211> 4832
   <212> DNA
   <213> artificial sequence
<220>
   <223> pA4E211 (CD63delLEL); künstliche Sequenz als Shuttle Vektor von E. coli nach Eukaryotischen Zellen (hier: Säugetierzellen)
<400> 1
   cctcgaggaa ggctactacg cctaagctac cgacaggttg gctgataagt ccccggtctc      60
   aaggcgcgcc tagttattaa tagtaatcaa ttacggggtc attagttcat agcccatata     120
   tggagttccg cgttacataa cttacggtaa atggcccgcc tggctgaccg cccaacgacc     180
   cccgcccatt gacgtcaata atgacgtatg ttcccatagt aacgccaata gggactttcc     240
   attgacgtca atgggtggag tatttacggt aaactgccca cttggcagta catcaagtgt     300
   atcatctgcc aagtacgccc cctattgacg tcaatgacgg taaatggccc gcctggcatt     360
   atgcccagta catgacctta tgggactttc ctacttggca gtacatctac gtattagtca     420
   tcgctattac cacggtgatg cggttttggc agtacatcaa tgggcgtgga tagcggtttg     480
   actcacgggg atttccaagt ctccacccca ttgacgtcaa tgggagtttg ttttggcacc     540
   aaaatcaacg ggactttcca aaatgtcgta acaactccgc cccattgacg caaatgggcg     600
   gtaggcgtgt acggtgggag gtctatataa gcagagctct ctggctaact agagaaccca     660
   ctgcttactg gcttatcgaa attaatacga ctcactatag gaagccgcca cctgctagca     720
   atttatagcc accatggatt acaaggatga cgacgataag cccatggcgg tggaaggagg     780
   aatgaaatgt gtgaagttct tgctctacgt cctcctgctg gccttttgcg cctgtgcagt     840
   gggactgatt gccgtgggtg tcggggcaca gcttgtcctg agtcagacca taatccaggg     900
   ggctacccct ggctctctgt tgccagtggt catcatcgca gtgggtgtct tcctcttcct     960
   ggtggctttt gtgggctgct gcggggcctg caaggagaac tattgtctta tgattacgtt    1020
   tgccatcttt ctgtctctta tcatgttggt ggaggtggcc gcagccattg ctggctatgt    1080
   gtttagagat aagctgcaag aattcgagaa tctgtacttt cagtcgaagc ttggatccct    1140
   gcagcatcac catcaccatc acgaaaacct ctatttccag agcgaacaga aactgatatc 1200
   cgaagaagat ctggatatcg gaggcggagg caaaaatgtg ctggtggtag ctgccgcagc 1260
   ccttggaatt gcttttgtcg aggttttggg aattgtcttt gcctgctgcc tcgtgaagag 1320
   tatcagaagt ggctacgagg tgatgaaact tatcgatacc gtcgatctcc atatgccagc 1380
   catgaagata gaatgcagga ttacaggcac cctgaatggc gtggagttcg agctggtcgg 1440
   gggtggagaa ggaacacctg agcagggccg aatgacgaac aagatgaaga gcaccaaagg 1500
   agccctgaca tttagcccct atctcctctc tcatgttatg ggctacggct tttatcactt 1560
   cgggacttac ccaagcggat atgaaaaccc tttccttcac gccataaaca atgggggtta 1620
   caccaacacc cggattgaga aatatgagga cggtggcgtg ttgcatgtct ccttcagcta 1680
   cagatatgag gctggtcgcg tgatcggaga ttttaaggtt gtgggcacag gctttccaga 1740
   agactccgta atcttcaccg acaagatcat tcggtctaat gccacggtgg agcatcttca 1800
   ccctatgggc gataatgtcc tcgtcgggag ctttgccaga actttttcac tgagggatgg 1860
   gggatactat tcattcgtcg tagactccca catgcacttt aaatccgcaa tccacccatc 1920
   aatcctgcaa aacggagggc ctatgtttgc gttcagacgt gtagaggagc tgcattctaa 1980
   cactgaactc ggaatcgttg aataccagca cgctttcaaa actcccattg cattcgctgg 2040
   gccctgatca tgagagttta aacataaagc ctcgactgtg ccttctagtt gccagccatc 2100
   tgttgtttgc ccctcccccg tgccttcctt gaccctggaa ggtgccactc ccactgtcct 2160
   ttcctaataa aatgaggaaa ttgcatcgca ttgtctgagt aggtgtcatt ctattctggg 2220
   gggtggggtg gggcaggaca gcaaggggga ggattgggaa gacaatagca ggcatgctgg 2280
   ggatgcggtg ggctctatgg aaaaaacgcg cgcggatata atataacttc gtataatgta 2340
   tgctatacga agttatatat aatgcgtcga cgcactcaac cctatctcgg tctattcttt 2400
   tgatttataa gggattttgc cgatttcggc ctattggtta aaaaatgagc tgatttaaca 2460
   aaaatttaac gcgaatttta acaaaatatt aacgcttaca atttaggtgg cacttttcgg 2520
   ggaaatgtgc gcggaacccc tatttgttta tttttctaaa tacattcaaa tatgtatccg 2580
   ctcatgagac aataaccctg ataaatgctt caataatatt gaaaaaggaa gagtatgagt 2640
   attcaacatt tccgtgtcgc ccttattccc ttttttgcgg cattttgcct tcctgttttt 2700
   gctcacccag aaacgctggt gaaagtaaaa gatgctgaag atcagttggg tgcccgagtg 2760
   ggttacatcg aactggatct caacagcggt aagatccttg agagttttcg ccccgaagaa 2820
   cgttttccaa tgatgagcac ttttaaagtt ctgctatgtg gcgcggtatt atcccgtatt 2880
   gacgccgggc aagagcaact cggtcgccgc atacactatt ctcagaatga cttggttgag 2940
   tattcaccag tcacagaaaa gcatcttacg gatggcatga cagtaagaga attatgcagt 3000
   gctgccataa ccatgagtga taacactgcg gccaacttac ttctgacaac gattggagga 3060
   ccgaaggagc taaccgcttt tttgcacaac atgggggatc atgtaactcg ccttgatcgt 3120
   tgggaaccgg agctgaatga agccatacca aacgacgagc gtgacaccac gatgcctgta 3180
   gcaatggcaa caacgttgcg taaactatta actggcgaac tacttactct agcttcccgg 3240
   caacaattga tagactggat ggaggcggat aaagttgcag gaccacttct gcgctcggcc 3300
   cttccggctg gctggtttat tgctgataaa tctggagccg gtgagcgtgg ttctcgcggt 3360
   atcattgcag cactggggcc agatggtaag ccctcccgta tcgtagttat ctacacgacg 3420
   gggagtcagg caactatgga tgaacgaaat agacagatcg ctgagatagg tgcctcactg 3480
   attaagcatt ggtaactgtc agaccaagtt tactcatata tactttagat tgatttaaaa 3540
   cttcattttt aatttaaaag gatctaggtg aagatccttt ttgataatct tatgaccaaa 3600
   atcccttaac gtgagttttc gttccactga gcgtcagacc gcgatcgcag cagaccgggg 3660
   acttatcagc caacctgtcg ttaattaacc atgtcagccg ttaagtgttc ctgtgtcact 3720
   caaaattgct ttgagaggct ctaagggctt ctcagtgcgt tacatccctg gcttgttgtc 3780
   cacaaccgtt aaaccttaaa ggctttaaaa gccttatata ttcttttttt tcttataaaa 3840
   cttaaaacct tagaggctat ttaagttgct gatttatatt aattgaagag cattatacca 3900
   aaatccctta acgtgagttt tcgttccact gagcgtcaga ccccgtagaa aagatcaaag 3960
   gatcttcttg agatcctttt tttctgcgcg taatatgctg cttgcaaaca aaaaaaccac 4020
   cgctaccagc ggtggtttgt ttgccggatc aagagctacc aactcttttt ccgaaggtaa 4080
   ctggcttcag cagagcgcag ataccaaata ctgtccttct agtgtagccg tagttaggcc 4140
   accacttcaa gaactctgta gcaccgccta catacctcgc tctgctaatc ctgttaccag 4200
   tggctgctgc cagtggcgat aagtcgtgtc ttaccgggtt ggactcaaga cgatagttac 4260
   cggataaggc gcagcggtcg ggctgaacgg ggggttcgtg cacacagccc agcttggagc 4320
   gaacgaccta caccgaactg agatacctac agcgtgagct atgagaaagc gccacgcttc 4380
   ccgaagggag aaaggcggac aggtatccgg taagcggcag ggtcggaaca ggagagcgca 4440
   cgagggagct tccaggggga aacgcctggt atctttatag tcctgtcggg tttcgccacc 4500
   tctgacttga gcgtcgattt ttgtgatgct cgtcaggggg gcggagccta tggaaaaacg 4560
   ccagcaacgc ggccttttta cggttcctgg ccttttgctg gccttttgct catattagct 4620
   cttcaaattt tattgttcaa acatgagagc ttagtacgtg aaacatgaga gcttagtacg 4680
   ttagccatga gagcttagta cgttagccat gagggtttag ttcgttaaac atgagagctt 4740
   agtacgttaa acatgagagc ttagtacgtg aaacatgaga gcttagtacg tactatcaac 4800
   aggttgaact gctgatcttc agatcatata at 4832
<210> 2
   <211> 474
   <212> DNA
   <213> Artificial Sequence
<220>
   <223> Synthetische DNA Sequenz des humanen CD63 Gens (CD63delLEL); Eukaryotische Zellen, hier: Säugetierzellen); kodierende Sequenz von SEQ ID NO:1; Synthetisches CD63 - Codonen
<220>
   <221> misc_feature
   <222> (1)..(330)
   <223> TM1-TM3
<220>
   <221> misc_feature
   <222> (331) .. (360)
   <223> Minimallinker
<220>
   <221> misc_feature
   <222> (361)..(474)
   <223> TM4
<400> 2
   atggcggtgg aaggaggaat gaaatgtgtg aagttcttgc tctacgtcct cctgctggcc      60
   ttttgcgcct gtgcagtggg actgattgcc gtgggtgtcg gggcacagct tgtcctgagt     120
   cagaccataa tccagggggc tacccctggc tctctgttgc cagtggtcat catcgcagtg     180
   ggtgtcttcc tcttcctggt ggcttttgtg ggctgctgcg gggcctgcaa ggagaactat     240
   tgtcttatga tcacgtttgc catctttctg tctcttatca tgttggtgga ggtggccgca     300
   gccattgctg gctatgtgtt tagagataag ctgcaggaat tcgatatcgg aggaggagga     360
   aaaaatgtgc tggtggtagc tgcagcagcc cttggaattg cttttgtcga ggttttggga     420
   attgtctttg cctgctgcct cgtgaagagt atcagaagtg gctacgaggt gatg           474
<210> 3
   <211> 158
   <212> PRT
   <213> artificial sequence
<220>
   <223> CD63delLEL; Künstliche Sequenz im Shuttle Vektor - E. coli - Baculovirus/Insekten Zellen; translatierte Sequenz von SEQ ID NO:2
<220>
   <221> MISC_FEATURE
   <222> (1)..(110)
   <223> TM1-TM3
<220>
   <221> MISC_FEATURE
   <222> (111)..(120)
   <223> Minimallinker
<220>
   <221> MISC_FEATURE
   <222> (121)..(158)
   <223> TM4
<400> 3
   Met Ala Val Glu Gly Gly Met Lys Cys Val Lys Phe Leu Leu Tyr Val
   1               5                   10                  15
   Leu Leu Leu Ala Phe Cys Ala Cys Ala Val Gly Leu Ile Ala Val Gly
               20                  25                  30
   Val Gly Ala Gln Leu Val Leu Ser Gln Thr Ile Ile Gln Gly Ala Thr
           35                  40                  45
   Pro Gly Ser Leu Leu Pro Val Val Ile Ile Ala Val Gly Val Phe Leu
       50                  55                  60
   Phe Leu Val Ala Phe Val Gly Cys Cys Gly Ala Cys Lys Glu Asn Tyr
   65                  70                  75                  80
   Cys Leu Met Ile Thr Phe Ala Ile Phe Leu Ser Leu Ile Met Leu Val
                   85                  90                  95
   Glu Val Ala Ala Ala Ile Ala Gly Tyr Val Phe Arg Asp Lys Leu Gln
               100                 105                 110
   Glu Phe Asp Ile Gly Gly Gly Gly Lys Asn Val Leu Val Val Ala Ala
           115                 120                 125
   Ala Ala Leu Gly Ile Ala Phe Val Glu Val Leu Gly Ile Val Phe Ala
       130                 135                 140
   Cys Cys Leu Val Lys Ser Ile Arg Ser Gly Tyr Glu Val Met
   145                 150                 155
<210> 4
   <211> 30
   <212> DNA
   <213> artificial sequence
<220>
   <223> PA1-01; konstruktiv s. Patenttext
<400> 4
   tttttctcga gatggtgagc aagggcgagg 30
<210> 5
   <211> 32
   <212> DNA
   <213> artificial sequence
<220>
   <223> PA1-02; konstruktiv s. Patenttext
<400> 5
   tttttgggcc catcttgtac agctcgtcca tg 32
<210> 6
   <211> 33
   <212> DNA
   <213> artificial sequence
<220>
   <223> PA1-03; konstruktiv s. Patenttext
<400> 6
   ttttttggat ccatggcggt ggaaggagga atg 33
<210> 7
   <211> 27
   <212> DNA
   <213> artificial sequence
<220>
   <223> PA1-04; konstruktiv s. Patenttext
<400> 7
   tttttctgca gcttatctct aaacaca 27
<210> 8
   <211> 36
   <212> DNA
   <213> artificial sequence
<220>
   <223> PA1-05; konstruktiv s. Patenttext
<400> 8
   tttttgatat cggaggagga ggaaaaaatg tgctgg 36
<210> 9
   <211> 33
   <212> DNA
   <213> artificial sequence
<220>
   <223> PA1-06; konstruktiv s. Patenttext
<400> 9
   ttttttaagc ttcatcacct cgtagccact tct 33
<210> 10
   <211> 31
   <212> DNA
   <213> artificial sequence
<220>
   <223> PA1-07; konstruktiv s. Patenttext
<400> 10
   tttttgaatt catggtgagc aagggcgagg a 31
<210> 11
   <211> 31
   <212> DNA
   <213> artificial sequence
<220>
   <223> PA1-08; konstruktiv s. Patenttext
<400> 11
   tttttgatat cctttctgag tccggacttg t 31
<210> 12
   <211> 30
   <212> DNA
   <213> artificial sequence
<220>
   <223> PA1-09; konstruktiv s. Patenttext
<400> 12
   tttttgaatt cctgacggta caggccagac 30
<210> 13
   <211> 31
   <212> DNA
   <213> artificial sequence
<220>
   <223> PA1-10; konstruktiv s. Patenttext
<400> 13
   tttttgatat cgttaaacca attccacaaa c 31
<210> 14
   <211> 30
   <212> DNA
   <213> artificial sequence
<220>
   <223> PA1-11; konstruktiv s. Patenttext
<400> 14
   tttttgatat cctgcctaac tctattcact 30
<210> 15
   <211> 30
   <212> DNA
   <213> artificial sequence
<220>
   <223> PA1-12; konstruktiv s. Patenttext
<400> 15
   tttttgaatt cattgaagaa tcgcaaaacc 30
<210> 16
   <211> 31
   <212> DNA
   <213> artificial sequence
<220>
   <223> PA1-13; konstruktiv s. Patenttext
<400> 16
   tttttgatat cttttatata ccacagccaa t 31
<210> 17
   <211> 714
   <212> DNA
   <213> homo sapiens
<220>
   <221> misc_feature
   <222> (1)..(3)
   <223> Startkodon
<400> 17
   atggcggtgg aaggaggaat gaaatgtgtg aagttcttgc tctacgtcct cctgctggcc    60
   ttttgcgcct gtgcagtggg actgattgcc gtgggtgtcg gggcacagct tgtcctgagt   120
   cagaccataa tccagggggc tacccctggc tctctgttgc cagtggtcat catcgcagtg   180
   ggtgtcttcc tcttcctggt ggcttttgtg ggctgctgcg gggcctgcaa ggagaactat   240
   tgtcttatga tcacgtttgc catctttctg tctcttatca tgttggtgga ggtggccgca   300
   gccattgctg gctatgtgtt tagagataag gtgatgtcag agtttaataa caacttccgg   360
   cagcagatgg agaattaccc gaaaaacaac cacactgctt cgatcctgga caggatgcag   420
   gcagatttta agtgctgtgg ggctgctaac tacacagatt gggagaaaat cccttccatg   480
   tcgaagaacc gagtccccga ctcctgctgc attgatgtta ctgtgggctg tgggattaat   540
   ttcaacgaga aggcgatcca taaggagggc tgtgtggaga agattggggg ctggctgagg   600
   aaaaatgtgc tggtggtagc tgcagcagcc cttggaattg cttttgtcga ggttttggga   660
   attgtctttg cctgctgcct cgtgaagagt atcagaagtg gctacgaggt gatg         714
<210> 18
   <211> 238
   <212> PRT
   <213> homo sapiens
<220>
   <221> MISC_FEATURE
   <222> (1)..(238)
   <223> CD63; translatierte Sequenz zu SEQ ID NO:17 (Genbank Accession No: KF998086)
<400> 18
   Met Ala Val Glu Gly Gly Met Lys Cys Val Lys Phe Leu Leu Tyr Val
   1               5                   10                  15
   Leu Leu Leu Ala Phe Cys Ala Cys Ala Val Gly Leu Ile Ala Val Gly
               20                  25                  30
   Val Gly Ala Gln Leu Val Leu Ser Gln Thr Ile Ile Gln Gly Ala Thr
           35                  40                  45
   Pro Gly Ser Leu Leu Pro Val Val Ile Ile Ala Val Gly Val Phe Leu
       50                  55                  60
   Phe Leu Val Ala Phe Val Gly Cys Cys Gly Ala Cys Lys Glu Asn Tyr
   65                  70                  75                  80
   Cys Leu Met Ile Thr Phe Ala Ile Phe Leu Ser Leu Ile Met Leu Val
                   85                  90                  95
   Glu Val Ala Ala Ala Ile Ala Gly Tyr Val Phe Arg Asp Lys Val Met
               100                 105                 110
   Ser Glu Phe Asn Asn Asn Phe Arg Gln Gln Met Glu Asn Tyr Pro Lys
           115                 120                 125
   Asn Asn His Thr Ala Ser Ile Leu Asp Arg Met Gln Ala Asp Phe Lys
       130                 135                 140
   Cys Cys Gly Ala Ala Asn Tyr Thr Asp Trp Glu Lys Ile Pro Ser Met
   145                 150                 155                 160
   Ser Lys Asn Arg Val Pro Asp Ser Cys Cys Ile Asp Val Thr Val Gly
                   165                 170                 175
   Cys Gly Ile Asn Phe Asn Glu Lys Ala Ile His Lys Glu Gly Cys Val
               180                 185                 190
   Glu Lys Ile Gly Gly Trp Leu Arg Lys Asn Val Leu Val Val Ala Ala
           195                 200                 205
   Ala Ala Leu Gly Ile Ala Phe Val Glu Val Leu Gly Ile Val Phe Ala
       210                 215                 220
   Cys Cys Leu Val Lys Ser Ile Arg Ser Gly Tyr Glu Val Met
   225                 230                 235
<210> 19
   <211> 771
   <212> DNA
   <213> homo sapiens
<220>
   <221> misc_feature
   <222> (18)..(21)
   <223> Startkodon
<400> 19
   gctagcgcgg ccgccaccat ggattacaag gatgacgacg ataagagccc gggcggatcc       60
   atgccggtca aaggaggcac caagtgcatc aaatacctgc tgttcggatt taacttcatc      120
   ttctggcttg ccgggattgc tgtccttgcc attggactat ggctccgatt cgactctcag      180
   accaagagca tcttcgagca agaaactaat aataataatt ccagcttcta cacaggagtc      240
   tatattctga tcggagccgg cgccctcatg atgctggtgg gcttcctggg ctgctgcggg      300
   gctgtgcagg agtcccagtg catgctggga ctgttcttcg gcttcctctt ggtgatattc      360
   gccattgaaa tagctgcggc catctgggga tattcccaca aggatgaggt gattaaggaa      420
   gtccaggagt tttacaagga cacctacaac aagctgaaaa ccaaggatga gccccagcgg      480
   gaaacgctga aagccatcca ctatgcgttg aactgctgtg gtttggctgg gggcgtggaa      540
   cagtttatct cagacatctg ccccaagaag gacgtactcg aaaccttcac cgtgaagtcc      600
   tgtcctgatg ccatcaaaga ggtcttcgac aataaattcc acatcatcgg cgcagtgggc      660
   atcggcattg ccgtggtcat gatatttggc atgatcttca gtatgatctt gtgctgtgct      720
   atccgcagga accgcgagat ggtcaagctt atcgataccg tcgacctcga g               771
<210> 20
   <211> 257
   <212> PRT
   <213> homo sapiens
<220>
   <221> MISC_FEATURE
   <222> (7)..(7)
   <223> Startmethionin
<220>
   <221> MISC_FEATURE
   <222> (7)..(257)
   <223> CD9; translatierte Sequenz zu SEQ ID NO:19 (Genbank Accession No: NM_001769.3)
<400> 20
   Ala Ser Ala Ala Ala Thr Met Asp Tyr Lys Asp Asp Asp Asp Lys Ser
   1               5                   10                  15
   Pro Gly Gly Ser Met Pro Val Lys Gly Gly Thr Lys Cys Ile Lys Tyr
               20                  25                  30
   Leu Leu Phe Gly Phe Asn Phe Ile Phe Trp Leu Ala Gly Ile Ala Val
           35                  40                  45
   Leu Ala Ile Gly Leu Trp Leu Arg Phe Asp Ser Gln Thr Lys Ser Ile
       50                  55                  60
   Phe Glu Gln Glu Thr Asn Asn Asn Asn Ser Ser Phe Tyr Thr Gly Val
   65                  70                  75                  80
   Tyr Ile Leu Ile Gly Ala Gly Ala Leu Met Met Leu Val Gly Phe Leu
                   85                  90                  95
   Gly Cys Cys Gly Ala Val Gln Glu Ser Gln Cys Met Leu Gly Leu Phe
               100                 105                 110
   Phe Gly Phe Leu Leu Val Ile Phe Ala Ile Glu Ile Ala Ala Ala Ile
           115                 120                 125
   Trp Gly Tyr Ser His Lys Asp Glu Val Ile Lys Glu Val Gln Glu Phe
       130                 135                 140
   Tyr Lys Asp Thr Tyr Asn Lys Leu Lys Thr Lys Asp Glu Pro Gln Arg
   145                 150                 155                 160
   Glu Thr Leu Lys Ala Ile His Tyr Ala Leu Asn Cys Cys Gly Leu Ala
                   165                 170                 175
   Gly Gly Val Glu Gln Phe Ile Ser Asp Ile Cys Pro Lys Lys Asp Val
               180                 185                 190
   Leu Glu Thr Phe Thr Val Lys Ser Cys Pro Asp Ala Ile Lys Glu Val
           195                 200                 205
   Phe Asp Asn Lys Phe His Ile Ile Gly Ala Val Gly Ile Gly Ile Ala
       210                 215                 220
   Val Val Met Ile Phe Gly Met Ile Phe Ser Met Ile Leu Cys Cys Ala
   225                 230                 235                 240
   Ile Arg Arg Asn Arg Glu Met Val Lys Leu Ile Asp Thr Val Asp Leu
                   245                 250                 255
   Glu
<210> 21
   <211> 795
   <212> DNA
   <213> homo sapiens
<220>
   <221> misc_feature
   <222> (18)..(21)
   <223> Startkodon
<400> 21
   gctagcgcgg ccgccaccat ggattacaag gatgacgacg ataagagccc gggcggatcc      60
   atgggagtgg agggctgcac caagtgcatc aagtacctgc tcttcgtctt caatttcgtc     120
   ttctggctgg ctggaggcgt gatcctgggt gtggccctgt ggctccgcca tgacccgcag     180
   accaccaacc tcctgtatct ggagctggga gacaagcccg cgcccaacac cttctatgta     240
   ggcatctaca tcctcatcgc tgtgggcgct gtcatgatgt tcgttggctt cctgggctgc     300
   tacggggcca tccaggaatc ccagtgcctg ctggggacgt tcttcacctg cctggtcatc     360
   ctgtttgcct gtgaggtggc cgccggcatc tggggctttg tcaacaagga ccagatcgcc     420
   aaggatgtga agcagttcta tgaccaggcc ctacagcagg ccgtggtgga tgatgacgcc     480
   aacaacgcga aggctgtggt gaagaccttc cacgagacgc ttgactgctg tggctccagc     540
   acactgactg ctttgaccac ctcagtgctc aagaacaatt tgtgtccctc gggcagcaac     600
   atcatcagca acctcttcaa ggaggactgc caccagaaga tcgacgacct cttctccggg     660
   aagctgtacc tcatcggcat tgctgccatc gtggtcgctg tgatcatgat cttcgagatg     720
   atcctgagca tggtgctgtg ctgtggcatc cggaacagct ccgtgtacaa gcttatcgat     780
   accgtcgacc tcgag                                                      795
<210> 22
   <211> 265
   <212> PRT
   <213> homo sapiens
<220>
   <221> MISC_FEATURE
   <222> (7)..(7)
   <223> Startmethionin
<220>
   <221> MISC_FEATURE
   <222> (7)..(265)
   <223> CD81; translatierte Sequenz zu SEQ ID NO: 21 (Genbank Accession No: NM_004356.3)
<400> 22
   Ala Ser Ala Ala Ala Thr Met Asp Tyr Lys Asp Asp Asp Asp Lys Ser
   1               5                   10                  15
   Pro Gly Gly Ser Met Gly Val Glu Gly Cys Thr Lys Cys Ile Lys Tyr
               20                  25                  30
   Leu Leu Phe Val Phe Asn Phe Val Phe Trp Leu Ala Gly Gly Val Ile
           35                  40                  45
   Leu Gly Val Ala Leu Trp Leu Arg His Asp Pro Gln Thr Thr Asn Leu
       50                  55                  60
   Leu Tyr Leu Glu Leu Gly Asp Lys Pro Ala Pro Asn Thr Phe Tyr Val
   65                  70                  75                  80
   Gly Ile Tyr Ile Leu Ile Ala Val Gly Ala Val Met Met Phe Val Gly
                   85                  90                  95
   Phe Leu Gly Cys Tyr Gly Ala Ile Gln Glu Ser Gln Cys Leu Leu Gly
               100                 105                 110
   Thr Phe Phe Thr Cys Leu Val Ile Leu Phe Ala Cys Glu Val Ala Ala
           115                 120                 125
   Gly Ile Trp Gly Phe Val Asn Lys Asp Gln Ile Ala Lys Asp Val Lys
       130                 135                 140
   Gln Phe Tyr Asp Gln Ala Leu Gln Gln Ala Val Val Asp Asp Asp Ala
   145                 150                 155                 160
   Asn Asn Ala Lys Ala Val Val Lys Thr Phe His Glu Thr Leu Asp Cys
                   165                 170                 175
   Cys Gly Ser Ser Thr Leu Thr Ala Leu Thr Thr Ser Val Leu Lys Asn
               180                 185                 190
   Asn Leu Cys Pro Ser Gly Ser Asn Ile Ile Ser Asn Leu Phe Lys Glu
           195                 200                 205
   Asp Cys His Gln Lys Ile Asp Asp Leu Phe Ser Gly Lys Leu Tyr Leu
       210                 215                 220
   Ile Gly Ile Ala Ala Ile Val Val Ala Val Ile Met Ile Phe Glu Met
   225                 230                 235                 240
   Ile Leu Ser Met Val Leu Cys Cys Gly Ile Arg Asn Ser Ser Val Tyr
                   245                 250                 255
   Lys Leu Ile Asp Thr Val Asp Leu Glu
   260 265
<210> 23
   <211> 888
   <212> DNA
   <213> homo sapiens
<220>
   <221> misc_feature
   <222> (18)..(21)
   <223> Startkodon
<400> 23
   gctagcgcgg ccgccaccat ggattacaag gatgacgacg ataagagccc gggcggatcc      60
   atgggctcag cctgtatcaa agtcaccaaa tactttctct tcctcttcaa cttgatcttc     120
   tttatcctgg gcgcagtgat cctgggcttc ggggtgtgga tcctggccga caagagcagt     180
   ttcatctctg tcctgcaaac ctcctccagc tcgcttagga tgggggccta tgtcttcatc     240
   ggcgtggggg cagtcactat gctcatgggc ttcctgggct gcatcggcgc cgtcaacgag     300
   gtccgctgcc tgctggggct gtactttgct ttcctgctcc tgatcctcat tgcccaggtg     360
   acggccgggg cactcttcta cttcaacatg ggcaagctga agcaggagat gggtggcatc     420
   gtgactgagc tcattcgaga ctacaacagc agtcgcgagg acagcctgca ggatgcctgg     480
   gactacgtgc aggctcaggt gaagtgctgc ggctgggtca gcttctacaa ctggacagac     540
   aacgctgagc tcatgaatcg ccctgaggtc acctacccct gttcctgcga agtcaagggg     600
   gaagaggaca acagcctttc tgtgaggaag ggcttctgcg aggcccccgg caacaggacc     660
   cagagtggca accaccctga ggactggcct gtgtaccagg agggctgcat ggagaaggtg     720
   caggcgtggc tgcaggagaa cctgggcatc atcctcggcg tgggcgtggg tgtggccatg     780
   gtcgagctcc tggggatggt cctgtccatc tgcttgtgcc ggcacgtgca ttccgaagac     840
   tacagcaagg tccccaagta caagcttatc gataccgtcg acctcgag                  888
<210> 24
   <211> 296
   <212> PRT
   <213> homo sapiens
<220>
   <221> MISC_FEATURE
   <222> (7) .. (7)
   <223> Startmethionin
<220>
   <221> MISC_FEATURE
   <222> (7) .. (296)
   <223> CD82; translatierte Sequenz zu SEQ ID NO: 23 (Genbank Accession No: NM_002231.3)
<400> 24
   Ala Ser Ala Ala Ala Thr Met Asp Tyr Lys Asp Asp Asp Asp Lys Ser
   1               5                   10                  15
   Pro Gly Gly Ser Met Gly Ser Ala Cys Ile Lys Val Thr Lys Tyr Phe
               20                  25                  30
   Leu Phe Leu Phe Asn Leu Ile Phe Phe Ile Leu Gly Ala Val Ile Leu
           35                  40                  45
   Gly Phe Gly Val Trp Ile Leu Ala Asp Lys Ser Ser Phe Ile Ser Val
       50                  55                  60
   Leu Gln Thr Ser Ser Ser Ser Leu Arg Met Gly Ala Tyr Val Phe Ile
   65                  70                  75                  80
   Gly Val Gly Ala Val Thr Met Leu Met Gly Phe Leu Gly Cys Ile Gly
                   85                  90                  95
   Ala Val Asn Glu Val Arg Cys Leu Leu Gly Leu Tyr Phe Ala Phe Leu
               100                 105                 110
   Leu Leu Ile Leu Ile Ala Gln Val Thr Ala Gly Ala Leu Phe Tyr Phe
           115                 120                 125
   Asn Met Gly Lys Leu Lys Gln Glu Met Gly Gly Ile Val Thr Glu Leu
       130                 135                 140
   Ile Arg Asp Tyr Asn Ser Ser Arg Glu Asp Ser Leu Gln Asp Ala Trp
   145                 150                 155                 160
   Asp Tyr Val Gln Ala Gln Val Lys Cys Cys Gly Trp Val Ser Phe Tyr
                   165                 170                 175
   Asn Trp Thr Asp Asn Ala Glu Leu Met Asn Arg Pro Glu Val Thr Tyr
               180                 185                 190
   Pro Cys Ser Cys Glu Val Lys Gly Glu Glu Asp Asn Ser Leu Ser Val
           195                 200                 205
   Arg Lys Gly Phe Cys Glu Ala Pro Gly Asn Arg Thr Gln Ser Gly Asn
       210                 215                 220
   His Pro Glu Asp Trp Pro Val Tyr Gln Glu Gly Cys Met Glu Lys Val
   225                 230                 235                 240
   Gln Ala Trp Leu Gln Glu Asn Leu Gly Ile Ile Leu Gly Val Gly Val
   245 250 255
   Gly Val Ala Met Val Glu Leu Leu Gly Met Val Leu Ser Ile Cys Leu
   260 265 270
   Cys Arg His Val His Ser Glu Asp Tyr Ser Lys Val Pro Lys Tyr Lys
   275 280 285
   Leu Ile Asp Thr Val Asp Leu Glu
   290 295
<210> 25
   <211> 846
   <212> DNA
   <213> homo sapiens
<220>
   <221> misc_feature
   <222> (18)..(21)
   <223> Startkodon
<400> 25
   gctagcgcgg ccgccaccat ggattacaag gatgacgacg ataagagccc gggcggatcc      60
   atgggtgagt tcaacgagaa gaagacaaca tgtggcaccg tttgcctcaa gtacctgctg     120
   tttacctaca attgctgctt ctggctggct ggcctggctg tcatggcagt gggcatctgg     180
   acgctggccc tcaagagtga ctacatcagc ctgctggcct caggcaccta cctggccaca     240
   gcctacatcc tggtggtggc gggcactgtc gtcatggtga ctggggtctt gggctgctgc     300
   gccaccttca aggagcgtcg gaacctgctg cgcctgtact tcatcctgct cctcatcatc     360
   tttctgctgg agatcatcgc tggtatcctc gcctacgcct actaccagca gctgaacacg     420
   gagctcaagg agaacctgaa ggacaccatg accaagcgct accaccagcc gggccatgag     480
   gctgtgacca gcgctgtgga ccagctgcag caggagttcc actgctgtgg cagcaacaac     540
   tcacaggact ggcgagacag tgagtggatc cgctcacagg aggccggtgg ccgtgtggtc     600
   ccagacagct gctgcaagac ggtggtggct ctttgtggac agcgagacca tgcctccaac     660
   atctacaagg tggagggcgg ctgcatcacc aagttggaga ccttcatcca ggagcacctg     720
   agggtcattg ggctgtgggg gatcggcatt gcctgtgtgc aggtctttgg catgatcttc     780
   acgtgctgcc tgtacaggag tctcaagctg gagcactaca agcttatcga taccgtcgac     840
   ctcgag                                                                846
<210> 26
   <211> 282
   <212> PRT
   <213> homo sapiens
<220>
   <221> MISC_FEATURE
   <222> (7)..(7)
   <223> Startkodon
<220>
   <221> MISC_FEATURE
   <222> (7) .. (7)
   <223> Startmethionin
<220>
   <221> MISC_FEATURE
   <222> (8)..(282)
   <223> CD151; translatierte Sequenz zu SEQ ID No: 25 (Genbank Accession No: BT007397.1)
<400> 26
   Ala Ser Ala Ala Ala Thr Met Asp Tyr Lys Asp Asp Asp Asp Lys Ser
   1               5                   10                  15
   Pro Gly Gly Ser Met Gly Glu Phe Asn Glu Lys Lys Thr Thr Cys Gly
               20                  25                  30
   Thr Val Cys Leu Lys Tyr Leu Leu Phe Thr Tyr Asn Cys Cys Phe Trp
           35                  40                  45
   Leu Ala Gly Leu Ala Val Met Ala Val Gly Ile Trp Thr Leu Ala Leu
       50                  55                  60
   Lys Ser Asp Tyr Ile Ser Leu Leu Ala Ser Gly Thr Tyr Leu Ala Thr
   65                  70                  75                  80
   Ala Tyr Ile Leu Val Val Ala Gly Thr Val Val Met Val Thr Gly Val
                   85                  90                  95
   Leu Gly Cys Cys Ala Thr Phe Lys Glu Arg Arg Asn Leu Leu Arg Leu
               100                 105                 110
   Tyr Phe Ile Leu Leu Leu Ile Ile Phe Leu Leu Glu Ile Ile Ala Gly
           115                 120                 125
   Ile Leu Ala Tyr Ala Tyr Tyr Gln Gln Leu Asn Thr Glu Leu Lys Glu
       130                 135                 140
   Asn Leu Lys Asp Thr Met Thr Lys Arg Tyr His Gln Pro Gly His Glu
   145                 150                 155                 160
   Ala Val Thr Ser Ala Val Asp Gln Leu Gln Gln Glu Phe His Cys Cys
                   165                 170                 175
   Gly Ser Asn Asn Ser Gln Asp Trp Arg Asp Ser Glu Trp Ile Arg Ser
               180                 185                 190
   Gln Glu Ala Gly Gly Arg Val Val Pro Asp Ser Cys Cys Lys Thr Val
           195                 200                 205
   Val Ala Leu Cys Gly Gln Arg Asp His Ala Ser Asn Ile Tyr Lys Val
       210                 215                 220
   Glu Gly Gly Cys Ile Thr Lys Leu Glu Thr Phe Ile Gln Glu His Leu
   225                 230                 235                 240
   Arg Val Ile Gly Ala Val Gly Ile Gly Ile Ala Cys Val Gln Val Phe
                   245                 250                 255
   Gly Met Ile Phe Thr Cys Cys Leu Tyr Arg Ser Leu Lys Leu Glu His
               260                 265                 270
   Tyr Lys Leu Ile Asp Thr Val Asp Leu Glu
           275                 280
<210> 27
   <211> 648
   <212> DNA
   <213> artificial sequence
<220>
   <223> CD9delLEL-V5-6xHis; basierend auf Genbank Accession No.: NM_001769.3
<220>
   <221> misc_feature
   <222> (18)..(405)
   <223> TM1-TM3
<220>
   <221> misc_feature
   <222> (18)..(21)
   <223> Startkodon
<220>
   <221> misc_feature
   <222> (406)..(507)
   <223> V5-6xHis-Linker
<220>
   <221> misc_feature
   <222> (508) .. (648)
   <223> TM4
<400> 27
   gctagcgcgg ccgccaccat ggattacaag gatgacgacg ataagagccc gggcggatcc      60
   atgccggtca aaggaggcac caagtgcatc aaatacctgc tgttcggatt taacttcatc     120
   ttctggcttg ccgggattgc tgtccttgcc attggactat ggctccgatt cgactctcag     180
   accaagagca tcttcgagca agaaactaat aataataatt ccagcttcta cacaggagtc     240
   tatattctga tcggagccgg cgccctcatg atgctggtgg gcttcctggg ctgctgcggg     300
   gctgtgcagg agtcccagtg catgctggga ctgttcttcg gcttcctctt ggtgatattc     360
   gccattgaaa tagctgcggc catctgggga tattcccaca aggatgaatt cggaaagggc     420
   ccgcggttcg aaggtaagcc tatccctaac cctctcctcg gtctcgattc tacgcgtacc     480
   ggtcatcatc accatcacca tgatatcgga ggaggaggaa tcatcggcgc agtgggcatc     540
   ggcattgccg tggtcatgat atttggcatg atcttcagta tgatcttgtg ctgtgctatc     600
   cgcaggaacc gcgagatggt caagcttatc gataccgtcg acctcgag                  648
<210> 28
   <211> 216
   <212> PRT
   <213> artificial sequence
<220>
   <223> CD9delLEL-V5-6xHis; translatierte Sequenz
<220>
   <221> MISC_FEATURE
   <222> (7) .. (7)
   <223> Startmethionin
<220>
   <221> MISC_FEATURE
   <222> (7) .. (135)
   <223> TM1-TM3
<220>
   <221> MISC_FEATURE
   <222> (7) .. (216)
   <223> CD9delLEL-V5-6xHis
<220>
   <221> MISC_FEATURE
   <222> (136)..(169)
   <223> V5-6xHis-Linker
<220>
   <221> MISC_FEATURE
   <222> (170)..(216)
   <223> TM4
<400> 28
   Ala Ser Ala Ala Ala Thr Met Asp Tyr Lys Asp Asp Asp Asp Lys Ser
   1               5                   10                  15
   Pro Gly Gly Ser Met Pro Val Lys Gly Gly Thr Lys Cys Ile Lys Tyr
               20                  25                  30
   Leu Leu Phe Gly Phe Asn Phe Ile Phe Trp Leu Ala Gly Ile Ala Val
           35                  40                  45
   Leu Ala Ile Gly Leu Trp Leu Arg Phe Asp Ser Gln Thr Lys Ser Ile
       50                  55                  60
   Phe Glu Gln Glu Thr Asn Asn Asn Asn Ser Ser Phe Tyr Thr Gly Val
   65                  70                  75                  80
   Tyr Ile Leu Ile Gly Ala Gly Ala Leu Met Met Leu Val Gly Phe Leu
                   85                  90                  95
   Gly Cys Cys Gly Ala Val Gln Glu Ser Gln Cys Met Leu Gly Leu Phe
               100                 105                 110
   Phe Gly Phe Leu Leu Val Ile Phe Ala Ile Glu Ile Ala Ala Ala Ile
           115                 120                 125
   Trp Gly Tyr Ser His Lys Asp Glu Phe Gly Lys Gly Pro Arg Phe Glu
       130                 135                 140
   Gly Lys Pro Ile Pro Asn Pro Leu Leu Gly Leu Asp Ser Thr Arg Thr
   145                 150                 155                 160
   Gly His His His His His His Asp Ile Gly Gly Gly Gly Ile Ile Gly
                   165                 170                 175
   Ala Val Gly Ile Gly Ile Ala Val Val Met Ile Phe Gly Met Ile Phe
               180                 185                 190
   Ser Met Ile Leu Cys Cys Ala Ile Arg Arg Asn Arg Glu Met Val Lys
           195                 200                 205
   Leu Ile Asp Thr Val Asp Leu Glu
       210                 215
<210> 29
   <211> 663
   <212> DNA
   <213> artificial sequence
<220>
   <223> CD81delLEL-V5-6xHis; basierend auf Genbank Accession No.: NM_004356.3
<220>
   <221> misc_feature
   <222> (18)..(413)
   <223> TM1-TM3
<220>
   <221> misc_feature
   <222> (18)..(21)
   <223> Startkodon
<220>
   <221> misc_feature
   <222> (414)..(516)
   <223> V5-6xHis-Linker
<220>
   <221> misc_feature
   <222> (517) .. (663)
   <223> TM4
<400> 29
   gctagcgcgg ccgccaccat ggattacaag gatgacgacg ataagagccc gggcggatcc      60
   atgggagtgg agggctgcac caagtgcatc aagtacctgc tcttcgtctt caatttcgtc     120
   ttctggctgg ctggaggcgt gatcctgggt gtggccctgt ggctccgcca tgacccgcag     180
   accaccaacc tcctgtatct ggagctggga gacaagcccg cgcccaacac cttctatgta     240
   ggcatctaca tcctcatcgc tgtgggcgct gtcatgatgt tcgttggctt cctgggctgc     300
   tacggggcca tccaggaatc ccagtgcctg ctggggacgt tcttcacctg cctggtcatc     360
   ctgtttgcct gtgaggtggc cgccggcatc tggggctttg tcaacaagga ccaggaattc     420
   ggaaagggcc cgcggttcga aggtaagcct atccctaacc ctctcctcgg tctcgattct     480
   acgcgtaccg gtcatcatca ccatcaccat gatatcggag gaggaggaaa gctgtacctc     540
   atcggcattg ctgccatcgt ggtcgctgtg atcatgatct tcgagatgat cctgagcatg     600
   gtgctgtgct gtggcatccg gaacagctcc gtgtacaagc ttatcgatac cgtcgacctc     660
   gag                                                                   663
<210> 30
   <211> 221
   <212> PRT
   <213> artificial sequence
<220>
   <223> CD81delLEL-V5-6xHis; translatierte Sequenz
<220>
   <221> MISC_FEATURE
   <222> (7)..(7)
   <223> Startkodon
<220>
   <221> MISC_FEATURE
   <222> (7) .. (7)
   <223> Startmethionin
<220>
   <221> MISC_FEATURE
   <222> (7) .. (138)
   <223> TM1-TM3
<220>
   <221> MISC_FEATURE
   <222> (7)..(221)
   <223> CD81delLEL-V5-6xHis
<220>
   <221> MISC_FEATURE
   <222> (139)..(172)
   <223> V5-6xHis-Linker
<220>
   <221> MISC_FEATURE
   <222> (173)..(221)
   <223> TM4
<400> 30
   Ala Ser Ala Ala Ala Thr Met Asp Tyr Lys Asp Asp Asp Asp Lys Ser
   1               5                   10                  15
   Pro Gly Gly Ser Met Gly Val Glu Gly Cys Thr Lys Cys Ile Lys Tyr
               20                  25                  30
   Leu Leu Phe Val Phe Asn Phe Val Phe Trp Leu Ala Gly Gly Val Ile
           35                  40                  45
   Leu Gly Val Ala Leu Trp Leu Arg His Asp Pro Gln Thr Thr Asn Leu
       50                  55                  60
   Leu Tyr Leu Glu Leu Gly Asp Lys Pro Ala Pro Asn Thr Phe Tyr Val
   65                  70                  75                  80
   Gly Ile Tyr Ile Leu Ile Ala Val Gly Ala Val Met Met Phe Val Gly
                   85                  90                  95
   Phe Leu Gly Cys Tyr Gly Ala Ile Gln Glu Ser Gln Cys Leu Leu Gly
               100                 105                 110
   Thr Phe Phe Thr Cys Leu Val Ile Leu Phe Ala Cys Glu Val Ala Ala
           115                 120                 125
   Gly Ile Trp Gly Phe Val Asn Lys Asp Gln Glu Phe Gly Lys Gly Pro
       130                 135                 140
   Arg Phe Glu Gly Lys Pro Ile Pro Asn Pro Leu Leu Gly Leu Asp Ser
   145                 150                 155                 160
   Thr Arg Thr Gly His His His His His His Asp Ile Gly Gly Gly Gly
                   165                 170                 175
   Lys Leu Tyr Leu Ile Gly Ile Ala Ala Ile Val Val Ala Val Ile Met
               180                 185                 190
   Ile Phe Glu Met Ile Leu Ser Met Val Leu Cys Cys Gly Ile Arg Asn
           195                 200                 205
   Ser Ser Val Tyr Lys Leu Ile Asp Thr Val Asp Leu Glu
       210                 215                 220
<210> 31
   <211> 690
   <212> DNA
   <213> artificial sequence
<220>
   <223> CD82delLEL-V5-6xHis; basierend auf Genbank Accession No.: NM_002231.3
<220>
   <221> misc_feature
   <222> (18)..(21)
   <223> Startkodon
<220>
   <221> misc_feature
   <222> (18)..(405)
   <223> TM1-TM3
<220>
   <221> misc_feature
   <222> (406)..(507)
   <223> V5-6xHis-Linker
<220>
   <221> misc_feature
   <222> (508) .. (690)
   <223> TM4
<400> 31
   gctagcgcgg ccgccaccat ggattacaag gatgacgacg ataagagccc gggcggatcc      60
   atgggctcag cctgtatcaa agtcaccaaa tactttctct tcctcttcaa cttgatcttc     120
   tttatcctgg gcgcagtgat cctgggcttc ggggtgtgga tcctggccga caagagcagt     180
   ttcatctctg tcctgcaaac ctcctccagc tcgcttagga tgggggccta tgtcttcatc     240
   ggcgtggggg cagtcactat gctcatgggc ttcctgggct gcatcggcgc cgtcaacgag     300
   gtccgctgcc tgctggggct gtactttgct ttcctgctcc tgatcctcat tgcccaggtg     360
   acggccgggg cactcttcta cttcaacatg ggcaagctga agcaggaatt cggaaagggc     420
   ccgcggttcg aaggtaagcc tatccctaac cctctcctcg gtctcgattc tacgcgtacc     480
   ggtcatcatc accatcacca tgatatcgga ggaggaggag tgcaggcgtg gctgcaggag     540
   aacctgggca tcatcctcgg cgtgggcgtg ggtgtggcca tggtcgagct cctggggatg     600
   gtcctgtcca tctgcttgtg ccggcacgtg cattccgaag actacagcaa ggtccccaag     660
   tacaagctta tcgataccgt cgacctcgag                                      690
<210> 32
   <211> 230
   <212> PRT
   <213> artificial sequence
<220>
   <223> CD82delLEL-V5-6xHis; translatierte Sequenz
<220>
   <221> MISC_FEATURE
   <222> (7)..(7)
   <223> Startmethionin
<220>
   <221> MISC_FEATURE
   <222> (7) .. (135)
   <223> TM1-TM3
<220>
   <221> MISC_FEATURE
   <222> (7)..(230)
   <223> CD82delLEL-V5-6xHis
<220>
   <221> MISC_FEATURE
   <222> (136)..(169)
   <223> V5-6xHis-Linker
<220>
   <221> MISC_FEATURE
   <222> (170)..(230)
   <223> TM4
<400> 32
   Ala Ser Ala Ala Ala Thr Met Asp Tyr Lys Asp Asp Asp Asp Lys Ser
   1               5                   10                  15
   Pro Gly Gly Ser Met Gly Ser Ala Cys Ile Lys Val Thr Lys Tyr Phe
               20                  25                  30
   Leu Phe Leu Phe Asn Leu Ile Phe Phe Ile Leu Gly Ala Val Ile Leu
           35                  40                  45
   Gly Phe Gly Val Trp Ile Leu Ala Asp Lys Ser Ser Phe Ile Ser Val
       50                  55                  60
   Leu Gln Thr Ser Ser Ser Ser Leu Arg Met Gly Ala Tyr Val Phe Ile
   65                  70                  75                  80
   Gly Val Gly Ala Val Thr Met Leu Met Gly Phe Leu Gly Cys Ile Gly
                   85                  90                  95
   Ala Val Asn Glu Val Arg Cys Leu Leu Gly Leu Tyr Phe Ala Phe Leu
               100                 105                 110
   Leu Leu Ile Leu Ile Ala Gln Val Thr Ala Gly Ala Leu Phe Tyr Phe
           115                 120                 125
   Asn Met Gly Lys Leu Lys Gln Glu Phe Gly Lys Gly Pro Arg Phe Glu
       130                 135                 140
   Gly Lys Pro Ile Pro Asn Pro Leu Leu Gly Leu Asp Ser Thr Arg Thr
   145                 150                 155                 160
   Gly His His His His His His Asp Ile Gly Gly Gly Gly Val Gln Ala
                   165                 170                 175
   Trp Leu Gln Glu Asn Leu Gly Ile Ile Leu Gly Val Gly Val Gly Val
               180                 185                 190
   Ala Met Val Glu Leu Leu Gly Met Val Leu Ser Ile Cys Leu Cys Arg
           195                 200                 205
   His Val His Ser Glu Asp Tyr Ser Lys Val Pro Lys Tyr Lys Leu Ile
       210                 215                 220
   Asp Thr Val Asp Leu Glu
   225                 230
<210> 33
   <211> 663
   <212> DNA
   <213> artificial sequence
<220>
   <223> CD151delLEL-V5-6xHis; basierend auf Genbank Accession No.: BT001769.3
<220>
   <221> misc_feature
   <222> (18)..(21)
   <223> Startkodon
<220>
   <221> misc_feature
   <222> (18)..(420)
   <223> TM1-TM3
<220>
   <221> misc_feature
   <222> (421)..(522)
   <223> V5-6xHis-Linker
<220>
   <221> misc_feature
   <222> (523)..(663)
   <223> TM4
<400> 33
   gctagcgcgg ccgccaccat ggattacaag gatgacgacg ataagagccc gggcggatcc      60
   atgggtgagt tcaacgagaa gaagacaaca tgtggcaccg tttgcctcaa gtacctgctg     120
   tttacctaca attgctgctt ctggctggct ggcctggctg tcatggcagt gggcatctgg     180
   acgctggccc tcaagagtga ctacatcagc ctgctggcct caggcaccta cctggccaca     240
   gcctacatcc tggtggtggc gggcactgtc gtcatggtga ctggggtctt gggctgctgc     300
   gccaccttca aggagcgtcg gaacctgctg cgcctgtact tcatcctgct cctcatcatc     360
   tttctgctgg agatcatcgc tggtatcctc gcctacgcct actaccagca gctgaacacg     420
   gaattcggaa agggcccgcg gttcgaaggt aagcctatcc ctaaccctct cctcggtctc     480
   gattctacgc gtaccggtca tcatcaccat caccatgata tcggaggagg aggactgagg     540
   gtcattgggg ctgtggggat cggcattgcc tgtgtgcagg tctttggcat gatcttcacg     600
   tgctgcctgt acaggagtct caagctggag cactacaagc ttatcgatac cgtcgacctc     660
   gag                                                                   663
<210> 34
   <211> 221
   <212> PRT
   <213> artificial sequence
<220>
   <223> CD151delLEL-V5-6xHis; translatierte Sequenz
<220>
   <221> MISC_FEATURE
   <222> (7) .. (7)
   <223> Startmethionin
<220>
   <221> MISC_FEATURE
   <222> (7)..(140)
   <223> TM1-TM3
<220>
   <221> MISC_FEATURE
   <222> (7)..(221)
   <223> CD151delLEL-V5-6xHis
<220>
   <221> MISC_FEATURE
   <222> (141)..(174)
   <223> V5-6xHis-Linker
<220>
   <221> MISC_FEATURE
   <222> (175)..(221)
   <223> TM4
<400> 34
   Ala Ser Ala Ala Ala Thr Met Asp Tyr Lys Asp Asp Asp Asp Lys Ser
   1               5                   10                  15
   Pro Gly Gly Ser Met Gly Glu Phe Asn Glu Lys Lys Thr Thr Cys Gly
               20                  25                  30
   Thr Val Cys Leu Lys Tyr Leu Leu Phe Thr Tyr Asn Cys Cys Phe Trp
           35                  40                  45
   Leu Ala Gly Leu Ala Val Met Ala Val Gly Ile Trp Thr Leu Ala Leu
       50                  55                  60
   Lys Ser Asp Tyr Ile Ser Leu Leu Ala Ser Gly Thr Tyr Leu Ala Thr
   65                  70                  75                  80
   Ala Tyr Ile Leu Val Val Ala Gly Thr Val Val Met Val Thr Gly Val
                   85                  90                  95
   Leu Gly Cys Cys Ala Thr Phe Lys Glu Arg Arg Asn Leu Leu Arg Leu
               100                 105                 110
   Tyr Phe Ile Leu Leu Leu Ile Ile Phe Leu Leu Glu Ile Ile Ala Gly
           115                 120                 125
   Ile Leu Ala Tyr Ala Tyr Tyr Gln Gln Leu Asn Thr Glu Phe Gly Lys
       130                 135                 140
   Gly Pro Arg Phe Glu Gly Lys Pro Ile Pro Asn Pro Leu Leu Gly Leu
   145                 150                 155                 160
   Asp Ser Thr Arg Thr Gly His His His His His His Asp Ile Gly Gly
                   165                 170                 175
   Gly Gly Leu Arg Val Ile Gly Ala Val Gly Ile Gly Ile Ala Cys Val
               180                 185                 190
   Gln Val Phe Gly Met Ile Phe Thr Cys Cys Leu Tyr Arg Ser Leu Lys
           195                 200                 205
   Leu Glu His Tyr Lys Leu Ile Asp Thr Val Asp Leu Glu
       210                 215                 220
<210> 35
   <211> 102
   <212> DNA
   <213> artificial sequence
<220>
   <223> V5-6xHis-Linker in Tetraspanin delLEL-V5-6xHis Konstrukten
<220>
   <221> misc_feature
   <222> (1)..(6)
   <223> EcoRI Schnittstelle
<220>
   <221> misc_feature
   <222> (97)..(102)
   <223> EcoRV Schnittstelle
<400> 35
   gaattcggaa agggcccgcg gttcgaaggt aagcctatcc ctaaccctct cctcggtctc        60
   gattctacgc gtaccggtca tcatcaccat caccatgata tc                          102
<210> 36
   <211> 34
   <212> PRT
   <213> artificial sequence
<220>
   <223> V5-6xHis-Linker in Tetraspanin delLEL-V5-6xHis Konstrukten: translatierte Sequenz
<220>
   <221> MISC_FEATURE
   <222> (3)..(23)
   <223> V5-Epitop
<220>
   <221> MISC_FEATURE
   <222> (27)..(32)
   <223> 6xHis-Epitop
<400> 36
   Glu Phe Gly Lys Gly Pro Arg Phe Glu Gly Lys Pro Ile Pro Asn Pro
   1               5                   10                  15
   Leu Leu Gly Leu Asp Ser Thr Arg Thr Gly His His His His His His
               20                  25                  30
   Asp Ile
<210> 37
   <211> 126
   <212> DNA
   <213> artificial sequence
<220>
   <223> 2F5-4E10-Linker
<220>
   <221> misc_feature
   <222> (1) .. (6)
   <223> EcoRI Schnittstelle
<220>
   <221> misc_feature
   <222> (121) .. (126)
   <223> EcoRV Schnittstelle
<400> 37
   gaattcattg aagaatcgca aaaccagcaa gaaaagaatg aacaagaatt attggaatta        60
   gataaatggg caagtttgtg gaattggttt aacataacaa attggctgtg gtatataaaa       120
   gatatc                                                                  126
<210> 38
   <211> 42
   <212> PRT
   <213> artificial sequence
<220>
   <223> 2F5-4E10-Linker: translatierte Sequenz
<220>
   <221> MISC_FEATURE
   <222> (19) .. (24)
   <223> 2F5-Epitop
<220>
   <221> MISC_FEATURE
   <222> (28) .. (33)
   <223> 4E10-Epitop
<400> 38
   Glu Phe Ile Glu Glu Ser Gln Asn Gln Gln Glu Lys Asn Glu Gln Glu
   1               5                   10                  15
   Leu Leu Leu Glu Asp Lys Trp Ala Ser Leu Trp Asn Trp Phe Asn Ile
               20                  25                  30
   Thr Asn Trp Leu Trp Tyr Ile Lys Asp Ile
           35                  40
<210> 39
   <211> 120
   <212> DNA
   <213> Artificial Sequence
<220>
   <223> Linker version (8)
<220>
   <221> misc_feature
   <222> (1)..(6)
   <223> EcoRI Schnittstelle
<220>
   <221> misc_feature
   <222> (7).. (26)
   <223> TEV-Proteolyse-I
<220>
   <221> misc_feature
   <222> (27)..(33)
   <223> HindIII Schnittstelle
<220>
   <221> misc_feature
   <222> (34)..(39)
   <223> BamHI Schnittstelle
<220>
   <221> misc_feature
   <222> (40)..(46)
   <223> PstI Schnittstelle
<220>
   <221> misc_feature
   <222> (46)..(63)
   <223> His6-Tag
<220>
   <221> misc_feature
   <222> (64)..(84)
   <223> TEV-Proteolyse-II
<220>
   <221> misc_feature
   <222> (85) .. (97)
   <223> c-Myc-Epitope1
<220>
   <221> misc_feature
   <222> (103)..(114)
   <223> c-Myc-Epitope2
<220>
   <221> misc_feature
   <222> (115)..(120)
   <223> EcoRV Schnittstelle
<400> 39
   gaattcgaga atctgtactt tcagtcgaag cttggatccc tgcagcatca ccatcaccat       60
   cacgaaaacc tctatttcca gagcgaacag aaactgatat ccgaagaaga tctggatatc      120
<210> 40
   <211> 40
   <212> PRT
   <213> Artificial Sequence
<220>
   <223> Linker version (8), translatierte Sequenz
<220>
   <221> MISC_FEATURE
   <222> (3)..(8)
   <223> TEV-Proteolysis-I
<220>
   <221> MISC_FEATURE
   <222> (16) .. (21)
   <223> His6-Tag
<220>
   <221> MISC_FEATURE
   <222> (22) .. (28)
   <223> TEV-Proteolysis-I
<220>
   <221> MISC_FEATURE
   <222> (29) .. (33)
   <223> c-Myc-Epitope1
<220>
   <221> MISC_FEATURE
   <222> (34) .. (38)
   <223> c-Myc-Epitope2
<400> 40
   Glu Phe Glu Asn Leu Tyr Phe Gln Ser Lys Leu Gly Ser Leu Gln His
   1               5                   10                  15
   His His His His His Glu Asn Leu Tyr Phe Gln Ser Glu Gln Lys Leu
               20                  25                  30
   Ile Ser Glu Glu Asp Leu Asp Ile
           35                  40
<210> 41
   <211> 27
   <212> DNA
   <213> artificial sequence
<220>
   <223> Linker im Vektor pCMV-CD63de1LEL-mCherry
   (Minimallinker (9))
<220>
   <221> misc_feature
   <222> (1)..(6)
   <223> PstI Schnittstelle
<220>
   <221> misc_feature
   <222> (7)..(12)
   <223> EcoRI Schnittstelle
<220>
   <221> misc_feature
   <222> (13)..(18)
   <223> EcoRV Schnittstelle
<220>
   <221> misc_feature
   <222> (19) .. (27)
   <223> 4x Glycin
<400> 41
   ctgcaggaat tcgatatcgg aggagga 27
<210> 42
   <211> 10
   <212> PRT
   <213> artificial sequence
<220>
   <223> Linker im Vektor pCMV-CD63delLEL-mCherry: translatierte Sequenz (Minimallinker (9))
<400> 42
   Leu Gln Glu Phe Asp Ile Gly Gly Gly Gly
   1               5                   10
<210> 43
   <211> 41
   <212> DNA
   <213> artificial sequence
<220>
   <223> Linker im Vektor pCMV-CD63delLEL-V2-mCherry
<220>
   <221> misc_feature
   <222> (3)..(8)
   <223> BamHI Schnittstelle
<220>
   <221> misc_feature
   <222> (18)..(23)
   <223> BglIII Schnittstelle
<220>
   <221> misc_feature
   <222> (24) .. (29)
   <223> PstI Schnittstelle
<400> 43
   ttggatccag cggccgcaga tctctgcagg gaggcggagg c 41
<210> 44
   <211> 13
   <212> PRT
   <213> artificial sequence
<220>
   <223> Linker im Vektor pCMV-CD63delLEL-V2-mCherry: translatierte Sequenz (Linker)
<400> 44
   Gly Ser Ser Gly Arg Arg Ser Leu Gln Gly Gly Gly Gly
   1               5                   10
<210> 45
   <211> 30
   <212> DNA
   <213> Artificial Sequence
<220>
   <223> Linker zwischen CD63delLEL und Fluoreszenzprotein
<400> 45
   aagcttatcg ataccgtcga cctcgagaaa 30
<210> 46
   <211> 10
   <212> PRT
   <213> Artificial Sequence
<220>
   <223> Linker zwischen CD63delLEL und Fluoreszenzprotein; translatierte Sequenz
<400> 46
   Lys Leu Ile Asp Thr Val Asp Leu Glu Lys
   1               5                   10